# EUROPEAN PATENT APPLICATION

(11) **EP 3 865 511 A1**
(43) Date of publication of application: **18.08.2021**
(21) Application number: 21158862.9
(22) Date of filing: 13.04.2017
(51) Int. Cl.: C07K 16/18, C07K 16/30

(54) **ANTI-RSPO3 ANTIBODIES AND METHODS OF USE**

(30) Priority: 14.04.2016 US 201662322717 P
(62) Divisional of application: 17720346.0
(71) Applicant: F. Hoffmann-La Roche AG, 4070 Basel (CH)
(72) Inventor: STORM, Elaine, South San Francisco, CA 94080 (US)
(74) Representative: Aoki, Kenichiro

(57) **Abstract**

Provided herein are anti-RSPO3 antibodies and methods of using the same.

## Description

### FIELD

Provided herein are anti-RSPO3 antibodies and methods of using the same.

### BACKGROUND

The R-spondin (RSPO) family is a small group of four secreted proteins (RSPO1-RSPO4) that are widely expressed in vertebrate embryos and the adult. RSPOs have pleiotropic functions in development and stem cell growth by strongly enhancing Wnt pathway activation (Kazanskaya et al. Dev. Cell 7:525-534 (2004); Kim et al., Cell Cycle 5:23-26 (2006); WO 2005/040418). Mammalian RSPO1-RSPO4 share 40%-60% amino acid sequence identities and consist of a signal peptide, two adjacent furin-like cysteine-rich domains (FU-CRDs) followed by a thrombospondin type I repeat (TSR) domain, and a positively charged C-terminal region. The two FU-CRDs are essential and sufficient to promote Wnt/β-catenin signaling (Kazanskaya et al., Dev. Cell 7:525-534 (2004); WO 2005/040418).

LGR4 (leucine-rich repeat [LRR]-containing G-protein-coupled receptor [GPCR] 4), LGR5, and LGR6 (Hsu et al., Mol. Endocrinol. 12:1830-1845 (1998) and Hsu et al., Mol. Endocinol. 14:1257-1271 (2000)) are receptors for RSPOs. A common feature of the LGR4/5/6 receptors is their expression in distinct types of adult stem cells. LGR5 has already been described as a marker for resident stem cells in Wnt-dependent compartments, including the small intestine, colon, stomach, and hair follicle (Barker and Clevers Gastroenterology 138:1681-1696 (2010); Seshagiri et al., Nature 488:660-664 (2012)). LGR6 also serves as a marker of multipotent stem cells in the epidermis (Snippert et al., Science 327:1385-1389 (2010)). LGR4 is widely expressed in proliferating cells (Van Schoore et al., Histochem Cell Biol. 124:35-50 (2005)), and its knockout mice show developmental defects in many organs, including bone, kidney, testis, skin, and gall bladder (Mustata et al., EMBO Rep 12:558-564 (2011)). LGR4/5/6 receptors have a central array of 17 LRRs flanked by cysteine-rich sequences at both the N- and C-termini in the extracellular domain before seven transmembrane helices, and the extracellular domain is essential and sufficient for high-affinity binding with RSPOs (de Lau et al., Genome Biol. 13:242 (2011) and Wang et al, Genes & Dev. 27:1339-1344 (2013)).

LGR4/5/6 receptors may physically interact with low-density lipoprotein receptor-related protein 5/6 (LRP5/6) after RSPO recognition, and thereby RSPOs and Wnt ligands work together to activate Wnt/β-catenin signaling (de Lau et al., Genome Biol. 13:242 (2011); Carmon et al., Proc Natl Acad Sci 108:11452-11457 (2012)). RSPOs are also able to promote Wnt/β-catenin signaling by stabilizing the Frizzled and LRP5/6 receptors (Hao et al., Nature 485:195-200 (2012)). Zinc and RING finger 3 (ZNRF3) and its homolog, RING finger 43 (RNF43), are transmembrane E3 ubiquitin ligases that promote turnover of the Frizzled and LRP6 receptors on the cell surface (Hao et al., Nature 485:195-200 (2012); Koo et al., Nature 488:665-669 (2012)). ZNRF3 and RNF43 inhibit Wnt/β-catenin signaling by promoting ubiquitination and subsequent internalization and degradation of the Wnt receptors Frizzled and LRP6. (Hao et al., Cancers 8: 54-66 (2016).) RSPOs may induce clearance of ZNRF3 from the membrane by interacting with the extracellular domains of LGR4/5/6 and ZNRF3/RNF43, which stabilizes the Frizzled and LRP6 receptors to enhance Wnt/β-catenin signaling (Hao et al., Nature 485:195-200 (2012)).

Accordingly, binding of RSPO3 to LGR4 and 5 and to ZNRF3 or RNF43 may act as a negative feedback loop to down-regulate Wnt/β-catenin signaling. (Hao et al., Cancers 8: 54-66 (2016).) Specifically, RSPO may down-regulate Wnt/β-catenin signaling by simultaneously binding to the extracellular domains of ZNRF3/RNF43 and LGR4/5, which induces auto-ubiquitination and membrane clearance of ZNRF3/RNF43, resulting in an increased cell surface level of Frizzled. (Hao et al., Cancers 2016.) ZNRF3 and RNF43 may provide strong negative feedback control of Wnt/β-catenin signaling and, as a result, may prevent over amplification of intestinal stem cells. (Hao et al., Cancers 2016.) Knockouts of Znrf3 and Rnf43 proteins in mouse intestinal epithelium leads to unrestricted expansion of the intestinal stem cell zone, while systemic overexpression of RSPO induces a strong expansion of intestinal crypts. (Hao et al., Cancers 2016.)

Cancers may have aberrant Wnt/β-catenin signaling, which may be caused by gain of function mutations in RSPO2 and RSPO3 or by loss of function mutations in ZNRF3 or RNF43. In particular, fusions of RSPO3 and RSPO2 have been found in a percentage of colon tumors and the fused RSPO3 or RSPO2 proteins are believed to be capable of potentiating Wnt signaling. For example, exons 1 or 7 of protein tyrosine phosphatase receptor type K (PTPRK) may be fused to exon 2 of RSPO3. (S. Seshagiri et al., Nature 488: 660-664 (2012).) Furthermore, models have been created, for example, of screened patient derived xenograft samples harboring RSPO3 fusions, for example PTPRK-RSPO3 fusions. Such models, for example, may express elevated levels of RSPO3 but may not express any of RSPO1, 2, or 4. This is similar to what has been observed in RSPO3 fusion colon tumors, and is in contrast to normal colon, which expresses both RSPO2 and RSPO3. (E.E. Storm et al., Nature 529: 97-100 (January, 2016).) In addition, mutations in RNF43, such as frameshift, insertion, deletion, and nonsense mutations, have also been found in several cancers including endometrial, pancreatic, ovarian, liver, and colorectal cancers. (B. Madan & D. M. Virshup, Mol. Cancer Ther. 14(5): 1087-1094 (2015).)

As shown in the examples section below, the present invention provides anti-RSPO3 antibodies that, *inter alia,* may in some cases show a reduction of tumor growth in such models as well as improved RSPO3 binding affinity and better pharmacokinetics, including a longer half-life in vivo, in comparison to previously obtained anti-RSPO3 antibodies. In some embodiments, the anti-RSPO3 antibodies may inhibit binding of RSPO3 to each of LGR4, LGR5, and RNF43.

All references cited herein, including patent applications and publications, are incorporated by reference in their entirety.

### SUMMARY

The invention provides anti-RSPO3 antibodies and methods of using the same.

For example, provided herein in some embodiments are isolated antibodies that bind to RSPO3, comprising (a) light chain variable region (VL) comprising (i) a light chain hypervariable region 1 (HVR-L1) comprising the amino acid sequence of SEQ ID NO:5, (ii) HVR-L2 comprising the amino acid sequence of SEQ ID NO:6, and (iii) HVR-L3 comprising the amino acid sequence of SEQ ID NO:7; and a heavy chain variable region (VH) comprising (i) heavy chain hypervariable region 1 (HVR-H1) comprising the amino acid sequence of SEQ ID NO:8, (ii) HVR-H2 comprising the amino acid sequence of SEQ ID NO:9, and (iii) HVR-H3 comprising the amino acid sequence of SEQ ID NO:10; also referred to herein as the HVRs of antibody 4A6; or comprising (b) a VL comprising (i) HVR-L1 comprising the amino acid sequence of SEQ ID NO: 11, (ii) HVR-L2 comprising the amino acid sequence of SEQ ID NO: 12, and (iii) HVR-L3 comprising the amino acid sequence of SEQ ID NO: 13; and a VH comprising (i) HVR-H1 comprising the amino acid sequence of SEQ ID NO:14, (ii) HVR-H2 comprising the amino acid sequence of SEQ ID NO:15, and (iii) HVR-H3 comprising the amino acid sequence of SEQ ID NO:16; also referred to herein as the HVRs of antibody 11C10; or comprising (c) a VL comprising (i) HVR-L1 comprising the amino acid sequence of SEQ ID NO: 17, (ii) HVR-L2 comprising the amino acid sequence of SEQ ID NO:18, and (iii) HVR-L3 comprising the amino acid sequence of SEQ ID NO: 19; and a VH comprising (i) HVR-H1 comprising the amino acid sequence of SEQ ID NO:20, (ii) HVR-H2 comprising the amino acid sequence of SEQ ID NO:21, and (iii) HVR-H3 comprising the amino acid sequence of SEQ ID NO:22; also collectively referred to herein as the HVRs of antibody 15F3.

In some embodiments, the anti-RSPO3 antibody comprises one of the following sets of light chain variable region (VL) and heavy chain variable region (VH) sequences: (a) a VL sequence comprising SEQ ID NO:23 and a VH sequence comprising SEQ ID NO:24; (b) a VL sequence comprising SEQ ID NO:25 and a VH sequence comprising SEQ ID NO:26; (c) a VL sequence comprising SEQ ID NO:27 and a VH sequence comprising SEQ ID NO:28; (d) a VL sequence comprising SEQ ID NO:29 and a VH sequence comprising SEQ ID NO:30; (e) a VL sequence comprising SEQ ID NO:31 and a VH sequence comprising SEQ ID NO:32; (f) a VL sequence comprising SEQ ID NO:33 and a VH sequence comprising SEQ ID NO:34; (g) a VL sequence comprising SEQ ID NO:35 and a VH sequence comprising SEQ ID NO:36; (h) a VL sequence comprising SEQ ID NO:37 and a VH sequence comprising SEQ ID NO:38; (i) a VL sequence comprising SEQ ID NO:39 and a VH sequence comprising SEQ ID NO:40; (j) a VL sequence comprising SEQ ID NO:41 and a VH sequence comprising SEQ ID NO:42; (k) a VL sequence comprising SEQ ID NO:43 and a VH sequence comprising SEQ ID NO:44; (1) a VL sequence comprising SEQ ID NO:45 and a VH sequence comprising SEQ ID NO:46; (m) a VL sequence comprising SEQ ID NO:47 and a VH sequence comprising SEQ ID NO:48; (n) a VL sequence comprising SEQ ID NO:49 and a VH sequence comprising SEQ ID NO:50; (o) a VL sequence comprising SEQ ID NO:51 and a VH sequence comprising SEQ ID NO:52; (p) a VL sequence comprising SEQ ID NO:53 and a VH sequence comprising SEQ ID NO:54; (q) a VL sequence comprising SEQ ID NO:55 and a VH sequence comprising SEQ ID NO:56; (r) a VL sequence comprising SEQ ID NO:57 and a VH sequence comprising SEQ ID NO:58; (w) a VL sequence comprising SEQ ID NO:59 and a VH sequence comprising SEQ ID NO:60; or (x) a VL sequence comprising SEQ ID NO:61 and a VH sequence comprising SEQ ID NO:62.

In some embodiments, the anti-RSPO3 antibody comprises one of the following sets of light and heavy chain sequences: (a) a light chain sequence comprising SEQ ID NO:63 and a heavy chain sequence comprising SEQ ID NO:64; (b) a light chain sequence comprising SEQ ID NO:65 and a heavy chain sequence comprising SEQ ID NO:66; (c) a light chain sequence comprising SEQ ID NO:67 and a heavy chain sequence comprising SEQ ID NO:68; (d) a light chain sequence comprising SEQ ID NO:69 and a heavy chain sequence comprising SEQ ID NO:70; (e) a light chain sequence comprising SEQ ID NO:71 and a heavy chain sequence comprising SEQ ID NO:72; (f) a light chain sequence comprising SEQ ID NO:73 and a heavy chain sequence comprising SEQ ID NO:74; (g) a light chain sequence comprising SEQ ID NO:75 and a heavy chain sequence comprising SEQ ID NO:76 or 171; (h) a light chain sequence comprising SEQ ID NO:77 and a heavy chain sequence comprising SEQ ID NO:78; (i) a light chain sequence comprising SEQ ID NO:79 and a heavy chain sequence comprising SEQ ID NO:80; (j) a light chain sequence comprising SEQ ID NO:81 and a heavy chain sequence comprising SEQ ID NO:82; (k) a light chain sequence comprising SEQ ID NO:83 and a heavy chain sequence comprising SEQ ID NO:84; (1) a light chain sequence comprising SEQ ID NO:85 and a heavy chain sequence comprising SEQ ID NO:86; (m) a light chain sequence comprising SEQ ID NO:87 and a heavy chain sequence comprising SEQ ID NO:88 or 172; (n) a light chain sequence comprising SEQ ID NO:89 and a heavy chain sequence comprising SEQ ID NO:90; (o) a light chain sequence comprising SEQ ID NO:91 and a heavy chain sequence comprising SEQ ID NO:92; (p) a light chain sequence comprising SEQ ID NO:93 and a heavy chain sequence comprising SEQ ID NO:94; (q) a light chain sequence comprising SEQ ID NO:95 and a heavy chain sequence comprising SEQ ID NO:96; (r) a light chain sequence comprising SEQ ID NO:97 and a heavy chain sequence comprising SEQ ID NO:98; (w) a light chain sequence comprising SEQ ID NO:99 and a heavy chain sequence comprising SEQ ID NO: 100; or (x) a light chain sequence comprising SEQ ID NO: 101 and a heavy chain sequence comprising SEQ ID NO: 102 or 173. In some embodiments, the antibody is a humanized antibody comprising a wild-type human IgG1, IgG2, IgG3, or IgG4 constant region or comprising a human IgG1, IgG2, IgG3, or IgG4 constant region comprising a substitution at Asn297 (or its equivalent residue), for example, to reduce fucosylation of the antibody. In some embodiments, the antibody heavy chain comprises an Asn297Ala or Asn297Gly mutation.

Provided herein are also isolated antibodies that bind to RSPO3 having one or more of the following characteristics: (a) binding to human RSPO3 but not human RSPO1, human RSPO2, or human RSPO4; (b) binding to human RSPO3 with a Kd of less than 0.5 nM; (c) binding to cynomolgus RSPO3 with a Kd of less than 0.5 nM; (d) binds to murine RSPO3 with a Kd of less than 0.5 nM; (e) binding to human RSPO3 with a Kd of less than 1.0 nM, binds cyno RSPO3 with a Kd of less than 1.0 nM, and binds murine RSPO3 with a Kd of less than 1.0 nM; (f) binding to human RSPO3 with a Kd of less than 0.5 nM, binds cyno RSPO3 with a Kd of less than 0.5 nM, and binds murine RSPO3 with a Kd of less than 0.5 nM; (g) having a half-life of at least 6 days following a single 10 mg/kg intravenous administration in cynomolgus monkeys; and (h) reducing tumor growth or causing tumor regression in a patient derived RSPO3 fusion xenograft model, such as a PTPRK-RSPO3 fusion model, such as a PTPRK(exon1)-RSPO3(exon2) fusion or a PTPRK(exon7)-RSP03(exon2) fusion model. In some embodiments, the antibodies inhibit the interaction of human RSPO3 with one or more of transmembrane E3 ubiquitinase, such as ZNRF3 and RNF43, LGR4, LGR5, and LGR6. In some embodiments, the antibodies have one or more of the following properties: (a) inhibiting interaction of human RSPO3 and human RNF43 in a competition assay with an IC50 of 0.03 to 0.05 µg/ml; (b) inhibiting interaction of human RSPO3 and human LGR4 in a competition assay with an IC50 of 0.06 to 0.09 µg/ml; (c) inhibiting interaction of human RSPO3 and human LGR5 in a completion assay with an IC50 of 0.03 to 0.05 µg/ml; and (d) inhibiting interaction of human RSPO3 with one or more of human RNF43, LGR4, and LGR5 in a competition assay with a lower IC50 value (i.e. stronger inhibition) than that of a humanized IgG1 antibody 131R010 disclosed in WO2014/014007, otherwise referred to herein as "antibody A."

In some of the above embodiments, the antibodies do not bind to human RSPO2. In some of the above embodiments, the antibodies bind to human RSPO3 with a Kd of less than 4 nM, such as less than 3.5 nM, such as less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, or less than 0.2 nM. In some of the above embodiments, the antibodies bind to cynomolgus RSPO3 with a Kd of less than 3.5 nM, such as less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, or less than 0.2 nM. In some embodiments, the antibodies bind to murine RSPO3 with a Kd of less than 3.5 nM, such as less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, or less than 0.2 nM. In some embodiments, the antibodies bind human RSPO3 with a Kd of less than 1.0 nM, bind cyno RSPO3 with a Kd of less than 1.0 nM, and bind murine RSPO3 with a Kd of less than 1.0 nM. In some embodiments, the antibodies bind human RSPO3 with a Kd of less than 1.0 nM, bind cyno RSPO3 with a Kd of less than 0.5 nM, and bind murine RSPO3 with a Kd of less than 1.0 nM. In some embodiments, the antibodies bind human RSPO3 with a Kd of less than 0.5 nM, bind cyno RSPO3 with a Kd of less than 0.5 nM, and bind murine RSPO3 with a Kd of less than 0.5 nM. In some embodiments, the antibodies bind human RSPO3 with a Kd of less than 0.5 nM, bind cyno RSPO3 with a Kd of less than 0.3 nM, and bind murine RSPO3 with a Kd of less than 0.5 nM. In the above embodiments, binding to RSPO3 may be determined by surface plasmon resonance (e.g. BIACORE®) assays.

In some of the above embodiments, an RSPO3 antibody may have a half-life of at least 6 days, such as at least 8 days, at least 9 days, at least 10 days, at least 11 days, or at least 12 days following a single 10 mg/kg intravenous administration in cynomolgus monkeys. In some embodiments, the antibody may have a half-life of at least 6 days, such as at least 8 days, at least 9 days, at least 10 days, at least 11 days, or at least 12 days following a single 10 mg/kg intravenous administration in cynomolgus monkeys and may bind human RSPO3 with a Kd of less than 3.5 nM, bind cyno RSPO3 with a Kd of less than 2 nM, and bind murine RSPO3 with a Kd of less than 3.5 nM.

Also provided herein are antibodies that bind human RSPO3 with a Kd of less than 1 nM, bind cyno RSPO3 with a Kd of less than 0.5 nM, and bind murine RSPO3 with a Kd of less than 1 nM; and that have a half-life of at least 6 days following a single 10 mg/kg intravenous administration in cynomolgus monkeys. In some embodiments, the antibodies may bind human RSPO3 with a Kd of less than 0.5 nM, bind cyno RSPO3 with a Kd of less than 0.5 nM, and bind murine RSPO3 with a Kd of less than 0.5 nM; and has also have a half-life of at least 6 days following a single 10 mg/kg intravenous administration in cynomolgus monkeys. In some embodiments, the antibodies may bind human RSPO3 with a Kd of less than 0.5 nM, bind cyno RSPO3 with a Kd of less than 0.3 nM, and bind murine RSPO3 with a Kd of less than 0.5 nM; and have a half-life of at least 6 days following a single 10 mg/kg intravenous administration in cynomolgus monkeys. In some of the above embodiments, the antibodies may have a half-life of at least 8 days following a single 10 mg/kg intravenous administration in cynomolgus monkeys. In some of the above embodiments, the antibodies may have a half-life of at least 10 days following a single 10 mg/kg intravenous administration in cynomolgus monkeys.

In some embodiments, antibodies with the above functional properties may include antibodies comprising (a) light chain variable region (VL) comprising (i) a light chain hypervariable region 1 (HVR-L1) comprising the amino acid sequence of SEQ ID NO:5, (ii) HVR-L2 comprising the amino acid sequence of SEQ ID NO:6, and (iii) HVR-L3 comprising the amino acid sequence of SEQ ID NO:7; and a heavy chain variable region (VH) comprising (i) heavy chain hypervariable region 1 (HVR-H1) comprising the amino acid sequence of SEQ ID NO:8, (ii) HVR-H2 comprising the amino acid sequence of SEQ ID NO:9, and (iii) HVR-H3 comprising the amino acid sequence of SEQ ID NO:10; or comprising (b) a VL comprising (i) HVR-L1 comprising the amino acid sequence of SEQ ID NO:11, (ii) HVR-L2 comprising the amino acid sequence of SEQ ID NO:12, and (iii) HVR-L3 comprising the amino acid sequence of SEQ ID NO: 13; and a VH comprising (i) HVR-H1 comprising the amino acid sequence of SEQ ID NO: 14, (ii) HVR-H2 comprising the amino acid sequence of SEQ ID NO: 15, and (iii) HVR-H3 comprising the amino acid sequence of SEQ ID NO: 16; or comprising (c) a VL comprising (i) HVR-L1 comprising the amino acid sequence of SEQ ID NO: 17, (ii) HVR-L2 comprising the amino acid sequence of SEQ ID NO: 18, and (iii) HVR-L3 comprising the amino acid sequence of SEQ ID NO: 19; and a VH comprising (i) HVR-H1 comprising the amino acid sequence of SEQ ID NO:20, (ii) HVR-H2 comprising the amino acid sequence of SEQ ID NO:21, and (iii) HVR-H3 comprising the amino acid sequence of SEQ ID NO:22.

Further examples may include antibodies that comprise one of the following sets of light chain variable region (VL) and heavy chain variable region (VH) sequences: (a) a VL sequence comprising SEQ ID NO:23 and a VH sequence comprising SEQ ID NO:24; (b) a VL sequence comprising SEQ ID NO:25 and a VH sequence comprising SEQ ID NO:26; (c) a VL sequence comprising SEQ ID NO:27 and a VH sequence comprising SEQ ID NO:28; (d) a VL sequence comprising SEQ ID NO:29 and a VH sequence comprising SEQ ID NO:30; (e) a VL sequence comprising SEQ ID NO:31 and a VH sequence comprising SEQ ID NO:32; (f) a VL sequence comprising SEQ ID NO:33 and a VH sequence comprising SEQ ID NO:34; (g) a VL sequence comprising SEQ ID NO:35 and a VH sequence comprising SEQ ID NO:36; (h) a VL sequence comprising SEQ ID NO:37 and a VH sequence comprising SEQ ID NO:38; (i) a VL sequence comprising SEQ ID NO:39 and a VH sequence comprising SEQ ID NO:40; (j) a VL sequence comprising SEQ ID NO:41 and a VH sequence comprising SEQ ID NO:42; (k) a VL sequence comprising SEQ ID NO:43 and a VH sequence comprising SEQ ID NO:44; (l) a VL sequence comprising SEQ ID NO:45 and a VH sequence comprising SEQ ID NO:46; (m) a VL sequence comprising SEQ ID NO:47 and a VH sequence comprising SEQ ID NO:48; (n) a VL sequence comprising SEQ ID NO:49 and a VH sequence comprising SEQ ID NO:50; (o) a VL sequence comprising SEQ ID NO:51 and a VH sequence comprising SEQ ID NO:52; (p) a VL sequence comprising SEQ ID NO:53 and a VH sequence comprising SEQ ID NO:54; (q) a VL sequence comprising SEQ ID NO:55 and a VH sequence comprising SEQ ID NO:56; (r) a VL sequence comprising SEQ ID NO:57 and a VH sequence comprising SEQ ID NO:58; (w) a VL sequence comprising SEQ ID NO:59 and a VH sequence comprising SEQ ID NO:60; or (x) a VL sequence comprising SEQ ID NO:61 and a VH sequence comprising SEQ ID NO:62.

Yet further examples may include antibodies that comprise one of the following sets of light and heavy chain sequences: (a) a light chain sequence comprising SEQ ID NO:63 and a heavy chain sequence comprising SEQ ID NO:64; (b) a light chain sequence comprising SEQ ID NO:65 and a heavy chain sequence comprising SEQ ID NO:66; (c) a light chain sequence comprising SEQ ID NO:67 and a heavy chain sequence comprising SEQ ID NO:68; (d) a light chain sequence comprising SEQ ID NO:69 and a heavy chain sequence comprising SEQ ID NO:70; (e) a light chain sequence comprising SEQ ID NO:71 and a heavy chain sequence comprising SEQ ID NO:72; (f) a light chain sequence comprising SEQ ID NO:73 and a heavy chain sequence comprising SEQ ID NO:74; (g) a light chain sequence comprising SEQ ID NO:75 and a heavy chain sequence comprising SEQ ID NO:76 or 171; (h) a light chain sequence comprising SEQ ID NO:77 and a heavy chain sequence comprising SEQ ID NO:78; (i) a light chain sequence comprising SEQ ID NO:79 and a heavy chain sequence comprising SEQ ID NO:80; (j) a light chain sequence comprising SEQ ID NO:81 and a heavy chain sequence comprising SEQ ID NO:82; (k) a light chain sequence comprising SEQ ID NO:83 and a heavy chain sequence comprising SEQ ID NO:84; (1) a light chain sequence comprising SEQ ID NO:85 and a heavy chain sequence comprising SEQ ID NO:86; (m) a light chain sequence comprising SEQ ID NO:87 and a heavy chain sequence comprising SEQ ID NO:88 or 172; (n) a light chain sequence comprising SEQ ID NO:89 and a heavy chain sequence comprising SEQ ID NO:90; (o) a light chain sequence comprising SEQ ID NO:91 and a heavy chain sequence comprising SEQ ID NO:92; (p) a light chain sequence comprising SEQ ID NO:93 and a heavy chain sequence comprising SEQ ID NO:94; (q) a light chain sequence comprising SEQ ID NO:95 and a heavy chain sequence comprising SEQ ID NO:96; (r) a light chain sequence comprising SEQ ID NO:97 and a heavy chain sequence comprising SEQ ID NO:98; (w) a light chain sequence comprising SEQ ID NO:99 and a heavy chain sequence comprising SEQ ID NO: 100; or (x) a light chain sequence comprising SEQ ID NO: 101 and a heavy chain sequence comprising SEQ ID NO: 102 or 173. In some embodiments, the antibody is a humanized antibody comprising a wild-type human IgG1, IgG2, IgG3, or IgG4 constant region or comprising a human IgG1, IgG2, IgG3, or IgG4 constant region comprising a substitution at Asn297 (or its equivalent residue), for example, to reduce fucosylation of the antibody. In some embodiments, the antibody heavy chain comprises an Asn297Ala or Asn297Gly mutation.

In some embodiments of any of the above anti-RSPO3 antibodies, the antibody may inhibit RSPO3 mediated wnt signaling in a patient-derived xenograft model with an RSPO3 fusion, such as a PTPRK-RSPO3 fusion, such as a PTPRK(exon1)-RSPO3(exon2) fusion or a PTPRK(exon7)-RSPO3(exon2) fusion, such as the CRC-D or CRC-C models described in the working examples herein. In some embodiment, the antibody inhibits cancer stem cell growth, an/or induces and/or promotes cancer cell *(e.g.,* cancer stem cell) differentiation *(e.g.,* terminal differentiation and/or differentiation into progenitor cell) in such a model. In some embodiments, treatment of a mouse cancer xenograft model with an antibody of the invention leads to tumor regression or a reduction of tumor growth, for example in a xenograft model with an RSPO3 fusion, such as a PTPRK-RSPO3 fusion, such as a PTPRK(exon1)-RSPO3(exon2) fusion or a PTPRK(exon7)-RSPO3(exon2) fusion, such as the CRC-D or CRC-C model. In some embodiments, the antibody is more potent (i.e. achieves at least the same result at a lower dosage) in a xenograft model with an RSPO3 fusion (such as a PTPRK-RSPO3 fusion, such as a PTPRK(exon1)-RSPO3(exon2) fusion or a PTPRK(exon7)-RSPO3(exon2) fusion, such as the CRC-D or CRC-C model) than a previously identified anti-RSPO3 antibody, such as the IgG1 antibody designated 131R010 in PCT publication WO 2014/012007, also called "antibody A" herein.

In some embodiments of any of the anti-RSPO3 antibodies, the antibody is a monoclonal antibody. In some embodiments of any of the anti-RSPO3 antibodies, the antibody is a human, humanized, or chimeric antibody, or is a bi-specific or multispecific antibody. In some embodiments of any of the anti-RSPO3 antibodies, the antibody is a full length IgG1 or IgG2a antibody, or is an antigen binding fragment, such as comprising a Fab, F(ab)₂, Fv, or scFv fragment. In some embodiments of any of the anti-RSPO3 antibodies, the antibody has reduced or depleted effector function. In some embodiments of any of the anti-RSPO3 antibodies, the anti-RSPO3 antibody comprises an engineered alanine at amino acid position 297 according to EU numbering convention. In some embodiments of any of the anti-RSPO3 antibodies, the anti-RSPO3 antibody comprises an engineered alanine at amino acid position 265 according to EU numbering convention.

In some embodiments of any of the anti-RSPO3 antibodies, the antibody is for use as a medicament. In some embodiments of any of the anti-RSPO3 antibodies, the antibody is for use in treating cancer. In some embodiments, the cancer is gastrointestinal cancer, stomach cancer, colon cancer, colorectal cancer, or rectal cancer. In some embodiments, the cancer is characterized by increased expression of RSPO3 compared to a reference. In some embodiments, the cancer is characterized by an RSPO3 translocation. In some embodiments of any of the anti-RSPO3 antibodies, the antibody is for use in inhibiting wnt signaling, inhibiting angiogenesis and/or vasculogenesis, and/or inhibiting cell proliferation.

Provided here are also isolated nucleic acids or sets of nucleic acids encoding the antibodies described herein. A set of nucleic acids, for example, may include separate isolated nucleic acids encoding a light chain and a heavy chain of an antibody or domains of a bi-specific or multispecific antibody. Further provided herein are host cells comprising the nucleic acid or sets of nucleic acids encoding the antibodies described herein. Provided here in are methods of producing an antibody described herein comprising culturing the host cell comprising the nucleic acid of an antibody described herein so that the antibody is produced. In some embodiments, the method of producing further comprising recovering the antibody from the host cell.

Provided here are immunoconjugates comprising an antibody described herein and a cytotoxic agent.

Further provided herein are pharmaceutical formulations comprising an antibody described herein and a pharmaceutically acceptable carrier. In some embodiments, the pharmaceutical formulation further comprises an additional therapeutic agent. In some embodiments, the additional therapeutic agent is a chemotherapy agent. In some embodiments, the additional therapeutic agent is a taxane. In some embodiments, the taxane is paclitaxel or docetaxel. In some embodiments, the additional therapeutic agent is a platinum agent. In some embodiments, the platinum agent is carboplatin, oxaliplatin, and/or cisplatin. In some embodiments, the additional therapeutic agent is a topoisomerase inhibitor. In some embodiments, the topoisomerase inhibitor is irinotecan, topotecan, etoposide, and/or mitoxantrone. In some embodiments, the additional therapeutic agent is folinic acid (e.g., Leucovorin). In some embodiments, the additional therapeutic agent is a nucleoside metabolic inhibitor. In some embodiments, the nucleoside metabolic inhibitor is fluorouracil, capecitabine, and/or gemcitabine. In some embodiments, the additional therapeutic agent is folinic acid, 5-fluorouracil, and/or oxaliplatin. In some embodiments, the additional therapeutic agent is 5-fluorouracil and irinotecan. In some embodiments, the additional therapeutic agent is a taxane and platinum agent. In some embodiments, the additional therapeutic agent is paclitaxel and carboplatin. In some embodiments, the additional therapeutic agent is pemetrexate. In some embodiments, the additional therapeutic agent is a hedgehog inhibitor (e.g., vismodegib).

Provided herein are uses of an antibody described herein in the manufacture of a medicament for treatment of cancer. In some embodiments, the cancer is gastrointestinal cancer, stomach cancer, colon cancer, colorectal cancer, or rectal cancer. In some embodiments, the cancer is lung cancer. In some embodiments, the cancer is characterized by increased expression of RSPO3 compared to a reference. In some embodiments, the cancer is characterized by an RSPO3 translocation. In some embodiments, the cancer is characterized by an RSPO3 fusion, such as a PTPRK-RSPO3 fusion, such as a PTPRK(exon1)-RSPO3(exon2) fusion or a PTPRK(exon7)-RSPO3(exon2) fusion (aka. PTPRK(e7)/RSPO3(e2)). Further, provided herein are uses of an antibody described herein in the manufacture of a medicament for inhibiting wnt signaling, inhibiting angiogenesis and/or vasculogenesis, and/or inhibiting cell proliferation. In some embodiments, the anti-RSPO antibody is used in combination with an additional therapeutic agent (e.g., administered sequentially or concurrently). In some embodiments, the additional therapeutic agent is a chemotherapy agent. In some embodiments, the additional therapeutic agent is a taxane. In some embodiments, the taxane is paclitaxel or docetaxel. In some embodiments, the additional therapeutic agent is a platinum agent. In some embodiments, the platinum agent is carboplatin, oxaliplatin, and/or cisplatin. In some embodiments, the additional therapeutic agent is a topoisomerase inhibitor. In some embodiments, the topoisomerase inhibitor is irinotecan, topotecan, etoposide, and/or mitoxantrone. In some embodiments, the additional therapeutic agent is folinic acid (e.g., Leucovorin). In some embodiments, the additional therapeutic agent is a nucleoside metabolic inhibitor. In some embodiments, the nucleoside metabolic inhibitor is fluorouracil, capecitabine, and/or gemcitabine. In some embodiments, the additional therapeutic agent is folinic acid, 5-fluorouracil, and/or oxaliplatin. In some embodiments, the additional therapeutic agent is 5-fluorouracil and irinotecan. In some embodiments, the additional therapeutic agent is a taxane and platinum agent. In some embodiments, the additional therapeutic agent is paclitaxel and carboplatin. In some embodiments, the additional therapeutic agent is pemetrexate. In some embodiments, the additional therapeutic agent is a hedgehog inhibitor (e.g., vismodegib).

Provided herein are methods of treating an individual having cancer comprising administering to the individual an effective amount of an antibody described herein. In some embodiments, the cancer is gastrointestinal cancer, stomach cancer, colon cancer, colorectal cancer, or rectal cancer. In some embodiments, the cancer is lung cancer. In some embodiments, the method further comprises administering an additional therapeutic agent to the individual, for example, a chemotherapy agent. In some embodiments, the cancer is lung cancer. In some embodiments, the cancer is characterized by increased expression of RSPO3 compared to a reference. In some embodiments, the cancer is characterized by an RSPO3 translocation. In some embodiments, the cancer is characterized by an RSPO3 fusion, such as a PTPRK-RSPO3 fusion, such as a PTPRK(exon1)-RSPO3(exon2) fusion or a PTPRK(exon7)-RSPO3(exon2) fusion (aka. PTPRK(e7)/RSPO3(e2)). Also provided herein are methods of inhibiting wnt signaling, inhibiting angiogenesis and/or vasculogenesis, and/or inhibiting cell proliferation in an individual comprising administering to the individual an effective amount of an antibody described herein to inhibit wnt signaling, inhibit angiogenesis and/or vasculogenesis, and/or inhibit cell proliferation. In some embodiments, the method comprises administering an additional therapeutic agent. In some embodiments, the additional therapeutic agent is a chemotherapy agent. In some embodiments, the additional therapeutic agent is a taxane. In some embodiments, the taxane is paclitaxel or docetaxel. In some embodiments, the additional therapeutic agent is a platinum agent. In some embodiments, the platinum agent is carboplatin, oxaliplatin, and/or cisplatin. In some embodiments, the additional therapeutic agent is a topoisomerase inhibitor. In some embodiments, the topoisomerase inhibitor is irinotecan, topotecan, etoposide, and/or mitoxantrone. In some embodiments, the additional therapeutic agent is folinic acid (e.g., Leucovorin). In some embodiments, the additional therapeutic agent is a nucleoside metabolic inhibitor. In some embodiments, the nucleoside metabolic inhibitor is fluorouracil, capecitabine, and/or gemcitabine. In some embodiments, the additional therapeutic agent is folinic acid, 5-fluorouracil, and/or oxaliplatin. In some embodiments, the additional therapeutic agent is 5-fluorouracil and irinotecan. In some embodiments, the additional therapeutic agent is a taxane and platinum agent. In some embodiments, the additional therapeutic agent is paclitaxel and carboplatin. In some embodiments, the additional therapeutic agent is pemetrexate. In some embodiments, the additional therapeutic agent is a hedgehog inhibitor (e.g., vismodegib).

### BRIEF DESCRIPTION OF THE FIGURES

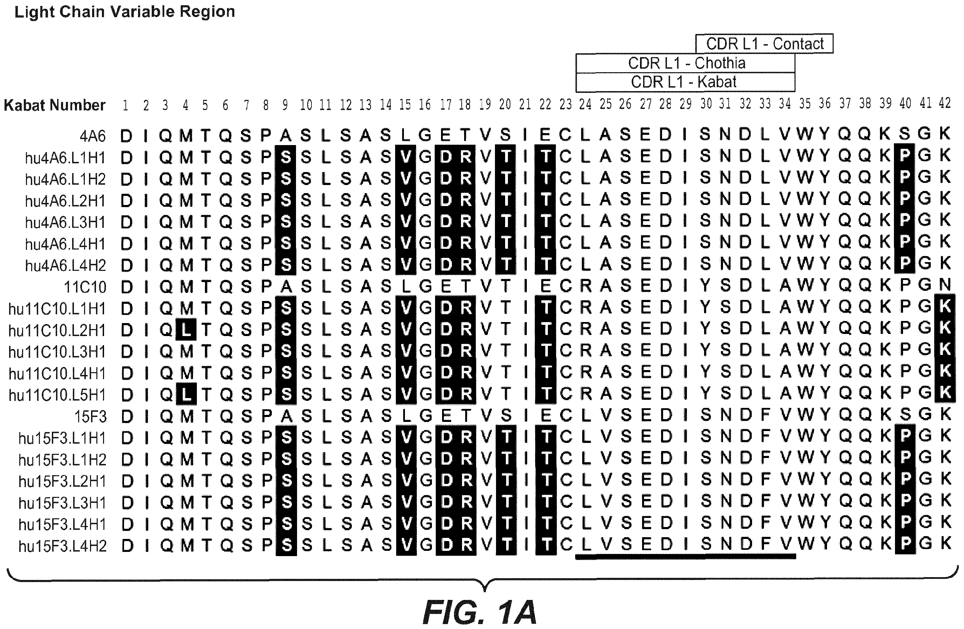

**Figures 1a-1c** show alignments of the light chain variable regions of antibodies 4A6, 11C10, and 15F3 and their humanized versions. The amino acid positions are shown in Kabat numbering and the Kabat and Chothia CDR sequences are underlined below the figures and noted in shaded boxes above the figures. Positions of contact are also denoted with an unshaded box above the figures and a thin line below the figures.

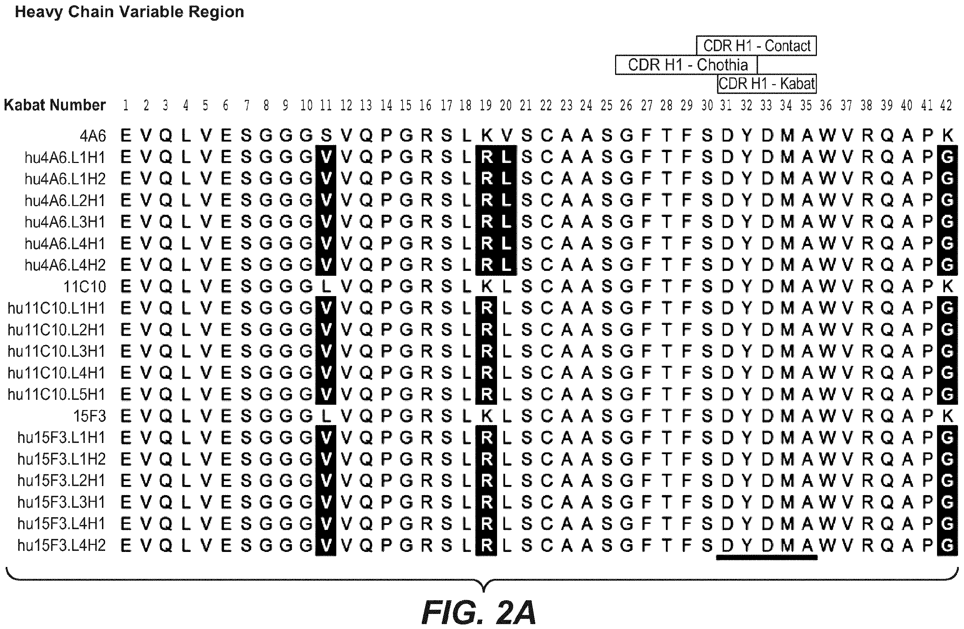

**Figures 2a-2c** show alignments of the heavy chain variable regions of antibodies 4A6, 11C10, and 15F3 and their humanized versions. The amino acid positions are shown in Kabat numbering and the Kabat and Chothia CDR sequences are underlined below the figures and noted in shaded boxes above the figures. Positions of contact are also denoted with an unshaded box above the figures and a thin line below the figures.

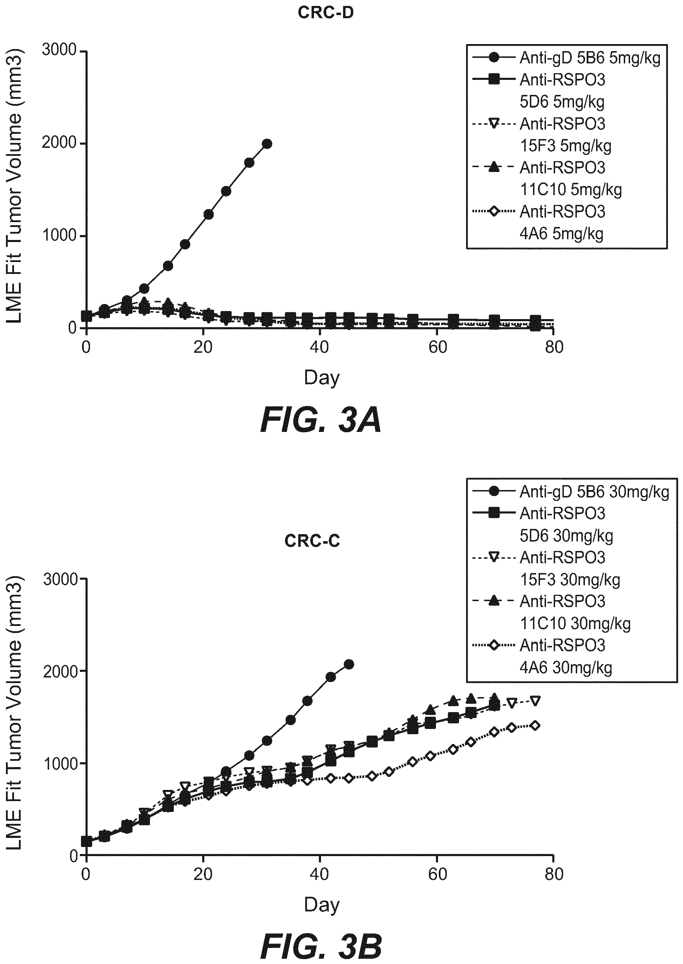

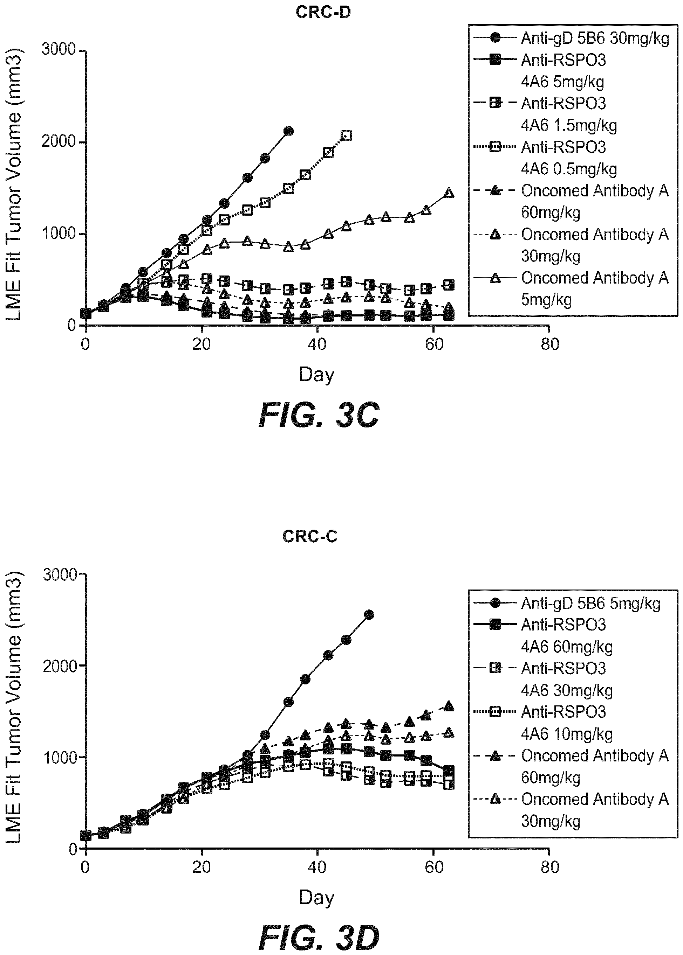

**Figures 3a-3d****.** Figure 3a shows tumor volume in a colorectal cancer patient derived xenograft model with a PTPRK-RSPO3fusion, called CRC-D in response to treatment with 5 mg/kg of humanized anti-RSPO3 antibodies hu4A6.L4H2, hu11C10.L5H1, and hu15F3.L4H2, each described herein, or a previously described anti-RSPO3 antibody 5D6 (see WO2015/058132), or an anti-gD control antibody also at 5 mg/kg. The anti-RSPO3 antibodies also have an N297G modification in the heavy chain constant region. The antibodies are depicted simply as 4A6, 11C10, 15F3, and 5D6 in the figures. Figure 3a shows that anti-RSPO3 treatment resulted in tumor regression. Figure 3b shows tumor volume in a colorectal cancer patient derived xenograft model with a PTPRK-RSPO3 fusion, called CRC-C in response to treatment with 30 mg/kg of the anti-RSPO3 antibodies 4A6, 11C10, 15F3, and 5D6 or the anti-gD control antibody also at 30 mg/kg. Treatment resulted in delayed onset of significant, durable reduction in tumor growth. Figure 3c shows a similar experiment in the CRC-D model comparing the antibody 5D6 at 30 mg/kg, antibody 4A6 at 0.5, 1.0, and 5 mg/kg, and another previously identified anti-RSPO3 antibody called antibody A (IgG1 antibody 131R010 of WO2014/012007) at 5, 30, and 60 mg/kg doses. Both antibody A and 4A6 demonstrated dose-dependent tumor regression in the model, with roughly equivalent tumor regression at 5 mg/kg 4A6 and 60 mg/kg antibody A. However, 4A6 was about 10-fold more potent than antibody A. Figure 3d shows a similar experiment to Figure 3c in the CRC-C model. Here, 5 mg/kg 5D6, 10, 30, and 60 mg/kg 4A6, and 30 and 60 mg/kg antibody A were tested and compared. At all tested dose levels antibody 4A6 showed superior reduction of tumor growth than antibody A. Antibody 4A6 was also more potent than antibody A, given that a 10 mg/kg dose of 4A6 showed superior results to both the 30 and 60 mg/kg doses of antibody A.

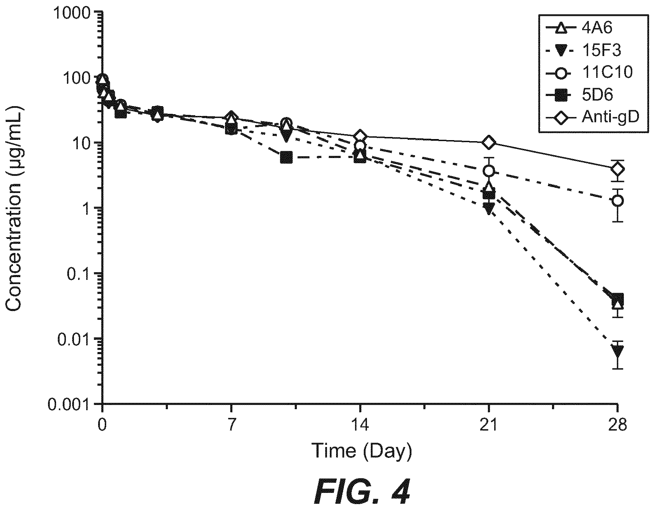

Figure 4 shows the results of pharmacokinetic studies of the humanized antibodies hu4A6.L4H2, hu11C10.L5H2, hu15F3.L4H2, antibody 5D6, and control anti-gD in Balb/c nude mice following a single 5 mg/kg IV dose. Mean serum concentrations of serum antibodies are shown over time. The 4A6, 15F3, and 11C10 antibodies demonstrated biphasic disposition profiles typical of IgG1 antibodies and were indistinguishable from the control anti-gD antibody. The 5D6 antibody showed faster clearance than the others.

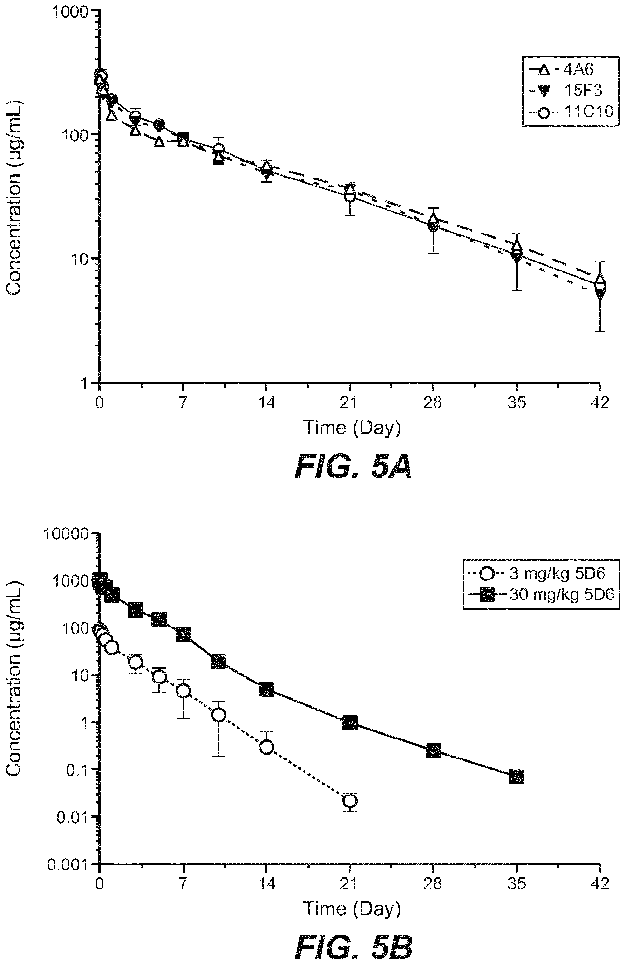

**Figures 5a-5b** show results of pharmacokinetic studies of the humanized antibodies hu4A6.L4H2, hu11C10.L5H2, hu15F3.L4H2 following a single bolus dose of 10 mg/kg (Figure 5a) and pharmacokinetic studies following a single bolus dose of 3 or 30 mg/kg 5D6 antibody (Figure 5b). The figures show mean serum anti-RSPO3 antibody concentration over time. The 4A6, 11C10, and 15F3 antibodies show a slower overall clearance than the 5D6 antibody as well as a clearance typical for an IgG1 antibody.

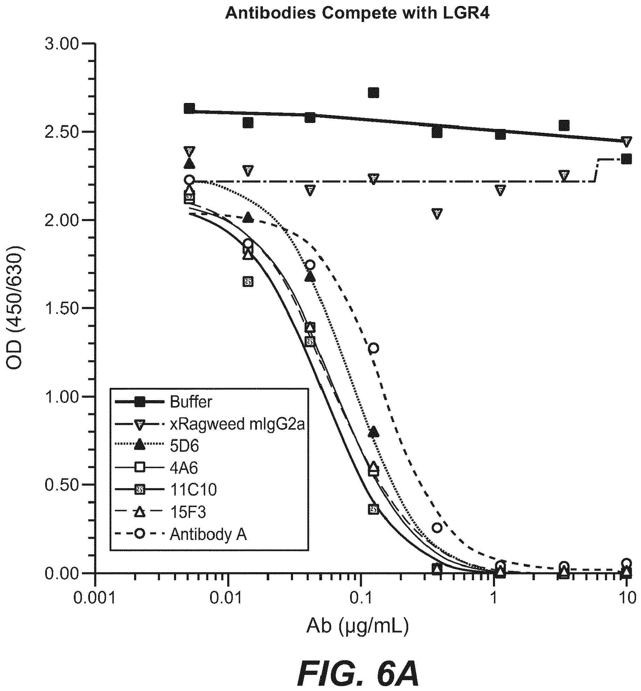

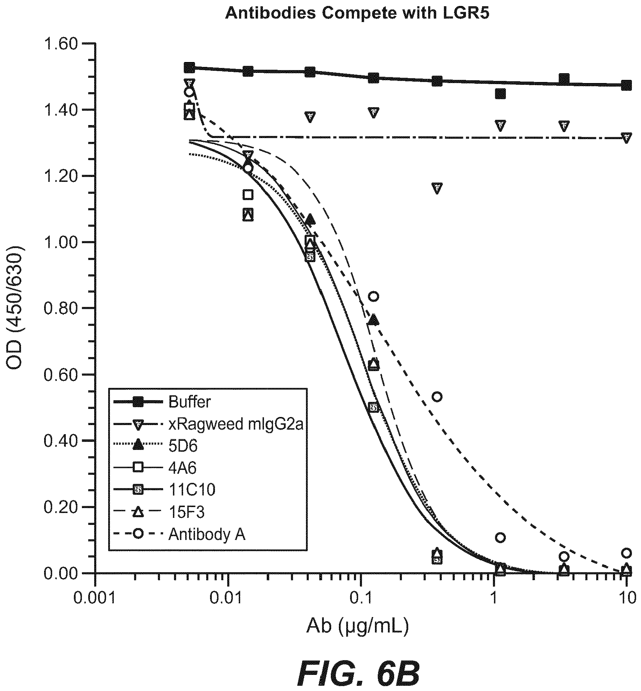

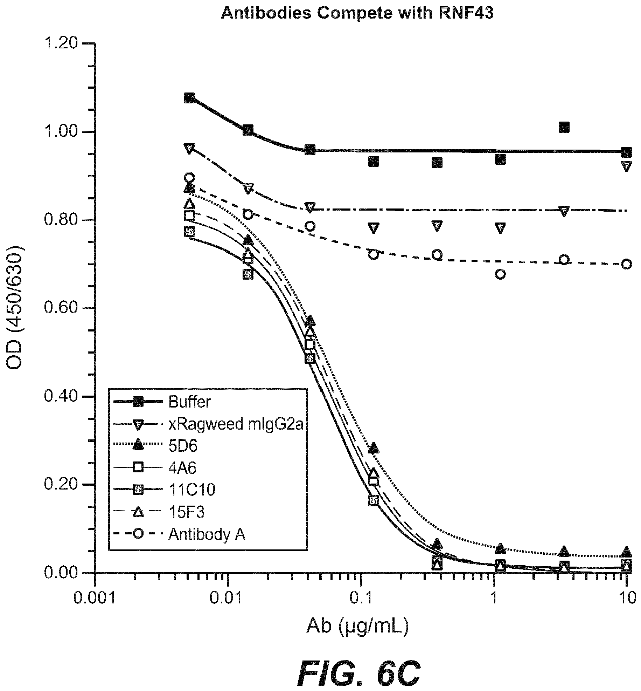

**Figures 6a-6c** show results of competition ELISA assays comparing activity of anti-RSPO3 antibodies 5D6, 4A6, 11C10, 15F3, and antibody A as well as buffer and anti-ragweed antibody controls in blocking the binding of LGR4 extracellular domain (ECD) (Fig. 6a) and LGR5 extracellular domain (ECD) (Fig 6b) and RNF43 (Fig. 6c) to RSPO3. Decreases in optical density signal from bound RSPO3 with increasing antibody concentration are plotted in each figure.

### DETAILED DESCRIPTION

### I. DEFINITIONS

The terms "R-spondin" and "RSPO," as used herein, refer to any native RSPO (*e.g.,* RSPO1, RSPO2, RSPO3, and/or RSPO4) from any vertebrate source, including mammals such as primates (*e.g.,* humans) and rodents (*e.g.,* mice and rats), unless otherwise indicated. The term encompasses "full-length," unprocessed RSPO as well as any form of RSPO that results from processing in the cell. The term also encompasses naturally occurring variants of RSPO, *e.g.,* splice variants or allelic variants. In some embodiments, the amino acid sequence of an exemplary human RSPO is RSPO1, for example, as shown in SEQ ID NO:3. In some embodiments, the amino acid sequence of an exemplary human RSPO is RSPO2, for example, as shown in SEQ ID NO:1. In some embodiments, the amino acid sequence of an exemplary human RSPO is RSPO3, for example, as shown in SEQ ID NO:2. In some embodiments, the amino acid sequence of an exemplary human RSPO is RSPO4, for example, as shown in SEQ ID NO:4.

The terms "R-spondin 2" and "RSPO2," as used herein, refers to any native RSPO2 from any vertebrate source, including mammals such as primates (*e.g.,* humans) and rodents (*e.g.,* mice and rats), unless otherwise indicated. The term encompasses "full-length," unprocessed RSPO2 as well as any form of RSPO2 that results from processing in the cell. The term also encompasses naturally occurring variants of RSPO2, *e.g*., splice variants or allelic variants. In some embodiments, the amino acid sequence of an exemplary human RSPO2 is UNIPROT Q6UXX9-1 as of October 18, 2013. In some embodiments, the amino acid sequence of an exemplary human RSPO2 is UNIPROT Q6UXX9-2 as of October 18, 2013. In some embodiments, the amino acid sequence of an exemplary human RSPO2 is UNIPROT Q6UXX9-3 as of October 18, 2013. In some embodiments, the amino acid sequence of an exemplary human RSPO2 is shown in SEQ ID NO:1.

The terms "R-spondin 3" and "RSPO3," as used herein, refers to any native RSPO3 from any vertebrate source, including mammals such as primates (*e.g.,* humans) and rodents (*e.g.,* mice and rats), unless otherwise indicated. The term encompasses "full-length," unprocessed RSPO3 as well as any form of RSPO3 that results from processing in the cell. The term also encompasses naturally occurring variants of RSPO3, *e.g*., splice variants or allelic variants. In some embodiments, the amino acid sequence of an exemplary human RSPO2 is UNIPROT Q9BXY4-1 as of October 18, 2013. In some embodiments, the amino acid sequence of an exemplary human RSPO2 is UNIPROT Q9BXY4-2 as of October 18, 2013. In some embodiments, the amino acid sequence of an exemplary human RSPO3 is shown in SEQ ID NO:2.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

"Affinity" refers to the strength of the sum total of noncovalent interactions between a single binding site of a molecule (*e.g.,* an antibody) and its binding partner (*e.g.,* an antigen). Unless indicated otherwise, as used herein, "binding affinity" refers to intrinsic binding affinity which reflects a 1:1 interaction between members of a binding pair (*e.g*., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-RSPO3 antibody" and "an antibody that binds to RSPO3" refer to an antibody that is capable of binding RSPO3 with sufficient affinity such that the antibody is useful as a diagnostic and/or therapeutic agent in targeting RSPO3. In one embodiment, the extent of binding of an anti-RSPO3 antibody to a non-RSPO3 protein is less than about 10% of the binding of the antibody to RSPO3 as measured, e.g., by a radioimmunoassay (RIA). In certain embodiments, an antibody that binds to RSPO3 has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (*e.g.,* 10⁻⁸ M or less, *e.g.* from 10⁻⁸ M to 10⁻¹³ M, *e.g.,* from 10⁻⁹ M to 10⁻¹³ M). In certain embodiments, an anti-RSPO3 antibody binds to an epitope of RSPO3 that is conserved among RSPO3 from different species.

The term "bind" when used in the context of an antibody that "binds" to a particular target such as RSPO3 means that the antibody interacts with the target with sufficient affinity such that the antibody is useful as a diagnostic and/or therapeutic agent in targeting RSPO3. An antibody that "does not bind" a particular molecule, such as RSPO2, does not interact with that molecule with sufficient affinity such that the antibody would be useful as a diagnostic and/or therapeutic agent. In some embodiments, the antibody that does not bind to a particular molecule, does not bind to the molecule with an affinity any tighter than it would have towards a non-RSPO, control polypeptide.

The term "antibody" herein is used in the broadest sense and encompasses various antibody structures, including but not limited to monoclonal antibodies, polyclonal antibodies, multispecific antibodies (*e.g*., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (*e.g*,. scFv); and multispecific antibodies formed from antibody fragments.

An "antibody that competes for binding with" a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), *e.g*., IgG₁, IgG₂, IgG₃, IgG₄, IgA₁, and IgA₂. The heavy chain constant domains that correspond to the different classes of immunoglobulins are called α, δ, ε, γ, and µ, respectively.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (*e.g*. B cell receptor); and B cell activation.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one that possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework that represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, *e.g*., a non-human antibody, refers to an antibody that has undergone humanization.

The term "hypervariable region" or "HVR" as used herein refers to each of the regions of an antibody variable region which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (HI, H2, H3), and three in the VL (L1, L2, L3). Exemplary HVRs herein include:
(a) hypervariable loops occurring at amino acid residues 26-32 (L1), 50-52 (L2), 91-96 (L3), 26-32 (HI), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (HI), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (HI), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (HI), 26-35b (HI), 49-65 (H2), 93-102 (H3), and 94-102 (H3).

Unless otherwise indicated, HVR residues and other residues in the variable domain *(e.g.,* FR residues) are numbered herein according to Kabat et al., *supra.*

The term "variable region" or "variable domain" refer interchangeably to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (*See, e.g.,* Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. *See, e.g.,* Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

An "immunoconjugate" is an antibody conjugated to one or more heterologous molecule(s), including but not limited to a cytotoxic agent.

An "isolated" antibody is one that has been separated from a component of its natural environment. In some embodiments, an antibody is purified to greater than 95% or 99% purity as determined by, for example, electrophoretic (*e.g*., SDS-PAGE, isoelectric focusing (IEF), capillary electrophoresis) or chromatographic (*e.g*., ion exchange or reverse phase HPLC). For review of methods for assessment of antibody purity, *see, e.g.,* Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-RSPO3 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell. An "isolated set of nucleic acids encoding an anti-RSPO3 antibody" refers to more than one nucleic acid molecule encoding antibody heavy and light chains (or fragments thereof).

The term "monoclonal antibody" as used herein refers to an antibody obtained from a population of substantially homogeneous antibodies, *i.e.,* the individual antibodies comprising the population are identical and/or bind the same epitope, except for possible variant antibodies, *e.g*., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CHI, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

"Bi-specific" and "multispecific" antibodies refer to antibodies that recognize more than one target. In some cases, such antibodies may comprise two or more VL and VH domains.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The terms "R-spondin translocation" and "RSPO translocation" refer herein to an R-spondin wherein a portion of a broken chromosome including, for example, R-spondin, variant, or fragment thereof or a second gene, variant, or fragment thereof, reattaches in a different chromosome location, for example, a chromosome location different from R-spondin native location or a chromosome location in and/or around the R-spondin native location which is different from the second gene's native location. The R-spondin translocation may be a RSPO1 translocation, RSPO2 translocation, RSPO3 translocation, and/or RSPO4 translocation.

The terms "R-spondin-translocation fusion polynucleotide" and "RSPO-translocation fusion polynucleotide" refer herein to the nucleic acid sequence of an R-spondin translocation gene product or fusion polynucleotide. The R-spondin-translocation fusion polynucleotide may be a RSPO1-translocation fusion polynucleotide, RSPO2-translocation fusion polynucleotide, RSPO3-translocation fusion polynucleotide, and/or RSPO4-translocation fusion polynucleotide. The terms "R-spondin-translocation fusion polypeptide" and "RSPO-translocation fusion polypeptide" refer herein to the amino acid sequence of an R-spondin translocation gene product or fusion polynucleotide. The R-spondin-translocation fusion polypeptide may be a RSPO1-translocation fusion polypeptide, RSPO2-translocation fusion polypeptide, RSPO3-translocation fusion polypeptide, and/or RSPO4-translocation fusion polypeptide.

The term "detection" includes any means of detecting, including direct and indirect detection.

The term "biomarker" as used herein refers to an indicator, *e.g*., a predictive, diagnostic, and/or prognostic indicator, which can be detected in a sample. The biomarker may serve as an indicator of a particular subtype of a disease or disorder (*e.g*., cancer) characterized by certain, molecular, pathological, histological, and/or clinical features. In some embodiments, the biomarker is a gene. In some embodiments, the biomarker is a variation (*e.g*., mutation and/or polymorphism) of a gene. In some embodiments, the biomarker is a translocation. Biomarkers include, but are not limited to, polynucleotides (*e.g*., DNA, and/or RNA), polypeptides, polypeptide and polynucleotide modifications (*e.g*., posttranslational modifications), carbohydrates, and/or glycolipid-based molecular markers.

The "presence," "amount," or "level" of a biomarker associated with an increased clinical benefit to an individual is a detectable level in a sample. These can be measured by methods known to one skilled in the art and also disclosed herein. The expression level or amount of biomarker assessed can be used to determine the response to the treatment.

The terms "level of expression" or "expression level" in general are used interchangeably and generally refer to the amount of a biomarker in a sample. "Expression" generally refers to the process by which information (*e.g*., gene-encoded and/or epigenetic) is converted into the structures present and operating in the cell. Therefore, as used herein, "expression" may refer to transcription into a polynucleotide, translation into a polypeptide, or even polynucleotide and/or polypeptide modifications (*e.g*., posttranslational modification of a polypeptide). Fragments of the transcribed polynucleotide, the translated polypeptide, or polynucleotide and/or polypeptide modifications (*e.g*., posttranslational modification of a polypeptide) shall also be regarded as expressed whether they originate from a transcript generated by alternative splicing or a degraded transcript, or from a post-translational processing of the polypeptide, *e.g*., by proteolysis. "Expressed genes" include those that are transcribed into a polynucleotide as mRNA and then translated into a polypeptide, and also those that are transcribed into RNA but not translated into a polypeptide (for example, transfer and ribosomal RNAs).

"Elevated expression," "elevated expression levels," or "elevated levels" refers to increased expression or increased levels of a biomarker in an individual relative to a control, such as an individual or individuals who are not suffering from the disease or disorder (*e.g*., cancer) or an internal control (*e.g*., housekeeping biomarker).

"Reduced expression," "reduced expression levels," or "reduced levels" refers to decrease expression or decreased levels of a biomarker in an individual relative to a control, such as an individual or individuals who are not suffering from the disease or disorder (*e.g.*, cancer) or an internal control (*e.g*., housekeeping biomarker).

The term "housekeeping biomarker" refers to a biomarker or group of biomarkers (*e.g*., polynucleotides and/or polypeptides) that are typically similarly present in all cell types. In some embodiments, the housekeeping biomarker is a "housekeeping gene." A "housekeeping gene" refers herein to a gene or group of genes which encode proteins whose activities are essential for the maintenance of cell function and which are typically similarly present in all cell types.

"Amplification," as used herein generally refers to the process of producing multiple copies of a desired sequence. "Multiple copies" mean at least two copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

The term "diagnosis" is used herein to refer to the identification or classification of a molecular or pathological state, disease or condition (*e.g*., cancer). For example, "diagnosis" may refer to identification of a particular type of cancer. "Diagnosis" may also refer to the classification of a particular subtype of cancer, *e.g.,* by histopathological criteria, or by molecular features *(e.g.,* a subtype characterized by expression of one or a combination of biomarkers (*e.g*., particular genes or proteins encoded by said genes)).

Samples include, but are not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebrospinal fluid, saliva, sputum, tears, perspiration, mucus, tumor lysates, and tissue culture medium, tissue extracts such as homogenized tissue, tumor tissue, cellular extracts, and combinations thereof.

A "reference sample", "reference cell", "reference tissue", "control sample", "control cell", or "control tissue", as used herein, refers to a sample, cell, tissue, standard, or level that is used for comparison purposes. In one embodiment, a reference sample, reference cell, reference tissue, control sample, control cell, or control tissue is obtained from a healthy and/or non-diseased part of the body (*e.g*., tissue or cells) of the same subject or individual. For example, healthy and/or non-diseased cells or tissue adjacent to the diseased cells or tissue (*e.g*., cells or tissue adjacent to a tumor). In another embodiment, a reference sample is obtained from an untreated tissue and/or cell of the body of the same subject or individual. In yet another embodiment, a reference sample, reference cell, reference tissue, control sample, control cell, or control tissue is obtained from a healthy and/or non-diseased part of the body (*e.g*., tissues or cells) of an individual who is not the subject or individual. In even another embodiment, a reference sample, reference cell, reference tissue, control sample, control cell, or control tissue is obtained from an untreated tissue and/or cell of the body of an individual who is not the subject or individual.

The phrase "substantially similar," as used herein, refers to a sufficiently high degree of similarity between two numeric values (generally one associated with a molecule and the other associated with a reference/comparator molecule) such that one of skill in the art would consider the difference between the two values to not be of statistical significance within the context of the biological characteristic measured by said values (*e.g*., Kd values). The difference between said two values may be, for example, less than about 20%, less than about 10%, and/or less than about 5% as a function of the reference/comparator value.

The phrase "substantially different," refers to a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (*e.g.*, Kd values). The difference between said two values may be, for example, greater than about 10%, greater than about 20%, greater than about 30%, greater than about 40%, and/or greater than about 50% as a function of the value for the reference/comparator molecule.

The term "cytotoxic agent" as used herein refers to a substance that inhibits or prevents a cellular function and/or causes cell death or destruction. Cytotoxic agents include, but are not limited to, radioactive isotopes (*e.g*., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (*e.g*., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

A "chemotherapeutic agent" refers to a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gammall and calicheamicin omegaI1 (see, *e.g.,* Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®), liposomal doxorubicin TLC D-99 (MYOCET®), peglylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoid, e.g., paclitaxel (TAXOL®), albumin-engineered nanoparticle formulation of paclitaxel (ABRAXANE™), and docetaxel (TAXOTERE®); chloranbucil; 6-thioguanine; mercaptopurine; methotrexate; platinum agents such as cisplatin, oxaliplatin (e.g., ELOXATIN®), and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosfamide; mitoxantrone; leucovorin; novantrone; edatrexate; daunomycin; aminopterin; ibandronate; topoisomerase inhibitor RFS 2000; difluoromethylornithine (DMFO); retinoids such as retinoic acid, including bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE® vaccine and gene therapy vaccines, for example, ALLOVECHN® vaccine, LEUVECTIN® vaccine, and VAXID® vaccine; topoisomerase 1 inhibitor (*e.g.,* LURTOTECAN®); rmRH (*e.g.,* ABARELIX®); BAY439006 (sorafenib; Bayer); SU-11248 (sunitinib, SUTENT®, Pfizer); perifosine, COX-2 inhibitor (*e.g.* celecoxib or etoricoxib), proteosome inhibitor (*e.g.* PS341); bortezomib (VELCADE®); CCI-779; tipifarnib (R11577); orafenib, ABT510; Bcl-2 inhibitor such as oblimersen sodium (GENASENSE®); pixantrone; EGFR inhibitors (see definition below); tyrosine kinase inhibitors (see definition below); serine-threonine kinase inhibitors such as rapamycin (sirolimus, RAPAMUNE®); farnesyltransferase inhibitors such as lonafarnib (SCH 6636, SARASAR™); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN™) combined with 5-FU and leucovorin.

Chemotherapeutic agents as defined herein include "anti-hormonal agents" or "endocrine therapeutics" which act to regulate, reduce, block, or inhibit the effects of hormones that can promote the growth of cancer. They may be hormones themselves, including, but not limited to: anti-estrogens with mixed agonist/antagonist profile, including, tamoxifen (NOLVADEX®), 4-hydroxytamoxifen, toremifene (FARESTON®), idoxifene, droloxifene, raloxifene (EVISTA®), trioxifene, keoxifene, and selective estrogen receptor modulators (SERMs) such as SERM3; pure anti-estrogens without agonist properties, such as fulvestrant (FASLODEX®), and EM800 (such agents may block estrogen receptor (ER) dimerization, inhibit DNA binding, increase ER turnover, and/or suppress ER levels); aromatase inhibitors, including steroidal aromatase inhibitors such as formestane and exemestane (AROMASIN®), and nonsteroidal aromatase inhibitors such as anastrazole (ARIMIDEX®), letrozole (FEMARA®) and aminoglutethimide, and other aromatase inhibitors include vorozole (RIVISOR®), megestrol acetate (MEGASE®), fadrozole, and 4(5)-imidazoles; lutenizing hormone-releaseing hormone agonists, including leuprolide (LUPRON® and ELIGARD®), goserelin, buserelin, and tripterelin; sex steroids, including progestines such as megestrol acetate and medroxyprogesterone acetate, estrogens such as diethylstilbestrol and premarin, and androgens/retinoids such as fluoxymesterone, all transretionic acid and fenretinide; onapristone; anti-progesterones; estrogen receptor down-regulators (ERDs); anti-androgens such as flutamide, nilutamide and bicalutamide; and pharmaceutically acceptable salts, acids or derivatives of any of the above; as well as combinations of two or more of the above.

The term "cytostatic agent" refers to a compound or composition which arrests growth of a cell either in vitro or in vivo. Thus, a cytostatic agent may be one which significantly reduces the percentage of cells in S phase. Further examples of cytostatic agents include agents that block cell cycle progression by inducing G0/G1 arrest or M-phase arrest. The humanized anti-Her2 antibody trastuzumab (HERCEPTIN®) is an example of a cytostatic agent that induces G0/G1 arrest. Classical M-phase blockers include the vincas (vincristine and vinblastine), taxanes, and topoisomerase II inhibitors such as doxorubicin, epirubicin, daunorubicin, etoposide, and bleomycin. Certain agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in Mendelsohn and Israel, eds., The Molecular Basis of Cancer, Chapter 1, entitled "Cell cycle regulation, oncogenes, and antineoplastic drugs" by Murakami et al. (W.B. Saunders, Philadelphia, 1995), *e.g.,* p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

As used herein, the term "EGFR inhibitor" refers to compounds that bind to or otherwise interact directly with EGFR and prevent or reduce its signaling activity, and is alternatively referred to as an "EGFR antagonist." Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn *et al*.) and variants thereof, such as chimerized 225 (C225 or Cetuximab; ERBUTIX®) and reshaped human 225 (H225) (see, WO 96/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF or Panitumumab (*see* WO98/50433, Abgenix/Amgen); EMD 55900 (Stragliotto et al. Eur. J. Cancer 32A:636-640 (1996)); EMD7200 (matuzumab) a humanized EGFR antibody directed against EGFR that competes with both EGF and TGF-alpha for EGFR binding (EMD/Merck); human EGFR antibody, HuMax-EGFR (GenMab); fully human antibodies known as E1.1, E2.4, E2.5, E6.2, E6.4, E2.11, E6. 3 and E7.6.3 and described in US 6,235,883; MDX-447 (Medarex Inc); and mAb 806 or humanized mAb 806 (Johns et al., J. Biol. Chem. 279(29):30375-30384 (2004)). The anti-EGFR antibody may be conjugated with a cytotoxic agent, thus generating an immunoconjugate (*see, e.g.,* EP659,439A2, Merck Patent GmbH). EGFR antagonists include small molecules such as compounds described in US Patent Nos: 5,616,582, 5,457,105, 5,475,001, 5,654,307, 5,679,683, 6,084,095, 6,265,410, 6,455,534, 6,521,620, 6,596,726, 6,713,484, 5,770,599, 6,140,332, 5,866,572, 6,399,602, 6,344,459, 6,602,863, 6,391,874, 6,344,455, 5,760,041, 6,002,008, and 5,747,498, as well as the following PCT publications: WO98/14451, WO98/50038, WO99/09016, and WO99/24037. Particular small molecule EGFR antagonists include OSI-774 (CP-358774, erlotinib, TARCEVA® Genentech/OSI Pharmaceuticals); PD 183805 (CI 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); ZD1839, gefitinib (IRESSA®) 4-(3'-Chloro-4'-fluoroanilino)-7-methoxy-6-(3-morpholinopropoxy)quinazoline, AstraZeneca); ZM 105180 ((6-amino-4-(3-methylphenyl-amino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4-[4-[(1-phenylethyl)amino]-1H-pyrrolo[2,3-d]pyrimidin-6-yl]-phenol); (R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl]-2-butynamide); EKB-569 (N-[4-[(3-chloro-4-fluorophenyl)amino]-3-cyano-7-ethoxy-6-quinolinyl]-4-(dimethylamino)-2-butenamide) (Wyeth); AG1478 (Pfizer); AG1571 (SU 5271; Pfizer); dual EGFR/HER2 tyrosine kinase inhibitors such as lapatinib (TYKERB®, GSK572016 or N-[3-chloro-4-[(3 fluorophenyl)methoxy]phenyl]6[5[[[2methylsulfonyl)ethyl]amino]methyl]-2-furanyl]-4-quinazolinamine; Glaxo-SmithKline).

The term "tumor" refers to all neoplastic cell growth and proliferation, whether malignant or benign, and all pre-cancerous and cancerous cells and tissues. The terms "cancer," "cancerous," "cell proliferative disorder," "proliferative disorder" and "tumor" are not mutually exclusive as referred to herein.

The terms "cell proliferative disorder" and "proliferative disorder" refer to disorders that are associated with some degree of abnormal cell proliferation. In one embodiment, the cell proliferative disorder is cancer.

The terms "cancer" and "cancerous" refer to or describe the physiological condition in mammals that is typically characterized by unregulated cell growth/proliferation. Examples of cancer include, but are not limited to, carcinoma, lymphoma (*e.g*., Hodgkin's and non-Hodgkin's lymphoma), blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

The term "colon tumor" or "colon cancer" refers to any tumor or cancer of the colon (the large intestine from the cecum to the rectum).

The term "colorectal tumor" or "colorectal cancer" refers to any tumor or cancer of the large bowel, which includes the colon (the large intestine from the cecum to the rectum) and the rectum, including, *e.g*., adenocarcinomas and less prevalent forms, such as lymphomas and squamous cell carcinomas.

An "effective amount" of an agent, *e.g.,* a pharmaceutical formulation, refers to an amount effective, at dosages and for periods of time necessary, to achieve the desired therapeutic or prophylactic result.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

An "individual" or "subject" is a mammal. Mammals include, but are not limited to, domesticated animals (*e.g.,* cows, sheep, cats, dogs, and horses), primates (*e.g.,* humans and non-human primates such as monkeys), rabbits, and rodents (*e.g.,* mice and rats). In certain embodiments, the individual or subject is a human.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

By "reduce" or "inhibit" is meant the ability to cause an overall decrease of 20%, 30%, 40%, 50%, 60%, 70%, 75%, 80%, 85%, 90%, 95%, or greater. In some embodiments, reduce or inhibit can refer to a relative reduction compared to a reference (*e.g*., reference level of biological activity (*e.g*., wnt signaling) or binding). In some embodiments, reduce or inhibit can refer to the symptoms of the disorder being treated, the presence or size of metastases, or the size of the primary tumor. In some embodiments, reduce or inhibit may apply to growth of a property (such as tumor growth) meaning that the growth is slowed or decreased.

As is understood by one skilled in the art, reference to "about" a value or parameter herein includes (and describes) embodiments that are directed to that value or parameter per se. For example, description referring to "about X" includes description of "X".

It is understood that aspect and embodiments of the invention described herein include "consisting" and/or "consisting essentially of' aspects and embodiments. As used herein, the singular form "a", "an", and "the" includes plural references unless indicated otherwise.

### II. COMPOSITIONS AND METHODS

Provided herein are anti-RSPO3 antibodies and uses thereof. Antibodies provided may be useful, *e.g.,* for the diagnosis or treatment of cancer, such as colorectal cancer.

In some embodiments, the antibodies have a higher affinity (i.e. lower Kd) for human, cynomolgus, and/or murine RSPO3 than previous anti-RSPO3 antibodies, such as anti-RSPO3 antibody 5D6 of PCT publication WO2015/058132 and/or the IgG1 antibody 131R010 of PCT publication WO2014/012007 designated "antibody A" herein. In some embodiments, the antibodies have greater potency reducing tumor growth in at least one mouse xenograft model of patient-derived RSPO3 fusion tumors than previous anti-RSPO3 antibodies, such as 5D6 and/or antibody A. In some embodiments, the antibodies have a longer half-life in vivo in mice and/or cynomolgus monkeys than previous anti-RSPO3 antibodies, such as antibody 5D6 and/or antibody A.

In some aspects, provided herein are a panel of anti-RSPO3 antibodies. For example, provided herein in some embodiments are isolated antibodies that bind to RSPO3, comprising (a) light chain variable region (VL) comprising (i) a light chain hypervariable region 1 (HVR-L1) comprising the amino acid sequence of SEQ ID NO:5, (ii) HVR-L2 comprising the amino acid sequence of SEQ ID NO:6, and (iii) HVR-L3 comprising the amino acid sequence of SEQ ID NO:7; and a heavy chain variable region (VH) comprising (i) heavy chain hypervariable region 1 (HVR-H1) comprising the amino acid sequence of SEQ ID NO:8, (ii) HVR-H2 comprising the amino acid sequence of SEQ ID NO:9, and (iii) HVR-H3 comprising the amino acid sequence of SEQ ID NO: 10; or comprising (b) a VL comprising (i) HVR-L1 comprising the amino acid sequence of SEQ ID NO: 11, (ii) HVR-L2 comprising the amino acid sequence of SEQ ID NO: 12, and (iii) HVR-L3 comprising the amino acid sequence of SEQ ID NO: 13; and a VH comprising (i) HVR-H1 comprising the amino acid sequence of SEQ ID NO:14, (ii) HVR-H2 comprising the amino acid sequence of SEQ ID NO:15, and (iii) HVR-H3 comprising the amino acid sequence of SEQ ID NO:16; or comprising (c) a VL comprising (i) HVR-L1 comprising the amino acid sequence of SEQ ID NO: 17, (ii) HVR-L2 comprising the amino acid sequence of SEQ ID NO:18, and (iii) HVR-L3 comprising the amino acid sequence of SEQ ID NO:19; and a VH comprising (i) HVR-H1 comprising the amino acid sequence of SEQ ID NO:20, (ii) HVR-H2 comprising the amino acid sequence of SEQ ID NO:21, and (iii) HVR-H3 comprising the amino acid sequence of SEQ ID NO:22.

In some embodiments, the anti-RSPO3 antibody comprises one of the following sets of light chain variable region (VL) and heavy chain variable region (VH) sequences: (a) a VL sequence comprising SEQ ID NO:23 and a VH sequence comprising SEQ ID NO:24; (b) a VL sequence comprising SEQ ID NO:25 and a VH sequence comprising SEQ ID NO:26; (c) a VL sequence comprising SEQ ID NO:27 and a VH sequence comprising SEQ ID NO:28; (d) a VL sequence comprising SEQ ID NO:29 and a VH sequence comprising SEQ ID NO:30; (e) a VL sequence comprising SEQ ID NO:31 and a VH sequence comprising SEQ ID NO:32; (f) a VL sequence comprising SEQ ID NO:33 and a VH sequence comprising SEQ ID NO:34; (g) a VL sequence comprising SEQ ID NO:35 and a VH sequence comprising SEQ ID NO:36; (h) a VL sequence comprising SEQ ID NO:37 and a VH sequence comprising SEQ ID NO:38; (i) a VL sequence comprising SEQ ID NO:39 and a VH sequence comprising SEQ ID NO:40; (j) a VL sequence comprising SEQ ID NO:41 and a VH sequence comprising SEQ ID NO:42; (k) a VL sequence comprising SEQ ID NO:43 and a VH sequence comprising SEQ ID NO:44; (l) a VL sequence comprising SEQ ID NO:45 and a VH sequence comprising SEQ ID NO:46; (m) a VL sequence comprising SEQ ID NO:47 and a VH sequence comprising SEQ ID NO:48; (n) a VL sequence comprising SEQ ID NO:49 and a VH sequence comprising SEQ ID NO:50; (o) a VL sequence comprising SEQ ID NO:51 and a VH sequence comprising SEQ ID NO:52; (p) a VL sequence comprising SEQ ID NO:53 and a VH sequence comprising SEQ ID NO:54; (q) a VL sequence comprising SEQ ID NO:55 and a VH sequence comprising SEQ ID NO:56; (r) a VL sequence comprising SEQ ID NO:57 and a VH sequence comprising SEQ ID NO:58; (w) a VL sequence comprising SEQ ID NO:59 and a VH sequence comprising SEQ ID NO:60; or (x) a VL sequence comprising SEQ ID NO:61 and a VH sequence comprising SEQ ID NO:62.

In some embodiments, the anti-RSPO3 antibody comprises one of the following sets of light and heavy chain sequences: (a) a light chain sequence comprising SEQ ID NO:63 and a heavy chain sequence comprising SEQ ID NO:64; (b) a light chain sequence comprising SEQ ID NO:65 and a heavy chain sequence comprising SEQ ID NO:66; (c) a light chain sequence comprising SEQ ID NO:67 and a heavy chain sequence comprising SEQ ID NO:68; (d) a light chain sequence comprising SEQ ID NO:69 and a heavy chain sequence comprising SEQ ID NO:70; (e) a light chain sequence comprising SEQ ID NO:71 and a heavy chain sequence comprising SEQ ID NO:72; (f) a light chain sequence comprising SEQ ID NO:73 and a heavy chain sequence comprising SEQ ID NO:74; (g) a light chain sequence comprising SEQ ID NO:75 and a heavy chain sequence comprising SEQ ID NO:76 or 171; (h) a light chain sequence comprising SEQ ID NO:77 and a heavy chain sequence comprising SEQ ID NO:78; (i) a light chain sequence comprising SEQ ID NO:79 and a heavy chain sequence comprising SEQ ID NO:80; (j) a light chain sequence comprising SEQ ID NO:81 and a heavy chain sequence comprising SEQ ID NO:82; (k) a light chain sequence comprising SEQ ID NO:83 and a heavy chain sequence comprising SEQ ID NO:84; (l) a light chain sequence comprising SEQ ID NO:85 and a heavy chain sequence comprising SEQ ID NO:86; (m) a light chain sequence comprising SEQ ID NO:87 and a heavy chain sequence comprising SEQ ID NO:88 or 172; (n) a light chain sequence comprising SEQ ID NO:89 and a heavy chain sequence comprising SEQ ID NO:90; (o) a light chain sequence comprising SEQ ID NO:91 and a heavy chain sequence comprising SEQ ID NO:92; (p) a light chain sequence comprising SEQ ID NO:93 and a heavy chain sequence comprising SEQ ID NO:94; (q) a light chain sequence comprising SEQ ID NO:95 and a heavy chain sequence comprising SEQ ID NO:96; (r) a light chain sequence comprising SEQ ID NO:97 and a heavy chain sequence comprising SEQ ID NO:98; (w) a light chain sequence comprising SEQ ID NO:99 and a heavy chain sequence comprising SEQ ID NO: 100; or (x) a light chain sequence comprising SEQ ID NO:101 and a heavy chain sequence comprising SEQ ID NO:102 or 173.

Provided herein are also isolated antibodies that bind to RSPO3 having one or more of the following characteristics: (a) binding to human RSPO3 but not human RSPO1, human RSPO2, or human RSPO4; (b) binding to human RSPO3 with a Kd of less than 0.5 nM; (c) binding to cynomolgus RSPO3 with a Kd of less than 0.5 nM; (d) binds to murine RSPO3 with a Kd of less than 0.5 nM; (e) binding to human RSPO3 with a Kd of less than 1.0 nM, binds cyno RSPO3 with a Kd of less than 1.0 nM, and binds murine RSPO3 with a Kd of less than 1.0 nM; (f) binding to human RSPO3 with a Kd of less than 0.5 nM, binds cyno RSPO3 with a Kd of less than 0.5 nM, and binds murine RSPO3 with a Kd of less than 0.5 nM; (g) having a half-life of at least 6 days following a single 10 mg/kg intravenous administration in cynomolgus monkeys; and (h) reducing tumor growth or causing tumor regression in a patient derived RSPO3 fusion xenograft model, such as a PTPRK-RSPO3 fusion model, such as a PTPRK(exon1)-RSPO3(exon2) fusion or a PTPRK(exon7)-RSPO3(exon2) fusion model. In some embodiments, the antibodies inhibit the interaction of human RSPO3 with one or more of transmembrane E3 ubiquitinase, LGR4, LGR5, and LGR6. In some of the above embodiments, the antibodies do not bind to human RSPO2. In some of the above embodiments, the antibodies bind to human RSPO3 with a Kd of less than 4 nM, such as less than 3.5 nM, such as less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, or less than 0.2 nM. In some of the above embodiments, the antibodies bind to cynomolgus RSPO3 with a Kd of less than 3.5 nM, such as less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, or less than 0.2 nM. In some embodiments, the antibodies bind to murine RSPO3 with a Kd of less than 3.5 nM, such as less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, or less than 0.2 nM. In some embodiments, the antibodies bind human RSPO3 with a Kd of less than 1.0 nM, bind cyno RSPO3 with a Kd of less than 1.0 nM, and bind murine RSPO3 with a Kd of less than 1.0 nM. In some embodiments, the antibodies bind human RSPO3 with a Kd of less than 1.0 nM, bind cyno RSPO3 with a Kd of less than 0.5 nM, and bind murine RSPO3 with a Kd of less than 1.0 nM. In some embodiments, the antibodies bind human RSPO3 with a Kd of less than 0.5 nM, bind cyno RSPO3 with a Kd of less than 0.5 nM, and bind murine RSPO3 with a Kd of less than 0.5 nM. In some embodiments, the antibodies bind human RSPO3 with a Kd of less than 0.5 nM, bind cyno RSPO3 with a Kd of less than 0.3 nM, and bind murine RSPO3 with a Kd of less than 0.5 nM. In the above embodiments, binding to RSPO3 may be determined by surface plasmon resonance (e.g. BIACORE®) assays.

In some of the above embodiments, an RSPO3 antibody may have a half-life of at least 6 days, such as at least 8 days, at least 9 days, at least 10 days, at least 11 days, or at least 12 days following a single 10 mg/kg intravenous administration in cynomolgus monkeys. In some embodiments, the antibody may have a half-life of at least 6 days, such as at least 8 days, at least 9 days, at least 10 days, at least 11 days, or at least 12 days following a single 10 mg/kg intravenous administration in cynomolgus monkeys and may bind human RSPO3 with a Kd of less than 3.5 nM, bind cyno RSPO3 with a Kd of less than 2 nM, and bind murine RSPO3 with a Kd of less than 3.5 nM.

Also provided herein are antibodies that bind human RSPO3 with a Kd of less than 1 nM, bind cyno RSPO3 with a Kd of less than 0.5 nM, and bind murine RSPO3 with a Kd of less than 1 nM; and that have a half-life of at least 6 days following a single 10 mg/kg intravenous administration in cynomolgus monkeys. In some embodiments, the antibodies may bind human RSPO3 with a Kd of less than 0.5 nM, bind cyno RSPO3 with a Kd of less than 0.5 nM, and bind murine RSPO3 with a Kd of less than 0.5 nM; and has also have a half-life of at least 6 days following a single 10 mg/kg intravenous administration in cynomolgus monkeys. In some embodiments, the antibodies may bind human RSPO3 with a Kd of less than 0.5 nM, bind cyno RSPO3 with a Kd of less than 0.3 nM, and bind murine RSPO3 with a Kd of less than 0.5 nM; and have a half-life of at least 6 days following a single 10 mg/kg intravenous administration in cynomolgus monkeys. In some of the above embodiments, the antibodies may have a half-life of at least 8 days following a single 10 mg/kg intravenous administration in cynomolgus monkeys. In some of the above embodiments, the antibodies may have a half-life of at least 10 days following a single 10 mg/kg intravenous administration in cynomolgus monkeys.

In some embodiments, the antibodies may inhibit the interaction of RSPO3 with a transmembrane E3 ubiquitinase such as ZNRF3 and/or RNF43. In some embodiments, the antibodies may inhibit the interaction of RSPO3 with one or more of ZNRF3, RNF43, LGR4, LGR5, and/or LGR6. In some embodiments, the antibodies may inhibit the interaction of RSPO3 with each of LGR4, LGR5, and RNF43. The antibodies of some embodiments may have one or more of the following properties: they may (a) inhibiting interaction of human RSPO3 and human RNF43 in a competition assay with an IC50 of 0.03 to 0.05 µg/ml, (b) inhibiting interaction of human RSPO3 and human LGR4 in a competition assay with an IC50 of 0.06 to 0.09 µg/ml, (c) inhibiting interaction of human RSPO3 and human LGR5 in a competition assay with an IC50 of 0.03 to 0.05 µg/ml, and (d) inhibiting interaction of human RSPO3 and one or more of human RNF43, LGR4, or LGR5 in a competition assay with a lower IC50 value (i.e. stronger inhibition) than that of humanized IgG1 antibody 131R010 disclosed in WO2014/014007 ("antibody A"). Examples of such antibodies may include, for example, antibodies comprising (a) light chain variable region (VL) comprising (i) a light chain hypervariable region 1 (HVR-L1) comprising the amino acid sequence of SEQ ID NO:5, (ii) HVR-L2 comprising the amino acid sequence of SEQ ID NO:6, and (iii) HVR-L3 comprising the amino acid sequence of SEQ ID NO:7; and a heavy chain variable region (VH) comprising (i) heavy chain hypervariable region 1 (HVR-H1) comprising the amino acid sequence of SEQ ID NO:8, (ii) HVR-H2 comprising the amino acid sequence of SEQ ID NO:9, and (iii) HVR-H3 comprising the amino acid sequence of SEQ ID NO: 10; or comprising (b) a VL comprising (i) HVR-L1 comprising the amino acid sequence of SEQ ID NO:11, (ii) HVR-L2 comprising the amino acid sequence of SEQ ID NO: 12, and (iii) HVR-L3 comprising the amino acid sequence of SEQ ID NO:13; and a VH comprising (i) HVR-H1 comprising the amino acid sequence of SEQ ID NO:14, (ii) HVR-H2 comprising the amino acid sequence of SEQ ID NO: 15, and (iii) HVR-H3 comprising the amino acid sequence of SEQ ID NO: 16; or comprising (c) a VL comprising (i) HVR-L1 comprising the amino acid sequence of SEQ ID NO: 17, (ii) HVR-L2 comprising the amino acid sequence of SEQ ID NO: 18, and (iii) HVR-L3 comprising the amino acid sequence of SEQ ID NO:19; and a VH comprising (i) HVR-H1 comprising the amino acid sequence of SEQ ID NO:20, (ii) HVR-H2 comprising the amino acid sequence of SEQ ID NO:21, and (iii) HVR-H3 comprising the amino acid sequence of SEQ ID NO:22.

Further examples may include antibodies that comprise one of the following sets of light chain variable region (VL) and heavy chain variable region (VH) sequences: (a) a VL sequence comprising SEQ ID NO:23 and a VH sequence comprising SEQ ID NO:24; (b) a VL sequence comprising SEQ ID NO:25 and a VH sequence comprising SEQ ID NO:26; (c) a VL sequence comprising SEQ ID NO:27 and a VH sequence comprising SEQ ID NO:28; (d) a VL sequence comprising SEQ ID NO:29 and a VH sequence comprising SEQ ID NO:30; (e) a VL sequence comprising SEQ ID NO:31 and a VH sequence comprising SEQ ID NO:32; (f) a VL sequence comprising SEQ ID NO:33 and a VH sequence comprising SEQ ID NO:34; (g) a VL sequence comprising SEQ ID NO:35 and a VH sequence comprising SEQ ID NO:36; (h) a VL sequence comprising SEQ ID NO:37 and a VH sequence comprising SEQ ID NO:38; (i) a VL sequence comprising SEQ ID NO:39 and a VH sequence comprising SEQ ID NO:40; (j) a VL sequence comprising SEQ ID NO:41 and a VH sequence comprising SEQ ID NO:42; (k) a VL sequence comprising SEQ ID NO:43 and a VH sequence comprising SEQ ID NO:44; (l) a VL sequence comprising SEQ ID NO:45 and a VH sequence comprising SEQ ID NO:46; (m) a VL sequence comprising SEQ ID NO:47 and a VH sequence comprising SEQ ID NO:48; (n) a VL sequence comprising SEQ ID NO:49 and a VH sequence comprising SEQ ID NO:50; (o) a VL sequence comprising SEQ ID NO:51 and a VH sequence comprising SEQ ID NO:52; (p) a VL sequence comprising SEQ ID NO:53 and a VH sequence comprising SEQ ID NO:54; (q) a VL sequence comprising SEQ ID NO:55 and a VH sequence comprising SEQ ID NO:56; (r) a VL sequence comprising SEQ ID NO:57 and a VH sequence comprising SEQ ID NO:58; (w) a VL sequence comprising SEQ ID NO:59 and a VH sequence comprising SEQ ID NO:60; or (x) a VL sequence comprising SEQ ID NO:61 and a VH sequence comprising SEQ ID NO:62.

Yet further examples may include antibodies that comprise one of the following sets of light and heavy chain sequences: (a) a light chain sequence comprising SEQ ID NO:63 and a heavy chain sequence comprising SEQ ID NO:64; (b) a light chain sequence comprising SEQ ID NO:65 and a heavy chain sequence comprising SEQ ID NO:66; (c) a light chain sequence comprising SEQ ID NO:67 and a heavy chain sequence comprising SEQ ID NO:68; (d) a light chain sequence comprising SEQ ID NO:69 and a heavy chain sequence comprising SEQ ID NO:70; (e) a light chain sequence comprising SEQ ID NO:71 and a heavy chain sequence comprising SEQ ID NO:72; (f) a light chain sequence comprising SEQ ID NO:73 and a heavy chain sequence comprising SEQ ID NO:74; (g) a light chain sequence comprising SEQ ID NO:75 and a heavy chain sequence comprising SEQ ID NO:76 or 171; (h) a light chain sequence comprising SEQ ID NO:77 and a heavy chain sequence comprising SEQ ID NO:78; (i) a light chain sequence comprising SEQ ID NO:79 and a heavy chain sequence comprising SEQ ID NO:80; (j) a light chain sequence comprising SEQ ID NO:81 and a heavy chain sequence comprising SEQ ID NO:82; (k) a light chain sequence comprising SEQ ID NO:83 and a heavy chain sequence comprising SEQ ID NO:84; (l) a light chain sequence comprising SEQ ID NO:85 and a heavy chain sequence comprising SEQ ID NO:86; (m) a light chain sequence comprising SEQ ID NO:87 and a heavy chain sequence comprising SEQ ID NO:88 or 172; (n) a light chain sequence comprising SEQ ID NO:89 and a heavy chain sequence comprising SEQ ID NO:90; (o) a light chain sequence comprising SEQ ID NO:91 and a heavy chain sequence comprising SEQ ID NO:92; (p) a light chain sequence comprising SEQ ID NO:93 and a heavy chain sequence comprising SEQ ID NO:94; (q) a light chain sequence comprising SEQ ID NO:95 and a heavy chain sequence comprising SEQ ID NO:96; (r) a light chain sequence comprising SEQ ID NO:97 and a heavy chain sequence comprising SEQ ID NO:98; (w) a light chain sequence comprising SEQ ID NO:99 and a heavy chain sequence comprising SEQ ID NO: 100; or (x) a light chain sequence comprising SEQ ID NO: 101 and a heavy chain sequence comprising SEQ ID NO: 102 or 173. In some embodiments, the antibody is a humanized antibody comprising a wild-type human IgG1, IgG2, IgG3, or IgG4 constant region or comprising a human IgG1, IgG2, IgG3, or IgG4 constant region comprising a substitution at Asn297 (or its equivalent residue), for example, to reduce fucosylation of the antibody. In some embodiments, the antibody heavy chain comprises an Asn297Ala or Asn297Gly mutation.

In some embodiments of any of the anti-RSPO3 antibodies, the antibody inhibits RSPO3 mediated wnt signaling, such as in a mouse cancer xenograft model, for example in a xenograft model with an RSPO3 fusion, such as a PTPRK-RSPO3 fusion, such as a PTPRK(exon1)-RSPO3(exon2) fusion or a PTPRK(exon7)-RSPO3(exon2) fusion. In some embodiment, the antibody inhibits cancer stem cell growth. In some embodiments of any of the anti-RSPO3 antibodies, the antibody induces and/or promotes cancer cell *(e.g.,* cancer stem cell) differentiation *(e.g.,* terminal differentiation and/or differentiation into progenitor cell). In some embodiments, the antibody inhibits tumor growth or promotes growth delay or stasis of a tumor or tumor regression in a mouse cancer xenograft model, for example in a xenograft model with an RSPO3 fusion, such as a PTPRK-RSPO3 fusion, such as a PTPRK(exon1)-RSPO3(exon2) fusion or a PTPRK(exon7)-RSPO3(exon2) fusion. In some embodiments, the antibody may be more potent than previously described antibodies such as antibody A at inhibiting tumor growth or causing tumor regression in the xenograft model.

In some embodiments of any of the anti-RSPO3 antibodies, the antibody is a monoclonal antibody. In some embodiments of any of the anti-RSPO3 antibodies, the antibody is a human, humanized, or chimeric antibody, or is a bi-specific or multispecific antibody. In some embodiments of any of the anti-RSPO3 antibodies, the antibody is a full length IgG1 or IgG2a antibody, or is an antigen binding fragment, such as comprising a Fab, F(ab)₂, Fv, or scFv fragment. In some embodiments of any of the anti-RSPO3 antibodies, the antibody has reduced or depleted effector function. In some embodiments of any of the anti-RSPO3 antibodies, the anti-RSPO3 antibody comprises an engineered alanine at amino acid position 297 according to EU numbering convention. In some embodiments of any of the anti-RSPO3 antibodies, the anti-RSPO3 antibody comprises an engineered alanine at amino acid position 265 according to EU numbering convention.

In some embodiments, the anti-RSPO3 antibody binds RSPO3, wherein the RSPO3 has the sequence set forth in SEQ ID NO:2. In some embodiments, the anti-RSPO3 antibody binds RSPO3, wherein the RSPO3 lacks the signaling peptide sequence (e.g., binds to amino acids within amino acids 22-272 of SEQ ID NO:2). In some embodiments, the anti-RSPO3 antibody binds to one or more furin-like cysteine-rich domains of RSPO3.

Some anti-RSPO3 antibodies of the disclosure may inhibit wnt signaling. In some embodiments, the anti-RSPO3 antibody may inhibit the interaction of RSPO3 and one or more of ZNRF3, RNF43, LGR4, LGR5, and/or LGR6. In some embodiments, the anti-RSPO3 antibody may not inhibit the interaction of RSPO3 and one or more of ZNRF3, RNF43, LGR4, LGR5, and/or LGR6 (e.g., enhances binding of RSPO3 to one or more of ZNRF3, RNF43, LGR4, LGR5, and/or LGR6). In some embodiments, the anti-RSPO3 antibody may inhibit the interaction of RSPO3 and a transmembrane E3 ubiquitinase (e.g., one or more of ZNRF3 and/or RNF43). In some embodiments, the anti-RSPO3 antibody may inhibit the interaction of RSPO3 with a syndecan (e.g., Sdc4). In some embodiments, the anti-RSPO3 antibody may inhibit the interaction of RSPO3 and one or more of LGR4, LGR5, and/or LGR6 and inhibits the interaction of RSPO3 and a transmembrane E3 ubiquitinase (e.g., one or more of ZNRF3 and/or RNF43). In some embodiments, the anti-RSPO3 antibody may inhibit the interaction of RSPO3 with all of RNF43, LGR4, and LGR5, for example, in a competition ELISA assay.

In some embodiments, the anti-RSPO3 antibody may inhibit cancer stem cell growth. In some embodiments, the anti-RSPO3 antibody may induce and/or promote cancer cell (*e.g.,* cancer stem cell) differentiation (*e.g*., terminal differentiation and/or differentiation into progenitor cell). In some embodiments, the anti-RSPO3 antibody may induce and/or promote cancer cell (*e.g.,* cancer stem cell) differentiation into a transit-amplifying cell. In some embodiments, the anti-RSPO3 antibody may induce and/or promote cancer cell (*e.g.,* cancer stem cell) differentiation into enterocyte, goblet cell, and/or enteroendocrine cell.

One skilled in the art would further appreciate that in some embodiments, the antibody could be engineered into an antibody format, in particular bispecific format, which would allow reactivity with RSPO3 and another target such as another RSPO, such as RSPO2. Anti-RSPO2/3 bispecific antibodies might have the ability to bind to RSPO2 and RSPO3, detect RSPO2and RSPO3 by IHC, inhibit the interaction of RSPO2 and RSPO3 and an LGR polypeptide, for example LGR4 and/or LGR5, inhibit the interaction of RSPO2 and RSPO3 and an E3 ubiquitinase polypeptide, for example, RNF43 and/or ZNRF3, and/or inhibit wnt signaling stimulated by RSPO2, RSPO3, RSPO2 polymorphisms, and RSPO2 translocation products.

### Monoclonal Antibody 4A6 and Certain Other Antibody Embodiments

In one aspect, the invention provides an anti-RSPO3 antibody comprising at least one, two, three, four, five, or six HVRs selected from (a) HVR-H1 comprising the amino acid sequence of SEQ ID NO:8; (b) HVR-H2 comprising the amino acid sequence of SEQ ID NO:9; (c) HVR-H3 comprising the amino acid sequence of SEQ ID NO: 10; (d) HVR-L1 comprising the amino acid sequence of SEQ ID NO:5; (e) HVR-L2 comprising the amino acid sequence of SEQ ID NO:6; and (f) HVR-L3 comprising the amino acid sequence of SEQ ID NO:7.

In one aspect, the invention provides an antibody comprising at least one, at least two, or all three VH HVR sequences selected from (a) HVR-H1 comprising the amino acid sequence of SEQ ID NO:8; (b) HVR-H2 comprising the amino acid sequence of SEQ ID NO:9; and (c) HVR-H3 comprising the amino acid sequence of SEQ ID NO: 10. In one embodiment, the antibody comprises HVR-H3 comprising the amino acid sequence of SEQ ID NO: 10. In another embodiment, the antibody comprises HVR-H3 comprising the amino acid sequence of SEQ ID NO:10 and HVR-L3 comprising the amino acid sequence of SEQ ID NO:7. In a further embodiment, the antibody comprises HVR-H3 comprising the amino acid sequence of SEQ ID NO: 10, HVR-L3 comprising the amino acid sequence of SEQ ID NO:7, and HVR-H2 comprising the amino acid sequence of SEQ ID NO:9. In a further embodiment, the antibody comprises (a) HVR-H1 comprising the amino acid sequence of SEQ ID NO:8; (b) HVR-H2 comprising the amino acid sequence of SEQ ID NO:9; and (c) HVR-H3 comprising the amino acid sequence of SEQ ID NO: 10.

In another aspect, the invention provides an antibody comprising at least one, at least two, or all three VL HVR sequences selected from (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO:7. In one embodiment, the antibody comprises (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO:7.

In another aspect, an antibody of the invention comprises (a) a VH domain comprising at least one, at least two, or all three VH HVR sequences selected from (i) HVR-H1 comprising the amino acid sequence of SEQ ID NO:8, (ii) HVR-H2 comprising the amino acid sequence of SEQ ID NO:9, and (iii) HVR-H3 comprising an amino acid sequence selected from SEQ ID NO: 10; and (b) a VL domain comprising at least one, at least two, or all three VL HVR sequences selected from (i) HVR-L1 comprising the amino acid sequence of SEQ ID NO: 5, (ii) HVR-L2 comprising the amino acid sequence of SEQ ID NO:6, and (c) HVR-L3 comprising the amino acid sequence of SEQ ID NO:7.

In another aspect, the invention provides an antibody comprising (a) HVR-H1 comprising the amino acid sequence of SEQ ID NO:8; (b) HVR-H2 comprising the amino acid sequence of SEQ ID NO:9; (c) HVR-H3 comprising the amino acid sequence of SEQ ID NO: 10; (d) HVR-L1 comprising the amino acid sequence of SEQ ID NO:5; (e) HVR-L2 comprising the amino acid sequence of SEQ ID NO:6; and (f) HVR-L3 comprising an amino acid sequence selected from SEQ ID NO:7.

In another aspect, an anti-RSPO3 antibody is provided, wherein the antibody comprises a VH as in any of the embodiments provided above, and a VL as in any of the embodiments provided above. In some embodiments, the antibody comprises any one of the following sets of light chain variable region (VL) and heavy chain variable region (VH) sequences: (a) a VL sequence comprising SEQ ID NO:23 and a VH sequence comprising SEQ ID NO:24; (b) a VL sequence comprising SEQ ID NO:25 and a VH sequence comprising SEQ ID NO:26; (c) a VL sequence comprising SEQ ID NO:27 and a VH sequence comprising SEQ ID NO:28; (d) a VL sequence comprising SEQ ID NO:29 and a VH sequence comprising SEQ ID NO:30; (e) a VL sequence comprising SEQ ID NO:31 and a VH sequence comprising SEQ ID NO:32; (f) a VL sequence comprising SEQ ID NO:33 and a VH sequence comprising SEQ ID NO:34; and (g) a VL sequence comprising SEQ ID NO:35 and a VH sequence comprising SEQ ID NO:36; including post-translational modifications of those sequences. In some embodiments, the antibody comprises any one of the following sets of light and heavy chain sequences: (a) a light chain sequence comprising SEQ ID NO:63 and a heavy chain sequence comprising SEQ ID NO:64; (b) a light chain sequence comprising SEQ ID NO:65 and a heavy chain sequence comprising SEQ ID NO:66; (c) a light chain sequence comprising SEQ ID NO:67 and a heavy chain sequence comprising SEQ ID NO:68; (d) a light chain sequence comprising SEQ ID NO:69 and a heavy chain sequence comprising SEQ ID NO:70; (e) a light chain sequence comprising SEQ ID NO:71 and a heavy chain sequence comprising SEQ ID NO:72; (f) a light chain sequence comprising SEQ ID NO:73 and a heavy chain sequence comprising SEQ ID NO:74; (g) a light chain sequence comprising SEQ ID NO:75 and a heavy chain sequence comprising SEQ ID NO:76 or 171; including post-translational modifications of those sequences. In some embodiments, the antibody is a humanized antibody comprising a wild-type human IgG1, IgG2, IgG3, or IgG4 constant region or comprising a human IgG1, IgG2, IgG3, or IgG4 constant region comprising a substitution at Asn297 (or its equivalent residue), for example, to reduce fucosylation of the antibody. In some embodiments, the antibody heavy chain comprises an Asn297Ala or Asn297Gly mutation.

### Monoclonal Antibody 11C10 and Certain Other Antibody Embodiments

In one aspect, the invention provides an anti-RSPO3 antibody comprising at least one, two, three, four, five, or six HVRs selected from (a) HVR-H1 comprising the amino acid sequence of SEQ ID NO:14; (b) HVR-H2 comprising the amino acid sequence of SEQ ID NO: 15; (c) HVR-H3 comprising the amino acid sequence of SEQ ID NO: 16; (d) HVR-L1 comprising the amino acid sequence of SEQ ID NO: 11; (e) HVR-L2 comprising the amino acid sequence of SEQ ID NO: 12; and (f) HVR-L3 comprising the amino acid sequence of SEQ ID NO: 13.

In one aspect, the invention provides an antibody comprising at least one, at least two, or all three VH HVR sequences selected from (a) HVR-H1 comprising the amino acid sequence of SEQ ID NO:14; (b) HVR-H2 comprising the amino acid sequence of SEQ ID NO: 15; and (c) HVR-H3 comprising the amino acid sequence of SEQ ID NO: 16. In one embodiment, the antibody comprises HVR-H3 comprising the amino acid sequence of SEQ ID NO: 16. In another embodiment, the antibody comprises HVR-H3 comprising the amino acid sequence of SEQ ID NO: 16 and HVR-L3 comprising the amino acid sequence of SEQ ID NO: 13. In a further embodiment, the antibody comprises HVR-H3 comprising the amino acid sequence of SEQ ID NO: 16, HVR-L3 comprising the amino acid sequence of SEQ ID NO: 13, and HVR-H2 comprising the amino acid sequence of SEQ ID NO: 15. In a further embodiment, the antibody comprises (a) HVR-H1 comprising the amino acid sequence of SEQ ID NO: 14; (b) HVR-H2 comprising the amino acid sequence of SEQ ID NO: 15; and (c) HVR-H3 comprising the amino acid sequence of SEQ ID NO: 16.

In another aspect, the invention provides an antibody comprising at least one, at least two, or all three VL HVR sequences selected from (a) HVR-L1 comprising the amino acid sequence of SEQ ID NO:11; (b) HVR-L2 comprising the amino acid sequence of SEQ ID NO: 12; and (c) HVR-L3 comprising the amino acid sequence of SEQ ID NO: 13. In one embodiment, the antibody comprises (a) HVR-L1 comprising the amino acid sequence of SEQ ID NO: 11; (b) HVR-L2 comprising the amino acid sequence of SEQ ID NO: 12; and (c) HVR-L3 comprising the amino acid sequence of SEQ ID NO: 13.

In another aspect, an antibody of the invention comprises (a) a VH domain comprising at least one, at least two, or all three VH HVR sequences selected from (i) HVR-H1 comprising the amino acid sequence of SEQ ID NO: 14, (ii) HVR-H2 comprising the amino acid sequence of SEQ ID NO: 15, and (iii) HVR-H3 comprising an amino acid sequence selected from SEQ ID NO: 16; and (b) a VL domain comprising at least one, at least two, or all three VL HVR sequences selected from (i) HVR-L1 comprising the amino acid sequence of SEQ ID NO: 11, (ii) HVR-L2 comprising the amino acid sequence of SEQ ID NO: 12, and (c) HVR-L3 comprising the amino acid sequence of SEQ ID NO:13.

In another aspect, the invention provides an antibody comprising (a) HVR-H1 comprising the amino acid sequence of SEQ ID NO: 14; (b) HVR-H2 comprising the amino acid sequence of SEQ ID NO: 15; (c) HVR-H3 comprising the amino acid sequence of SEQ ID NO: 16; (d) HVR-L1 comprising the amino acid sequence of SEQ ID NO: 11; (e) HVR-L2 comprising the amino acid sequence of SEQ ID NO: 12; and (f) HVR-L3 comprising an amino acid sequence selected from SEQ ID NO:13.

In another aspect, an anti-RSPO3 antibody is provided, wherein the antibody comprises a VH as in any of the embodiments provided above, and a VL as in any of the embodiments provided above. In some embodiments, the antibody comprises any one of the following sets of light chain variable region (VL) and heavy chain variable region (VH) sequences: (a) a VL sequence comprising SEQ ID NO:37 and a VH sequence comprising SEQ ID NO:38; (b) a VL sequence comprising SEQ ID NO:39 and a VH sequence comprising SEQ ID NO:40; (c) a VL sequence comprising SEQ ID NO:41 and a VH sequence comprising SEQ ID NO:42; (d) a VL sequence comprising SEQ ID NO:43 and a VH sequence comprising SEQ ID NO:44; (e) a VL sequence comprising SEQ ID NO:45 and a VH sequence comprising SEQ ID NO:46; and (f) a VL sequence comprising SEQ ID NO:47 and a VH sequence comprising SEQ ID NO:48; including post-translational modifications of those sequences. In some embodiments, the antibody comprises any one of the following sets of light and heavy chain sequences: (a) a light chain sequence comprising SEQ ID NO:77 and a heavy chain sequence comprising SEQ ID NO:78; (b) a light chain sequence comprising SEQ ID NO:79 and a heavy chain sequence comprising SEQ ID NO:80; (c) a light chain sequence comprising SEQ ID NO:81 and a heavy chain sequence comprising SEQ ID NO:82; (d) a light chain sequence comprising SEQ ID NO:83 and a heavy chain sequence comprising SEQ ID NO:84; (e) a light chain sequence comprising SEQ ID NO:85 and a heavy chain sequence comprising SEQ ID NO:86; and (f) a light chain sequence comprising SEQ ID NO:87 and a heavy chain sequence comprising SEQ ID NO:88 or 172; including post-translational modifications of those sequences. In some embodiments, the antibody is a humanized antibody comprising a wild-type human IgG1, IgG2, IgG3, or IgG4 constant region or comprising a human IgG1, IgG2, IgG3, or IgG4 constant region comprising a substitution at Asn297 (or its equivalent residue), for example, to reduce fucosylation of the antibody. In some embodiments, the antibody heavy chain comprises an Asn297Ala or Asn297Gly mutation.

### Monoclonal Antibody 15F3 and Certain Other Antibody Embodiments

In one aspect, the invention provides an anti-RSPO3 antibody comprising at least one, two, three, four, five, or six HVRs selected from (a) HVR-H1 comprising the amino acid sequence of SEQ ID NO:20; (b) HVR-H2 comprising the amino acid sequence of SEQ ID NO:21; (c) HVR-H3 comprising the amino acid sequence of SEQ ID NO:22; (d) HVR-L1 comprising the amino acid sequence of SEQ ID NO: 17; (e) HVR-L2 comprising the amino acid sequence of SEQ ID NO: 18; and (f) HVR-L3 comprising the amino acid sequence of SEQ ID NO: 19.

In one aspect, the invention provides an antibody comprising at least one, at least two, or all three VH HVR sequences selected from (a) HVR-H1 comprising the amino acid sequence of SEQ ID NO:20; (b) HVR-H2 comprising the amino acid sequence of SEQ ID NO:21; and (c) HVR-H3 comprising the amino acid sequence of SEQ ID NO:22. In one embodiment, the antibody comprises HVR-H3 comprising the amino acid sequence of SEQ ID NO:22. In another embodiment, the antibody comprises HVR-H3 comprising the amino acid sequence of SEQ ID NO:22 and HVR-L3 comprising the amino acid sequence of SEQ ID NO: 19. In a further embodiment, the antibody comprises HVR-H3 comprising the amino acid sequence of SEQ ID NO:22, HVR-L3 comprising the amino acid sequence of SEQ ID NO: 19, and HVR-H2 comprising the amino acid sequence of SEQ ID NO:21. In a further embodiment, the antibody comprises (a) HVR-H1 comprising the amino acid sequence of SEQ ID NO:20; (b) HVR-H2 comprising the amino acid sequence of SEQ ID NO:21; and (c) HVR-H3 comprising the amino acid sequence of SEQ ID NO:22.

In another aspect, the invention provides an antibody comprising at least one, at least two, or all three VL HVR sequences selected from (a) HVR-L1 comprising the amino acid sequence of SEQ ID NO: 17; (b) HVR-L2 comprising the amino acid sequence of SEQ ID NO: 18; and (c) HVR-L3 comprising the amino acid sequence of SEQ ID NO: 19. In one embodiment, the antibody comprises (a) HVR-L1 comprising the amino acid sequence of SEQ ID NO: 17; (b) HVR-L2 comprising the amino acid sequence of SEQ ID NO: 18; and (c) HVR-L3 comprising the amino acid sequence of SEQ ID NO:19.

In another aspect, an antibody of the invention comprises (a) a VH domain comprising at least one, at least two, or all three VH HVR sequences selected from (i) HVR-H1 comprising the amino acid sequence of SEQ ID NO:20, (ii) HVR-H2 comprising the amino acid sequence of SEQ ID NO:21, and (iii) HVR-H3 comprising an amino acid sequence selected from SEQ ID NO:22; and (b) a VL domain comprising at least one, at least two, or all three VL HVR sequences selected from (i) HVR-L1 comprising the amino acid sequence of SEQ ID NO: 17, (ii) HVR-L2 comprising the amino acid sequence of SEQ ID NO: 18, and (c) HVR-L3 comprising the amino acid sequence of SEQ ID NO:19.

In another aspect, the invention provides an antibody comprising (a) HVR-H1 comprising the amino acid sequence of SEQ ID NO:20; (b) HVR-H2 comprising the amino acid sequence of SEQ ID NO:21; (c) HVR-H3 comprising the amino acid sequence of SEQ ID NO:22; (d) HVR-L1 comprising the amino acid sequence of SEQ ID NO: 17; (e) HVR-L2 comprising the amino acid sequence of SEQ ID NO: 18; and (f) HVR-L3 comprising an amino acid sequence selected from SEQ ID NO:19.

In another aspect, an anti-RSPO3 antibody is provided, wherein the antibody comprises a VH as in any of the embodiments provided above, and a VL as in any of the embodiments provided above. In some embodiments, the antibody comprises any one of the following sets of light chain variable region (VL) and heavy chain variable region (VH) sequences: (a) a VL sequence comprising SEQ ID NO:49 and a VH sequence comprising SEQ ID NO:50; (b) a VL sequence comprising SEQ ID NO:51 and a VH sequence comprising SEQ ID NO:52; (c) a VL sequence comprising SEQ ID NO:53 and a VH sequence comprising SEQ ID NO:54; (d) a VL sequence comprising SEQ ID NO:55 and a VH sequence comprising SEQ ID NO:56; (e) a VL sequence comprising SEQ ID NO:57 and a VH sequence comprising SEQ ID NO:58; (f) a VL sequence comprising SEQ ID NO:59 and a VH sequence comprising SEQ ID NO:60; and (g) a VL sequence comprising SEQ ID NO:61 and a VH sequence comprising SEQ ID NO:62; including post-translational modifications of those sequences. In some embodiments, the antibody comprises any one of the following sets of light and heavy chain sequences: (a) a light chain sequence comprising SEQ ID NO:89 and a heavy chain sequence comprising SEQ ID NO:90; (b) a light chain sequence comprising SEQ ID NO:91 and a heavy chain sequence comprising SEQ ID NO:92; (c) a light chain sequence comprising SEQ ID NO:93 and a heavy chain sequence comprising SEQ ID NO:94; (d) a light chain sequence comprising SEQ ID NO:95 and a heavy chain sequence comprising SEQ ID NO:96; (e) a light chain sequence comprising SEQ ID NO:97 and a heavy chain sequence comprising SEQ ID NO:98; (f) a light chain sequence comprising SEQ ID NO:99 and a heavy chain sequence comprising SEQ ID NO:100; and (g) a light chain sequence comprising SEQ ID NO:101 and a heavy chain sequence comprising SEQ ID NO: 102 or 173; including post-translational modifications of those sequences. In some embodiments, the antibody is a humanized antibody comprising a wild-type human IgG1, IgG2, IgG3, or IgG4 constant region or comprising a human IgG1, IgG2, IgG3, or IgG4 constant region comprising a substitution at Asn297 (or its equivalent residue), for example, to reduce fucosylation of the antibody. In some embodiments, the antibody heavy chain comprises an Asn297Ala or Asn297Gly mutation.

### Further Exemplary Characteristics of Certain Antibody Embodiments

In any of the above embodiments, an anti-RSPO3 antibody may be humanized. In one embodiment, an anti-RSPO3 antibody comprises HVRs as in any of the above embodiments, and further comprises a human acceptor framework, *e.g.* a human immunoglobulin framework or a human consensus framework. In certain embodiments, the human acceptor framework is the human VL kappa I consensus (VL_{KI}) framework and/or the VH framework VH₁. In certain embodiments, the human acceptor framework is the human VL kappa I consensus (VL_{KI}) framework and/or the VH framework VH₁ comprising any one of the following mutations.

In another aspect, an anti-RSPO3 antibody may comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:24, 26, 28 30, 32, 34, 36, 38, 40, 42, 44, 46, 48, 50, 52, 54, 56, 58, 60, or 62. In certain embodiments, a VH sequence having at least 90%, 91%, 92%, 93%, 94%, 95%, 96%, 97%, 98%, or 99% identity to the amino acid sequence of SEQ ID NO:24, 26, 28 30, 32, 34, 36, 38, 40, 42, 44, 46, 48, 50, 52, 54, 56, 58, 60, or 62 contains substitutions (e.g., conservative substitutions), insertions, or deletions relative to the reference sequence, but an anti-RSPO3 antibody comprising that sequence retains the ability to bind to RSPO3. In certain embodiments, a total of 1 to 10 amino acids have been substituted, inserted and/or deleted in SEQ ID NO:24, 26, 28 30, 32, 34, 36, 38, 40, 42, 44, 46, 48, 50, 52, 54, 56, 58, 60, or 62. In certain embodiments, a total of 1 to 5 amino acids have been substituted, inserted and/or deleted in SEQ ID NO:24, 26, 28 30, 32, 34, 36, 38, 40, 42, 44, 46, 48, 50, 52, 54, 56, 58, 60, or 62. In certain embodiments, substitutions, insertions, or deletions occur in regions outside the HVRs (*i.e.,* in the FRs). Optionally, the anti-RSPO3 antibody comprises the VH sequence of SEQ ID NO:24, 26, 28 30, 32, 34, 36, 38, 40, 42, 44, 46, 48, 50, 52, 54, 56, 58, 60, or 62, including post-translational modifications of that sequence.

In another aspect, an anti-RSPO3 antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:23, 25, 27, 29, 31, 33, 35, 37, 39, 41, 43, 45, 47, 49, 51, 53, 55, 57, 59, or 61. In certain embodiments, a VL sequence having at least 90%, 91%, 92%, 93%, 94%, 95%, 96%, 97%, 98%, or 99% identity to the amino acid sequence of SEQ ID NO:23, 25, 27, 29, 31, 33, 35, 37, 39, 41, 43, 45, 47, 49, 51, 53, 55, 57, 59, or 61 contains substitutions (*e.g*., conservative substitutions), insertions, or deletions relative to the reference sequence, but an anti-RSPO3 antibody comprising that sequence retains the ability to bind to RSPO3. In certain embodiments, a total of 1 to 10 amino acids have been substituted, inserted and/or deleted in SEQ ID NO:23, 25, 27, 29, 31, 33, 35, 37, 39, 41, 43, 45, 47, 49, 51, 53, 55, 57, 59, or 61. In certain embodiments, a total of 1 to 5 amino acids have been substituted, inserted and/or deleted in SEQ ID NO:23, 25, 27, 29, 31, 33, 35, 37, 39, 41, 43, 45, 47, 49, 51, 53, 55, 57, 59, or 61. In certain embodiments, the substitutions, insertions, or deletions occur in regions outside the HVRs (*i.e.,* in the FRs). Optionally, the anti-RSPO3 antibody comprises the VL sequence of SEQ ID NO:23, 25, 27, 29, 31, 33, 35, 37, 39, 41, 43, 45, 47, 49, 51, 53, 55, 57, 59, or 61, including post-translational modifications of that sequence.

In a further aspect, provided are herein are antibodies that bind to the same epitope as an anti-RSPO3 antibody provided herein, such as the 4A6, 11C10, or 15F3 antibodies described in the subsections above. For example, in certain embodiments, an antibody is provided that binds to the same epitope as an anti-RSPO3 antibody comprising a VH and VL sequences of SEQ ID NO: 23 and 24, 25 and 26, 27 and 28, 29 and 30, 31 and 32, 33 and 34, 35 and 36, 37 and 38, 39 and 40, 41 and 42, 43 and 44, 45 and 46, 47 and 48, 49 and 50, 51 and 52, 53 and 54, 55 and 56, 57 and 58, 59 and 60, or 61 and 62, respectively. In some embodiments, the epitope is determined by crystallography.

Thus, accordingly, the VH and VL or heavy and light chain sequences of the 4A6, 11C10, or 15F3 antibodies may contain substitutions, insertions, or deletions as described above. In some embodiments, the resulting VH or VL or heavy or light chain sequence is at least 90%, 91%, 92%, 93%, 94%, 95%, 96%, 97%, 98%, or 99% identical to the above-listed VH, VL, heavy, or light chain sequences of 4A6, 11C10, or 15F3 as listed above. Examples of such sequences are provided in Table 6, which follows the working examples section below.

In a further aspect of the invention, an anti-RSPO3 antibody according to any of the above embodiments is a monoclonal antibody, including a human antibody. In one embodiment, an anti-RSPO3 antibody is an antibody fragment, *e.g*., a Fv, Fab, Fab', scFv, diabody, or F(ab')₂ fragment. In another embodiment, the antibody is a substantially full length antibody, *e.g*., an IgG1 antibody, IgG2a antibody or other antibody class or isotype as defined herein.

In a further aspect, an anti-RSPO3 antibody according to any of the above embodiments may incorporate any of the features, singly or in combination, as described in the sections that follow below.

### 1. Antibody Affinity

In certain embodiments, an antibody provided herein has a dissociation constant (Kd) for human RSPO3 of ≤ 1µM, ≤ 100 nM, ≤ 10 nM, ≤ 1 nM, ≤ 0.1 nM, ≤ 0.01 nM, or ≤ 0.001 nM (*e.g.* 10⁻⁸ M or less, *e.g.* from 10⁻⁸ M to 10⁻¹³ M, *e.g.,* from 10⁻⁹ M to 10⁻¹³ M). In certain embodiments, the Kd is less than 5 nM, such as less than 4nM, such as less than 3.5 nM, such as less than 3 nM, such as less than 2 nM, less than 1 nM, less than 0.9 nM, less than 0.8 nM, less than 0.7 nM, less than 0.6 nM, less than 0.5 nM, less than 0.4 nM, less than 0.3nM, less than 0.2nM, or less than 0.1nM. In certain embodiments, an antibody provided herein has a dissociation constant (Kd) for cynomolgus RSPO3 of ≤ 1µM, ≤ 100 nM, ≤ 10 nM, ≤ 1 nM, ≤ 0.1 nM, ≤ 0.01 nM, or ≤ 0.001 nM (*e.g.* 10⁻⁸ M or less, *e.g.* from 10⁻⁸ M to 10⁻¹³ M, *e.g.,* from 10⁻⁹ M to 10⁻¹³ M). In certain embodiments, the Kd is less than 5 nM, such as less than 4nM, such as less than 3 nM, such as less than 2 nM, less than 1 nM, less than 0.9 nM, less than 0.8 nM, less than 0.7 nM, less than 0.6 nM, less than 0.5 nM, less than 0.4 nM, less than 0.3nM, less than 0.2nM, or less than 0.1nM. In certain embodiments, an antibody provided herein has a dissociation constant (Kd) for murine RSPO3 of ≤ 1µM, ≤ 100 nM, ≤ 10 nM, ≤ 1 nM, ≤ 0.1 nM, ≤ 0.01 nM, or ≤ 0.001 nM (*e.g.* 10⁻⁸ M or less, *e.g.* from 10⁻⁸ M to 10⁻¹³ M, *e.g.,* from 10⁻⁹ M to 10⁻¹³ M). In certain embodiments, the Kd is less than 5 nM, such as less than 4nM, such as less than 3 nM, such as less than 2 nM, less than 1 nM, less than 0.9 nM, less than 0.8 nM, less than 0.7 nM, less than 0.6 nM, less than 0.5 nM, less than 0.4 nM, less than 0.3nM, less than 0.2nM, or less than 0. 1nM.In some embodiments, the antibodies bind to human RSPO3 with a Kd of less than 4 nM, such as less than 3.5 nM, such as less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, or less than 0.2 nM. In some of the above embodiments, the antibodies bind to cynomolgus RSPO3 with a Kd of less than 3.5 nM, such as less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, or less than 0.2 nM. In some embodiments, the antibodies bind to murine RSPO3 with a Kd of less than 3.5 nM, such as less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, or less than 0.2 nM. In some embodiments, the antibodies bind human RSPO3 with a Kd of less than 1.0 nM, bind cyno RSPO3 with a Kd of less than 1.0 nM, and bind murine RSPO3 with a Kd of less than 1.0 nM. In some embodiments, the antibodies bind human RSPO3 with a Kd of less than 1.0 nM, bind cyno RSPO3 with a Kd of less than 0.5 nM, and bind murine RSPO3 with a Kd of less than 1.0 nM. In some embodiments, the antibodies bind human RSPO3 with a Kd of less than 0.5 nM, bind cyno RSPO3 with a Kd of less than 0.5 nM, and bind murine RSPO3 with a Kd of less than 0.5 nM. In some embodiments, the antibodies bind human RSPO3 with a Kd of less than 0.5 nM, bind cyno RSPO3 with a Kd of less than 0.3 nM, and bind murine RSPO3 with a Kd of less than 0.5 nM.

A Kd may be measured, for example, by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (*see, e.g.,* Chen et al., J. Mol. Biol. 293:865-881(1999)). To establish conditions for the assay, MICROTITER® multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (*e.g.,* consistent with assessment of the anti-VEGF antibody, Fab-12, in Presta et al., Cancer Res. 57:4593-4599 (1997)). The Fab of interest is then incubated overnight; however, the incubation may continue for a longer period (*e.g*., about 65 hours) to ensure that equilibrium is reached. Thereafter, the mixtures are transferred to the capture plate for incubation at room temperature (*e.g.,* for one hour). The solution is then removed and the plate washed eight times with 0.1% polysorbate 20 (TWEEN-20®) in PBS. When the plates have dried, 150 (µl/well of scintillant (MICROSCINT-20 ™; Packard) is added, and the plates are counted on a TOPCOUNT ™ gamma counter (Packard) for ten minutes. Concentrations of each Fab that give less than or equal to 20% of maximal binding are chosen for use in competitive binding assays.

A Kd may also be measured using a BIACORE® surface plasmon resonance assay. For example, an assay using a BIACORE®-2000 or a BIACORE ®-3000 (BIAcore, Inc., Piscataway, NJ) is performed at 25°C with immobilized antigen CM5 chips at ∼10 response units (RU). In one embodiment, carboxymethylated dextran biosensor chips (CM5, BIACORE, Inc.) are activated with *N*-ethyl-*N'*-(3-dimethylaminopropyl)-carbodiimide hydrochloride (EDC) and *N-*hydroxysuccinimide (NHS) according to the supplier's instructions. Antigen is diluted with 10 mM sodium acetate, pH 4.8, to 5 µg/ml (∼0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 (µl/min. Association rates (kₒₙ) and dissociation rates (k_{off}) are calculated using a simple one-to-one Langmuir binding model (BIACORE ® Evaluation Software version 3.2) by simultaneously fitting the association and dissociation sensorgrams. The equilibrium dissociation constant (Kd) is calculated as the ratio k_{off}/kₒₙ. *See, e.g.,* Chen et al., J. Mol. Biol. 293:865-881 (1999). If the on-rate exceeds 106 M-1 s-1 by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO ™ spectrophotometer (ThermoSpectronic) with a stirred cuvette.

### 2. Antibody Fragments

In certain embodiments, an antibody provided herein is an antibody fragment. Antibody fragments include, but are not limited to, Fab, Fab', Fab'-SH, F(ab')₂, Fv, and scFv fragments, and other fragments described below. For a review of certain antibody fragments, *see* Hudson et al. Nat. Med. 9:129-134 (2003). For a review of scFv fragments, *see, e.g.,* Pluckthün, in The Pharmacology of Monoclonal Antibodies, vol. 113, Rosenburg and Moore eds., (Springer-Verlag, New York), pp. 269-315 (1994); *see also* WO 93/16185; and U.S. Patent Nos. 5,571,894 and 5,587,458. For discussion of Fab and F(ab')₂ fragments comprising salvage receptor binding epitope residues and having increased *in vivo* half-life, *see* U.S. Patent No. 5,869,046.

Diabodies are antibody fragments with two antigen-binding sites that may be bivalent or bispecific. *See,* for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see, e.g.,* U.S. Patent No. 6,248,516).

Antibody fragments can be made by various techniques, including but not limited to proteolytic digestion of an intact antibody as well as production by recombinant host cells (*e.g. E. coli* or phage), as described herein.

### 3. Chimeric and Humanized Antibodies

In certain embodiments, an antibody provided herein is a chimeric antibody. Certain chimeric antibodies are described, *e.g.,* in U.S. Patent No. 4,816,567; and Morrison et al., Proc. Natl. Acad. Sci. USA, 81:6851-6855 (1984)). In one example, a chimeric antibody comprises a non-human variable region (*e.g.,* a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, *e.g*., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (*e.g*., the antibody from which the HVR residues are derived), *e.g.,* to restore or improve antibody specificity or affinity.

Humanized antibodies and methods of making them are reviewed, *e.g*., in Almagro and Fransson, Front. Biosci. 13:1619-1633 (2008), and are further described, *e.g.,* in Riechmann et al., Nature 332:323-329 (1988); Queen et al., Proc. Nat'lAcad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (*see, e.g.,* Sims et al. J. Immunol. 151:2296 (1993)); framework regions derived from the consensus sequence of human antibodies of a particular subgroup of light or heavy chain variable regions (*see, e.g.,* Carter et al. Proc. Natl. Acad. Sci. USA, 89:4285 (1992); and Presta et al. J. Immunol., 151:2623 (1993)); human mature (somatically mutated) framework regions or human germline framework regions *(see, e.g.,* Almagro and Fransson, Front. Biosci. 13:1619-1633 (2008)); and framework regions derived from screening FR libraries *(see, e.g.,* Baca et al., J. Biol. Chem. 272:10678-10684 (1997) and Rosok et al., J. Biol. Chem. 271:22611-22618 (1996)).

In some embodiments, the humanized antibodies may comprise a human IgG1, IgG2, IgG3, or IgG4 heavy chain constant region.

### 4. Human Antibodies

In certain embodiments, an antibody provided herein is a human antibody. Human antibodies can be produced using various techniques known in the art. Human antibodies are described generally in vanDijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, *see* Lonberg, Nat. Biotech. 23:1117-1125 (2005). *See also, e.g.,* U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, *e.g*., by combining with a different human constant region.

Human antibodies can also be made by hybridoma-based methods. Human myeloma and mouse-human heteromyeloma cell lines for the production of human monoclonal antibodies have been described. (*See, e.g.,* Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3): 185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, *e.g.,* in Hoogenboom et al. in Methods in Molecular Biology 178:1-37 (O'Brien et al., ed., Human Press, Totowa, NJ, 2001) and further described, *e.g.,* in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Bi-Specific and Multispecific Antibodies

In certain embodiments, an antibody provided herein is a multispecific antibody, for example, a bispecific antibody. Multispecific antibodies are monoclonal antibodies that have binding specificities for at least two different sites. In certain embodiments, one of the binding specificities is RSPO3 and the other is for any other antigen. In certain embodiments, bispecific antibodies may bind to two different epitopes of RSPO3. Bispecific antibodies may also be used to localize cytotoxic agents to cells which express RSPO3. In some embodiments, the multispecific antibody binds to RSPO2 and RSPO3. In some embodiments, the multispecific antibody (e.g., bispecific antibody) comprises a first variable domain comprising the HVRs of 4A6 and a second variable domain comprising the HVRs of 15F3 or 11C10. Bispecific antibodies can be prepared as full length antibodies or antibody fragments.

Techniques for making multispecific antibodies include, but are not limited to, recombinant co-expression of two immunoglobulin heavy chain-light chain pairs having different specificities (*see* Milstein and Cuello, Nature 305: 537 (1983)), WO 93/08829, and Traunecker et al., EMBOJ. 10: 3655 (1991)), and "knob-in-hole" engineering *(see, e.g.,* U.S. Patent No. 5,731,168). Multi-specific antibodies may also be made by engineering electrostatic steering effects for making antibody Fc-heterodimeric molecules (WO 2009/089004A1); cross-linking two or more antibodies or fragments *(see, e.g.,* US Patent No. 4,676,980, and Brennan et al., Science, 229: 81 (1985)); using leucine zippers to produce bi-specific antibodies *(see, e.g.,* Kostelny et al., J. Immunol., 148(5):1547-1553 (1992)); using "diabody" technology for making bispecific antibody fragments (*see, e.g.,* Hollinger et al., Proc. Natl. Acad. Sci. USA, 90:6444-6448 (1993)); and using single-chain Fv (sFv) dimers (*see, e.g.* Gruber et al., J. Immunol., 152:5368 (1994)); and preparing trispecific antibodies as described, *e.g.,* in Tutt et al. J. Immunol. 147: 60 (1991).

Engineered antibodies with three or more functional antigen binding sites, including "Octopus antibodies," are also included herein (*see, e.g.* US 2006/0025576).

The antibody or fragment herein also includes a "Dual Acting FAb" or "DAF" comprising an antigen binding site that binds to multiple RSPOs (*e.g.,* RSPO2 and/or RSPO3) *(see,* US 2008/0069820, for example).

### 7. Antibody Variants

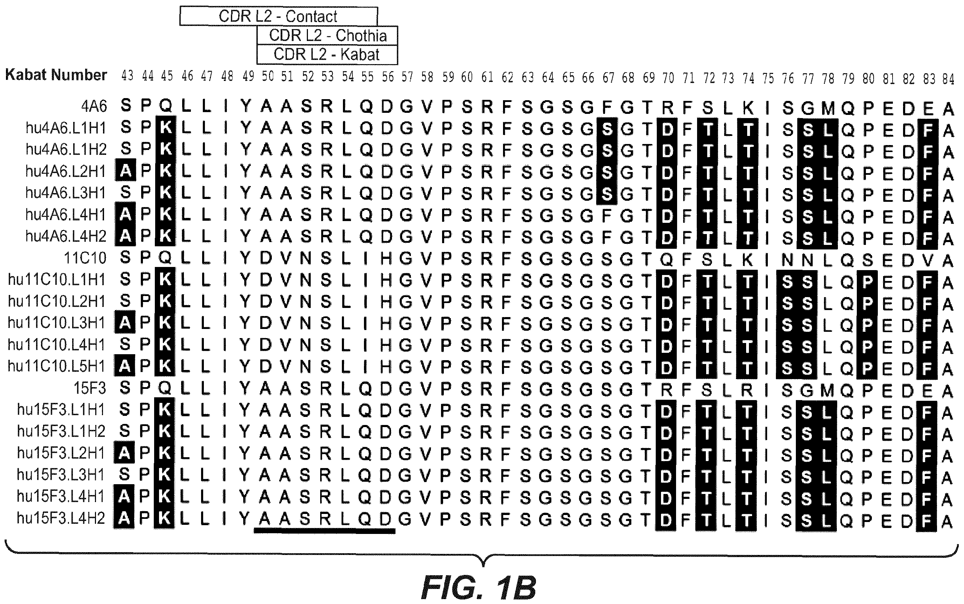

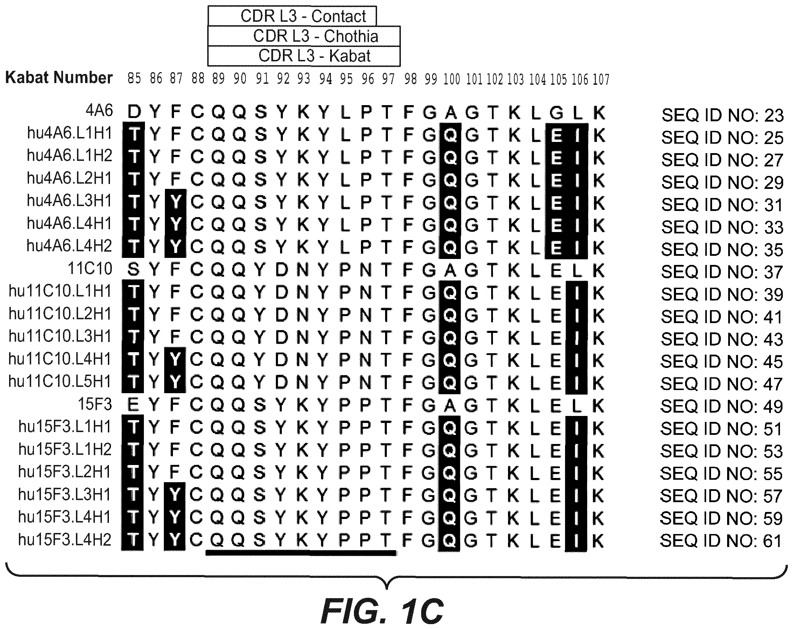

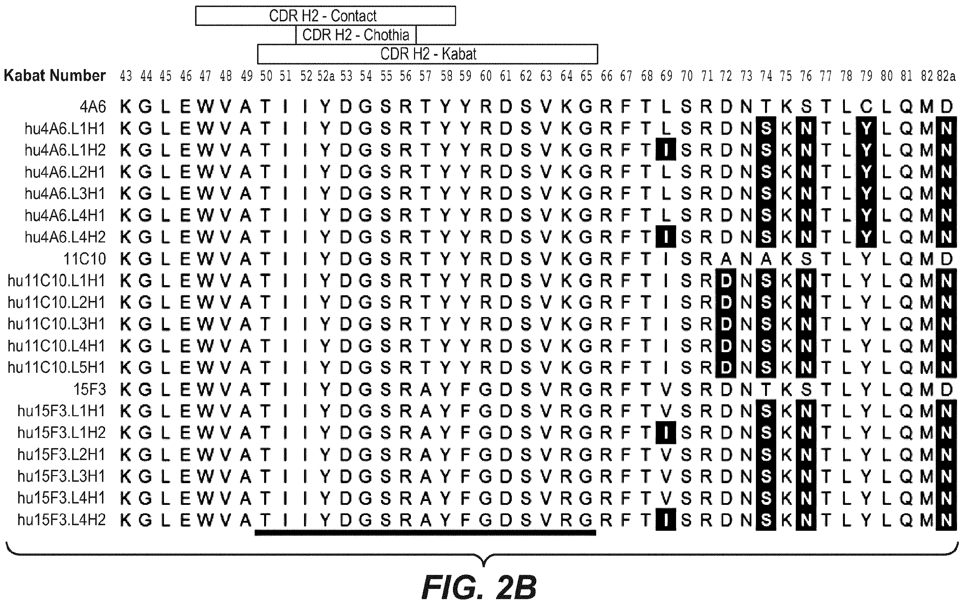

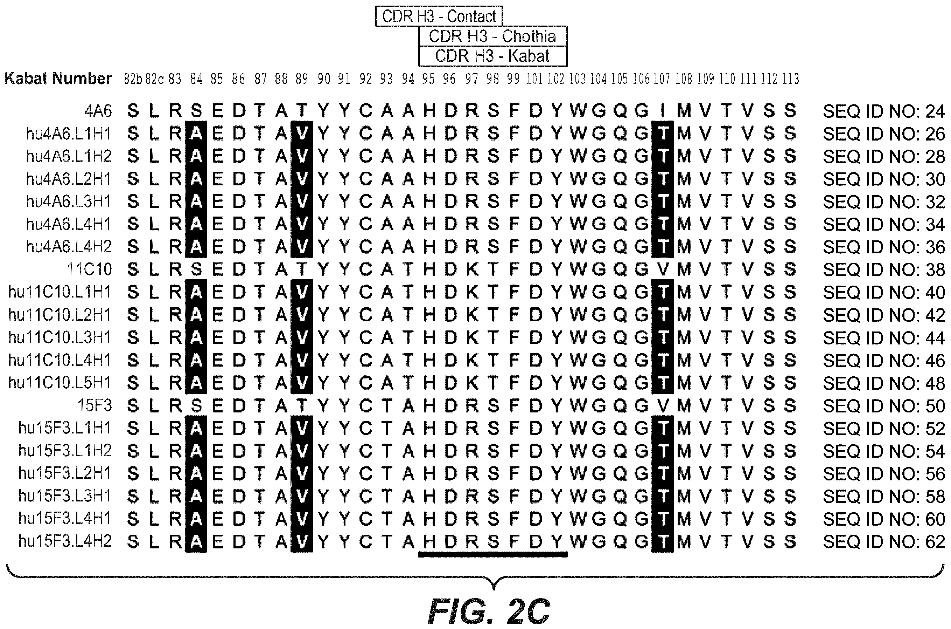

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, *e.g*., antigen-binding. Specific examples of humanized variants that were generated are described Figs. 1 and 2 herein and in Table 6 following the working examples section below.

### a) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, *e.g*., retained/improved antigen binding, decreased immunogenicity, or improved ADCC or CDC.

**TABLE 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g*. a humanized or human antibody). Generally, the resulting variant(s) selected for further study will have modifications (*e.g*., improvements) in certain biological properties (*e.g*., increased affinity, reduced immunogenicity) relative to the parent antibody and/or will have substantially retained certain biological properties of the parent antibody. An exemplary substitutional variant is an affinity matured antibody, which may be conveniently generated, *e.g*., using phage display-based affinity maturation techniques such as those described herein. Briefly, one or more HVR residues are mutated and the variant antibodies displayed on phage and screened for a particular biological activity (*e.g*. binding affinity).

Alterations (*e.g.,* substitutions) may be made in HVRs, *e.g.,* to improve antibody affinity. Such alterations may be made in HVR "hotspots," *i.e.,* residues encoded by codons that undergo mutation at high frequency during the somatic maturation process *(see, e.g.,* Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, *e.g.,* in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (*e.g*., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (*e.g*., 4-6 residues at a time) are randomized. HVR residues involved in antigen binding may be specifically identified, *e.g*., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (*e.g*., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (*e.g.,* charged residues such as Arg, Asp, His, Lys, and Glu) are identified and replaced by a neutral or negatively charged amino acid (*e.g*., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (*e.g*., for ADEPT) or a polypeptide which increases the serum half-life of the antibody.

### b) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. *See, e.g.,* Wright et al. TIBTECH 15:26-32 (1997). The oligosaccharide may include various carbohydrates, *e.g*., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, *i.e.,* between positions 294 and 300, due to minor sequence variations in antibodies. Such fucosylation variants may have improved ADCC function. *See, e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108, Presta, L; and WO 2004/056312 A1, Adams *et al.,* especially at Example 11), and knockout cell lines, such as alpha-1,6-fucosyltransferase gene, *FUT8,* knockout CHO cells *(see, e.g.,* Yamane-Ohnuki et al. Biotech. Bioeng. 87: 614 (2004); Kanda, Y. et al., Biotechnol. Bioeng., 94(4):680-688 (2006); and WO2003/085107). In some embodiments, antibodies may have a human IgG1, IgG2, IgG3, or IgG4 heavy chain constant region, for example, comprising a mutation at Asn297 to decrease fucosylation. In some embodiments, antibodies may have an Asn297Ala or Asn297Gly mutation.

Antibodies variants are further provided with bisected oligosaccharides, *e.g*., in which a biantennary oligosaccharide attached to the Fc region of the antibody is bisected by GlcNAc. Such antibody variants may have reduced fucosylation and/or improved ADCC function. Examples of such antibody variants are described, *e.g*., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, *e.g*., in WO 1997/30087 (Patel et al.); WO 1998/58964 (Raju, S.); and WO 1999/22764 (Raju, S.).

### c) Fc region variants

In certain embodiments, one or more amino acid modifications may be introduced into the Fc region of an antibody provided herein, thereby generating an Fc region variant. The Fc region variant may comprise a human Fc region sequence (*e.g*., a human IgG1, IgG2, IgG3 or IgG4 Fc region) comprising an amino acid modification (*e.g*., a substitution) at one or more amino acid positions.

In certain embodiments, the invention contemplates an antibody variant that possesses some but not all effector functions, which make it a desirable candidate for applications in which the half-life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (*see, e.g,.* Hellstrom, I. et al. Proc. Nat'l Acad. Sci. USA 83:7059-7063 (1986)) and Hellstrom, I et al., Proc. Nat'l A cad. Sci. USA 82:1499-1502 (1985); 5,821,337 *(see* Bruggemann, M. et al., J. Exp. Med. 166:1351-1361 (1987)). Alternatively, non-radioactive assays methods may be employed (*see,* for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. *See, e.g.,* C1q and C3c binding ELISA in WO 2006/029879 and WO 2005/100402. To assess complement activation, a CDC assay may be performed *(see,* for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half-life determinations can also be performed using methods known in the art *(see, e.g.,* Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581). In some embodiments, the antibody comprises an engineered alanine at amino acid position 265 according to EU numbering convention. In some embodiments, the antibody comprises an engineered alanine at amino acid position 297 according to EU numbering convention.

Certain antibody variants with improved or diminished binding to FcRs are described. (*See, e.g.,* U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, *e.g*., substitutions at positions 298, 333, and/or 334 of the Fc region (EU numbering of residues).

In some embodiments, alterations are made in the Fc region that result in altered (*i.e.,* either improved or diminished) C1q binding and/or Complement Dependent Cytotoxicity (CDC), *e.g.,* as described in US Patent No. 6,194,551, WO 99/51642, and Idusogie et al. J. Immunol. 164: 4178-4184 (2000).

Antibodies with increased half-lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, *e.g*., substitution of Fc region residue 434 (US Patent No. 7,371,826). *See* also Duncan & Winter, Nature 322:738-40 (1988); U.S. Patent No. 5,648,260; U.S. Patent No. 5,624,821; and WO 94/29351 concerning other examples of Fc region variants.

### d) Cysteine engineered antibody variants

In certain embodiments, it may be desirable to create cysteine engineered antibodies, *e.g*., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, *e.g*., in U.S. Patent No. 7,521,541.

### e) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### B. Recombinant Methods and Compositions

Antibodies may be produced using recombinant methods and compositions, *e.g*., as described in U.S. Patent No. 4,816,567. In one embodiment, isolated nucleic acid encoding an anti-RSPO antibody described herein is provided. Such nucleic acid may encode an amino acid sequence comprising the VL and/or an amino acid sequence comprising the VH of the antibody (*e.g*., the light and/or heavy chains of the antibody). In a further embodiment, one or more vectors (*e.g*., expression vectors) comprising such nucleic acid are provided. In a further embodiment, a host cell comprising such nucleic acid is provided. In one such embodiment, a host cell comprises (*e.g*., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (*e.g.,* Y0, NSO, Sp20 cell). In one embodiment, a method of making an anti-RSPO3 antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-RSPO3 antibody, nucleic acid encoding an antibody, *e.g*., as described above, is isolated and inserted into one or more vectors for further cloning and/or expression in a host cell. Such nucleic acid may be readily isolated and sequenced using conventional procedures (*e.g*., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, *see, e.g.,* U.S. Patent Nos. 5,648,237, 5,789,199, and 5,840,523. (*See* also Charlton, Methods in Molecular Biology, Vol. 248 (B.K.C. Lo, ed., Humana Press, Totowa, NJ, 2003), pp. 245-254, describing expression of antibody fragments in *E. coli.*) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. *See* Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See, e.g.,* US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, *e.g.,* in Graham et al., J. Gen Virol. 36:59 (1977)); baby hamster kidney cells (BHK); mouse sertoli cells (TM4 cells as described, *e.g.,* in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, *e.g.,* in Mather et al., Annals N.Y. Acad. Sci. 383:44-68 (1982); MRC 5 cells; and FS4 cells. Other useful mammalian host cell lines include Chinese hamster ovary (CHO) cells, including DHFR-CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, *see, e.g.,* Yazaki and Wu, Methods in Molecular Biology, Vol. 248 (B.K.C. Lo, ed., Humana Press, Totowa, NJ), pp. 255-268 (2003).

### C. Assays

Anti-RSPO3 antibodies provided herein may be identified, screened for, or characterized for their physical/chemical properties and/or biological activities by various assays known in the art.

### 1. Binding assays and other assays

In one aspect, an antibody of the invention is tested for its antigen binding activity, *e.g.,* by known methods such as ELISA, Western blot, etc.

Methods of determining binding affinity are known in the art. In some embodiments, the binding affinity may be determined according to a BIAcore® assay as described herein in Example 1. Specifically, in some embodiments, Kd may be measured using surface plasmon resonance assays using a BIACORE®-3000 (BIAcore, Inc., Piscataway, NJ).

Methods of determining the ability of an anti-RSPO3 antibody to disrupt and/or inhibit the binding of RSPO3 to a ligand such as an LGR (*e.g.,* LGR4, 5, and/or 6), syndecan (*e.g.,* SDC4), and/or an E3 ubiquitinase (*e.g.,* ZNRF3 and/or RNF43) are known in the art. *See e.g.,* WO2011/076932, WO2013012747, Lau et al. Nature 476:293-297 (2011), Hao et al. Nature 485:195-200 (2012), which are hereby incorporated by reference in their entirety. In some embodiments, the ability of an anti-RSPO3 antibody to significantly disrupt the binding of RSPO3 to an LGR, syndecan and/or E3 ubiquitinase may be determined by flow cytometry, BIAcore assay, and/or ELISA (e.g., Competitive Binding ELISA). In some embodiments, the ability of an anti-RSPO3 antibody to disrupt and/or inhibit the binding of RSPO3 to an LGR (*e.g.,* LGR4, 5, and/or 6), syndecan (SDC4), and/or an E3 ubiquitinase (*e.g*., ZNRF3 and/or RNF43) may be determined according to a competition assay such as Competitive Binding ELISA.

In another aspect, competition assays may be used to identify an antibody that competes with an antibody comprising the heavy and light chain variable regions of 4A6, 11C10, or 15F3 for binding to RSPO3.

Methods of determining antibody competition are known in the art. In an exemplary competition assay, immobilized RSPO3 may be incubated in a solution comprising a first labeled antibody that binds to RSPO3 (*e.g.,* an antibody comprising the heavy and light chain variable regions of 4A6, 11C10, or 15F3) and a second unlabeled antibody that is being tested for its ability to compete with the first antibody for binding to RSPO3. The second antibody may be present in a hybridoma supernatant. As a control, immobilized RSPO3 is incubated in a solution comprising the first labeled antibody but not the second unlabeled antibody. After incubation under conditions permissive for binding of the first antibody to RSPO3, excess unbound antibody is removed, and the amount of label associated with immobilized RSPO3 is measured. If the amount of label associated with immobilized RSPO3 is substantially reduced in the test sample relative to the control sample, then that indicates that the second antibody is competing with the first antibody for binding to RSPO3. *See* Harlow and Lane (1988) Antibodies: A Laboratory Manual ch.14 (Cold Spring Harbor Laboratory, Cold Spring Harbor, NY).

In certain embodiments, an antibody binds to the same epitope *(e.g.,* a linear or a conformational epitope) as one or more of 4A6, 11C10, and 15F3. Detailed exemplary methods for mapping an epitope to which an antibody binds are provided in Morris (1996) "Epitope Mapping Protocols," in Methods in Molecular Biology vol. 66 (Humana Press, Totowa, NJ). In some embodiments, the epitope is determined by peptide competition. In some embodiments, the epitope is determined by mass spectrometry. In some embodiments, the epitope is determined by crystallography. An exemplary method of crystallography is described in Example 1.

### 2. Activity assays

In one aspect, assays are provided for identifying anti-RSPO3 antibodies thereof having biological activity. Biological activity may include, *e.g*., inhibit wnt signaling, inhibit angiogenesis, inhibit cell proliferation, inhibit cancer stem cell proliferation, and/or deplete cancer stem cells. Antibodies having such biological activity in vivo and/or in vitro are also provided.

Methods of determining ability of an anti-RSPO3 antibody to disrupt wnt/beta-catenin signaling are known in the art. *See e.g.,* WO2005/040418 and WO2013/012747, which is hereby incorporated by reference in its entirety. In some embodiments, the ability of an anti-RSPO3 antibody to significantly disrupt wnt/beta-catenin signaling may be determined using a reporter gene assay. In some embodiments, for example, a reporter construct comprising a reporter gene (such as, for example, a luciferase gene) under the control of a wnt/beta-catenin responsive promoter (such as, for example, a promoter comprising multimerized TCF/LEF DNA-binding sites) may be transfected into cells. The cells are then contacted with a Wnt ligand, such as Wnt3a, and RSPO3, in the presence and absence of an RSPO3 antibody, and luciferase expression is measured.

Methods of determining ability of an anti-RSPO3 antibody inhibiting angiogenesis and/or vasculogenesis are known in the art. *See e.g.,* WO2008/046649, which is hereby incorporated by reference in its entirety. Examples of assays include the in vivo Matrigel® plug and corneal neovascularization assays, the in vivo/in vitro chick chorioallantoic membrane (CAM) assay, the in vitro cellular (proliferation, migration, tube formation) and organotypic (aortic ring) assays, the chick aortic arch assays, and the Matrigel® sponge assays.

Methods of determining the ability of an anti-RSPO3 antibody to induce stem cell differentiation and/or cancer stem cell depletion are known in the art. *See e.g.,* WO2013/036867, which is hereby incorporated by reference in its entirety. In some embodiments, stem cell differentiation may be assayed by determining ability to differentiation of crypt base columnar cells (CBCs), which are fast-cycling stem cells in the small intestine, into, for example, enterocytes, goblet cells, and/or enteroendocrine cells, in the presence and absence of an anti-RSPO3 antibody.

In certain embodiments, an antibody of the invention is tested for such biological activity and/or binding interactions by the assays described herein and in WO2005/040418, WO2008/046649, WO2011/076932, WO2013/012747, WO2013/054307, Lau et al. Nature 476:293-297 (2011), Hao et al. Nature 485:195-200 (2012), which are hereby incorporated by reference in their entirety.

In some embodiment, the epitope is determined by crystallography. In some embodiments, the epitope as determined by crystallography is determined using amino acids M33-E210 of RSPO3. In some embodiments, the epitope as determined by crystallography is performed by using a Labcyte, Inc. Echo® liquid handler to set several sparse matrix crystal screens using 100 nL sitting drops. Screens were stored at 18°C. In some embodiments, crystals may be obtained in a drop containing 100 mM MIB pH 9 and 25% PEG 1500 as the mother liquor. In some embodiments, crystals may be obtained in a drop containing 200 mM Sodium formate and 20% (w/v) PEG 3,350 as the mother liquor. In some embodiments, the crystal may be harvested and soaked in cryoprotectant solution for 10 seconds and flash-frozen in liquid nitrogen. In some embodiments, the cryoprotectant solution may be made by mixing 1 µL 70% glycerol with 1.8µL reservoir solution. In some embodiments, the crystals may be grown in PEG-based conditions, for example, about 20-25% PEG 3,350. In some embodiments, the crystals may be grown in about 20% PEG 6,000, about 20-25% PEG 4,000, and about 25% PEG 1,500. In some embodiments, the pH may range from about 3.5 - 9, for example, between about 7 and about 8. In some embodiments, the salt concentration is about 200 mM

### D. Immunoconjugates

The invention also provides immunoconjugates comprising an anti-RSPO3 antibody herein conjugated to one or more cytotoxic agents, such as chemotherapeutic agents or drugs, growth inhibitory agents, toxins (*e.g*., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (*see* U.S. Patent Nos. 5,208,020, 5,416,064 and European Patent EP 0 425 235 B1); an auristatin such as monomethylauristatin drug moieties DE and DF (MMAE and MMAF) (*see* U.S. Patent Nos. 5,635,483 and 5,780,588, and 7,498,298); a dolastatin; a calicheamicin or derivative thereof (*see* U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin *(see* Kratz et al., Current Med. Chem. 13:477-523 (2006); Jeffrey et al., Bioorganic &Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it may comprise a radioactive atom for scintigraphic studies, for example Tc99m or 1123, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCI), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. *See* WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) maybe used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### E. Methods and Compositions for Diagnostics and Detection

In certain embodiments, any of the anti-RSPO3 antibodies provided herein is useful for detecting the presence of RSPO3 in a sample. The term "detecting" as used herein encompasses quantitative or qualitative detection. In certain embodiments, a sample comprises a cell or tissue, such as gastrointestinal, stomach, esophageal, colon, rectal, and/or colorectal tissue. In some embodiments, a sample comprises a cell or tissue, such as adrenal, bladder, brain, breast, cervix, colon, head and neck, kidney, leukemia, liver, lung, lymphoid, ovarian, pancreas, prostate, rectum, skin, stomach, thyroid, and/or uterus tissue. In some embodiments, a sample comprises a cell or tissue, such as lung, ovarian, breast, liver, or multiple myeloma tissue.

In one embodiment, an anti-RSPO3 antibody for use in a method of diagnosis or detection is provided. In a further aspect, a method of detecting the presence of RSPO3 in a sample is provided. In certain embodiments, the method comprises contacting the sample with an anti-RSPO3 antibody as described herein under conditions permissive for binding of the anti-RSPO3 antibody to RSPO and detecting whether a complex is formed between the anti-RSPO3 antibody and RSPO3. Such method may be an *in vitro* or *in vivo* method. In one embodiment, an anti-RSPO3 antibody is used to select subjects eligible for therapy with an anti-RSPO3 antibody, e.g. where RSPO3 is a biomarker for selection of patients. In some embodiments, the individual and/or cancer has increased expression of one or more stem cell biomarkers. In some embodiments, the stem cell biomarker comprises Myc, Axin2, LGR5, TERT, BIRC5, and/or Ascl2. In some embodiments, the individual and/or cancer has decreased expression of one or more biomarker of differentiation. In some embodiments, the biomarker of differentiation comprises CEACAM7, SLC26A3, CA1, SYT15, CA4, TFF1, and/or KRT20.

For example, provided herein are methods of treating cancer in an individual, wherein the cancer comprises one or more biomarkers, comprising administering to the individual an effective amount of an anti-RSPO3 antibody. Also provided herein are methods of treating cancer in an individual comprising administering to the individual an effective amount of an anti-RSPO3 antibody, wherein treatment is based upon the individual having cancer comprising one or more biomarkers.

Translocation are exceptionally powerful cancer mutations, as they often have multiple effects on a target gene: in a single 'mutation' they can dramatically change expression, remove regulatory domains, force oligomerization, change the subcellular location of a protein or join it to novel binding domains. This is reflected clinically in the fact that some neoplasms are classified or managed according to the presence of a particular fusion gene. In some embodiments of any of the methods, the one or more biomarkers comprise a translocation (e.g., intrachromosomal translocation, interchromosomal translocation, rearrangement and/or fusion) of one or more genes listed in Table 2.

In some embodiments of any of the methods, the translocation is a PVT1. In some embodiments, the PVT1 translocation comprises *PVT1* and *MYC.* In some embodiments, an RSPO2 translocation comprises *PVT1* and *IncDNA.* In some embodiments of any of the methods, the translocation is an R-spondin translocation. In some embodiments, the R-spondin translocation is a RSPO1 translocation. In some embodiments, the R-spondin translocation is a RSPO2. In some embodiments, the RSPO2 translocation comprises *EMC2* and *RSPO2.* In some embodiments, the RSPO2 translocation comprises *EIF3E* and *RSPO2.* In some embodiments, the RSPO2 translocation comprises *EIF3E* exon 1 and *RSPO2* exon 2. In some embodiments, the RSPO2 translocation comprises *EIF3E* exon 1 and *RSPO2* exon 3. In some embodiments, the RSPO2 translocation comprises SEQ ID NO:103. In some embodiments, the RSPO2 translocation is detectable by primers which include SEQ ID NO:111, 141, and/or 142. In some embodiments, the RSPO2 translocation is driven by the *EIF3E* promoter. In some embodiments, the RSPO2 translocation is driven by the *RSPO2* promoter.

In some embodiments, the R-spondin translocation is a RSPO3 translocation. In some embodiments, the RSPO3 translocation comprises *PTPRK* and *RSPO3.* In some embodiments, the RSPO3 translocation comprises *PTPRK* exon 1 (PTPRK(e1)) and *RSPO3* exon 2 (RSPO3(e2)). In some embodiments, the RSPO3 translocation comprises *PTPRK* exon 7 (PTPRK(e7)) *andRSPO3* exon 2 (RPSO3(e2)). In some embodiments, the RSPO3 translocation comprises SEQ ID NO:105 and/or SEQ ID NO: 107. In some embodiments, the RSPO3 translocation is detectable by primers which include SEQ ID NO: 112, 113, 143, and/or 144. In some embodiments, the RSPO3 translocation is driven by the PTPRK promoter. In some embodiments, the RSPO3 translocation is driven by the *RSPO3* promoter. In some embodiments, the RSPO3 translocation) comprises the PTPRK secretion signal sequence (and/or does not comprise the RSPO3 secretion signal sequence).

**Table 2 - Gene Fusions**

| **5'GeneName** | **3'GeneName** | **Type** | **Genomic position** | **5' PCR primer** | **3' PCR primer** | **bp** |
|---|---|---|---|---|---|---|
| PVT1 | ENST000005 02082 | Intrachrom. | 8:128806980-8:128433074 | CTTGCGGAAAGGATGTTGG (SEQ ID NO:109) | TGGTGATCCAGAGAAGAAGC (SEQ ID NO:139) | 150 |
| EMC2 | RSPO2 | | 8:109455927-8:109095035 | CACCCCGCTGCCTCTAGGTTCTGGGAAGATG GCGAAGGTCTCAGAGCTTTACGATGTCACTT GGGAAG (SEQ ID NO:110) | GTTCGTGGCGGAGAGATGCTGATCGCGCTGAACTGACCG GTGCGGCCCGGGGGTGAGTGGCGAGTCTCCC (SEQ ID NO:140) | |
| EIF3E(e1) | RSPPO2(e2) | Deletion | 8: 109260842-8:109095035 | ACTACTCGCATCGCGCACT (SEQ ID NO:111) | GGGAGGACTCAGAGGGAGAC (SEQ ID NO:141) | 155 |
| EIF3E(e1) | RSPO2(e2) | Deletion | 8: 109260842-8:109095035 | ACTACTCGCATCGCGCACT (SEQ ID NO:111) | GGGAGGACTCAGAGGGAGAC (SEQ ID NO:141) | 155 |
| EIF3E(e1) | RSPO2(e3) | Deletion | 8: 109260842-8:109001472 | ACTACTCGCATCGCGCACT (SEQ ID NO:111) | TGCAGGCACTCTCCATACTG (SEQ ID NO:142) | 205 |
| EIF3E(e1) | RSPO2(e3) | Deletion | 8: 109260842-8:109001472 | ACTACTCGCATCGCGCACT (SEQ ID NO:111) | TGCAGGCACTCTCCATACTG (SEQ ID NO:142) | 205 |
| PTPRK(e1) | RSPO3(e2) | Inversion | 6:128841404-6:127469793 | AAACTCGGCATGGATACGAC (SEQ ID NO:112) | GCTTCATGCCAATTCTTTCC (SEQ ID NO:143) | 226 |
| PTPRK(e1) | RSPO3(e2) | Inversion | 6:128841404-6:127469793 | AAACTCGGCATGGATACGAC (SEQ ID NO:112) | GCTTCATGCCAATTCTTTCC (SEQ ID NO:143) | 226 |
| PTPRK(e1) | RSPO3(e2) | Inversion | 6:128841404-6:127469793 | AAACTCGGCATGGATACGAC (SEQ ID NO:112) | GCTTCATGCCAATTCTTTCC (SEQ ID NO:143) | 226 |
| PTPRK(e1) | RSPO3(e2) | Inversion | 6:128841404-6:127469793 | AAACTCGGCATGGATACGAC (SEQ ID NO:112) | GCTTCATGCCAATTCTTTCC (SEQ ID NO:143) | 226 |
| PTPRK(e7) | RSPO3(e2) | Inversion | 6: 128505577-6:127469793 | TGCAGTCAATGCTCCAACTT (SEQ ID NO:113) | GCCAATTCTTTCCAGAGCAA (SEQ ID NO:144) | 250 |
| ETV6 | NTRK3 | Interchrom | 12:12022903-15:88483984 | AAGCCCATCAACCTCTCTCA (SEQ ID NO:114) | GGGCTGAGGTTGTAGCACTC (SEQ ID NO:145) | 206 |
| ANXA2 | RORA | intrachrom. | 15:60674541-15:60824050 | CTCTACACCCCCAAGTGCAT (SEQ ID NO:115) | TGACACCATAATGGATTCCTG (SEQ ID NO:146) | 164 |
| TUBGCP3 | PDS5B | Inversion | 13:113200013-13:33327470 | AACAGGAGACCCGTACATGC (SEQ ID NO:116) | AAAGGGCACAGATTGCCATA (SEQ ID NO:147) | 221 |
| ARHGEF18 | NCRNA00157 | Interchrom | 19:7460133-21:19212970 | CCAGCTGCTAGCTACTGTGGA (SEQ ID NO:117) | ACTAGGTGGTCCAGGGTGTG (SEQ ID NO:148) | 186 |
| NT5C2 | ASAH2 | Deletion | 10:104899163-10:51978390 | TGAACCGAAGTTTAGCAATGG (SEQ ID NO:118) | TGCTCAAGCAGGTAAGATGC (SEQ ID NO:149) | 156 |
| NRBP2 | VPS28 | intrachrom. | 8:144919211-8:145649651 | TGATGAACTTTGCAGCCACT (SEQ ID NO:119) | ATGGTCTCCATCAGCTCTCG (SEQ ID NO:150) | 208 |
| CDC42SE2 | KIAA0146 | Interchrom | 5:130651837-8:48612965 | AGGGCCAGATTTGAGTGTGT (SEQ ID NO:120) | AAACTGAAAATCCCCGCTGT (SEQ ID NO:151) | 188 |
| MED13L | LAG3 | Inversion | 12:116675273-12:6886957 | GTGTATGGCGTCGTGATGTC (SEQ ID NO:121) | GCTCCAGTCACCAAAAGGAG (SEQ ID NO:152) | 205 |
| PEX5 | LOC389634 | Inversion | 12:7362838-12:8509737 | CATGTCGGAGAACATCTGGA (SEQ ID NO:122) | TGTGGAGTCTCTTGCGTGTC (SEQ ID NO:153) | 230 |
| PLCE1 | CYP2C19 | Deletion | 10:95792009-10:96602594 | CCTTACTGCCTTGTGGGAGA (SEQ ID NO:123) | TGGGGATGAGGTCGATGTAT (SEQ ID NO:154) | 224 |
| TPM3 | NTRK1 | Inversion | 1:154142876-1:156844363 | CAGAGACCCGTGCTGAGTTT (SEQ ID NO:124) | CCAAAAGGTGTTTCGTCCTT (SEQ ID NO:155) | 124 |
| PAN3 | RFC3 | Deletion | 13:28752072-13:34395269 | GACTTTGGTGCCCTCAACAT (SEQ ID NO:125) | CAATTTTTCCACTCCAACACC (SEQ ID NO:156) | 150 |
| CWC27 | RNF180 | intrachrom. | 5:64181373-5:63665442 | AACGGGAACTCTTAGCAGCA (SEQ ID NO:126) | CATGTCAAACCACCATCCAC (SEQ ID NO:157) | 182 |
| CAPN1 | SPDYC | intrachrom. | 11:64956217-11:64939414 | GAGACTTCATGCGGGAGTTC (SEQ ID NO:127) | ATCTGGAAGCAGGGGTCTTT (SEQ ID NO:158) | 199 |
| COG8 | TERF2 | intrachrom. | 16:69373079-16:69391464 | TGGCCTTCGCTAACTACAAGA (SEQ ID NO:128) | TCCCCATATTTCTGCACTCC (SEQ ID NO:159) | 233 |
| TADA2A | MEF2B | Interchrom | 17:35767040-19:19293492 | GCTCTTTGGCGCGGATTA (SEQ ID NO:129) | GGAGCTACCTGTGGCCCT (SEQ ID NO:160) | 152 |
| STRBP | DENND1A | intrachrom. | 9: 125935956-9:126220176 | GTTGCAAAAGGCTTGCTGAT (SEQ ID NO:130) | ACGAAGGCTTCCTCACAGAA (SEQ ID NO:161) | 155 |
| CXorf56 | UBE2A | Inversion | X:118694231-X:118717090 | TGATTGATGCTGCCAAACAT (SEQ ID NO:131) | CACGCTTTTCATATTCCCGT (SEQ ID NO:162) | 161 |
| MED13L | CD4 | Inversion | 12:116675273-12:6923308 | GTGTATGGCGTCGTGATGTC (SEQ ID NO:121) | TCCCAAAGGCTTCTTCTTGA (SEQ ID NO:163) | 151 |
| PRR12 | PRRG2 | intrachrom. | 19:50097872-19:50093157 | ATGAACCTTATCTCGGCCCT (SEQ ID NO:132) | GTCGTGTACCCCAGAGGCT (SEQ ID NO:164) | 227 |
| ATP9A | ARFGEF2 | Inversion | 20:50307278-20:47601266 | ATGTGTACGCAGAAGAGCCA (SEQ ID NO:133) | GTGCAGGAATTGGGCTATGT (SEQ ID NO:165) | 150 |
| ANKRD17 | HS3ST1 | Deletion | 4:73956384-4:11401737 | GGAAAATCCTCATATTTGCCA (SEQ ID NO:134) | AGCAGGGAAGCCTCCTAGTC (SEQ ID NO:166) | 158 |
| RBM47 | ATP8A1 | intrachrom. | 4:40517884-4:42629126 | AGACCCAGGAGGAGTGAGGT (SEQ ID NO:135) | GGTCAGCCAGTGAGGTCTTC (SEQ ID NO:167) | 151 |
| FRS2 | RAP1B | intrachrom. | 12:69924740-12:69042479 | AGATGCCCAGATGCAAAAGT (SEQ ID NO:136) | CAAAGCAGACTTTCCAACGC (SEQ ID NO:168) | 161 |
| CHEK2 | PARVB | Inversion | 22:29137757-22:44553862 | GGCTGAGGGTGGAGTTTGTA (SEQ ID NO:137) | CTTCTGATCGAAGCTTTCCG (SEQ ID NO:169) | 191 |
| SFI1 | TPST2 | Inversion | 22:31904362-22:26940641 | CCCCAGTTAGAAGGGGAAGA (SEQ ID NO:138) | CACTCTCATCTCTGGGCTCC (SEQ ID NO:170) | 190 |

In some embodiments, the R-spondin translocation is a RSPO4 translocation. In some embodiments, the R-spondin translocation results in elevated expression levels of R-spondin (*e.g.*, compared to a reference without the R-spondin translocation). In some embodiments, the R-spondin translocation results in elevated activity and/or activation of R-spondin (*e.g*., compared to a reference without the R-spondin translocation). In some embodiments, the presence of one or more biomarkers comprises an R-spondin translocation), such as a translocation in Table 2, and *KRAS* and/or *BRAF.* In some embodiments, the presence of one or more biomarkers is presence of an R-spondin translocation (*e.g.,* rearrangement and/or fusion), such as a translocation in Table 2, and a variation (*e.g.,* polymorphism or mutation) *KRAS* and/or *BRAF.* In some embodiments, the individual and/or cancer comprises a variation (polymorphism or mutation) in *KRAS* and/or *BRAF.* In some embodiments, the presence of one or more biomarkers is presence of an R-spondin translocation, such as a translocation in Table 2, and the absence of one or more biomarkers is absence of a variation (*e.g*., polymorphism or mutation) *CTNNB1* and/or *APC.*

In some embodiments of any of the translocation (*e.g*., intrachromosomal translocation, interchromosomal translocation, rearrangement and/or fusion), the translocation (*e.g.*, intrachromosomal translocation, interchromosomal translocation, rearrangement and/or fusion) is a somatic translocation (*e.g*., intrachromosomal translocation, interchromosomal translocation, rearrangement and/or fusion). In some embodiments, the translocation is an intrachromosomal translocation. In some embodiments, the translocation is an interchromosomal. In some embodiments, the translocation is an inversion. In some embodiments, the translocation is a deletion. In some embodiments, the translocation is a functional translocation fusion polynucleotide (*e.g*., functional R-spondin-translocation fusion polynucleotide) and/or functional translocation fusion polypeptide (*e.g*., functional R-spondin-translocation fusion polypeptide). In some embodiments, the functional translocation fusion polypeptide (*e.g*., functional R-spondin-translocation fusion polypeptide) activates a pathway known to be modulated by one of the tranlocated genes (*e.g*., wnt signaling pathway). In some embodiments, the pathway is canonical wnt signaling pathway. In some embodiments, the pathway is noncanonical wnt signaling pathway. In some embodiments, the Methods of determining pathway activation are known in the art and include luciferase reporter assays as described herein. In some embodiments, the method is one or more methods described in Seshagiri et al., Nature 488:660-664 (2012) and/or WO 2013/120056, which are incorporated by reference in their entirety.

Exemplary disorders that may be diagnosed using an antibody of the invention include tumors, cell proliferative disorders, cancer, gastrointestinal cancer, stomach cancer, colorectal cancer, colon cancer, and/or rectal cancer. Exemplary disorders that may be diagnosed using an antibody of the invention further include adrenal cancer, bladder cancer, brain cancer, breast cancer, cervix cancer, colon cancer, head and neck cancer, kidney cancer, leukemia, liver cancer, lung cancer (*e.g*., NSCLC), lymphoid cancer, ovarian cancer, pancreatic cancer, prostate cancer, rectum cancer, skin cancer (*e.g*., melanoma), stomach cancer, thyroid cancer, and/or uterine cancer. Exemplary disorders that may be diagnosed using an antibody of the invention also include lung cancer (*e.g*., NSCLC), ovarian cancer, breast cancer, liver cancer, or multiple myeloma.

Samples include, but are not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, and combinations thereof. In some embodiments, the sample is a sample from gastrointestinal, stomach, esophageal, colon, rectal, and/or colorectal tissue. In some embodiments, the sample is a sample from adrenal, bladder, brain, breast, cervix, colon, head and neck, kidney, leukemia, liver, lung, lymphoid, ovarian, pancreas, prostate, rectum, skin, stomach, thyroid, and/or uterus tissue. In some embodiments, the sample is a sample from lung, ovarian, breast, liver, or multiple myeloma tissue.

In certain embodiments, labeled anti-RSPO3 antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, *e.g*., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, *e.g*., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, *e.g*., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

In some embodiments of any of the methods, elevated expression refers to an overall increase of about any of 10%, 20%, 30%, 40%, 50%, 60%, 70%, 80%, 90%, 95%, 96%, 97%, 98%, 99% or greater, in the level of biomarker (*e.g.,* protein or nucleic acid (*e.g.,* gene or mRNA)), detected by standard art known methods such as those described herein, as compared to a reference sample, reference cell, reference tissue, control sample, control cell, or control tissue. In certain embodiments, the elevated expression refers to the increase in expression level/amount of a biomarker in the sample wherein the increase is at least about any of 1.5X, 1.75X, 2X, 3X, 4X, 5X, 6X, 7X, 8X, 9X, 10X, 25X, 50X, 75X, or 100X the expression level/amount of the respective biomarker in a reference sample, reference cell, reference tissue, control sample, control cell, or control tissue. In some embodiments, elevated expression refers to an overall increase of greater than about 1.5 fold, about 1.75 fold, about 2 fold, about 2.25 fold, about 2.5 fold, about 2.75 fold, about 3.0 fold, or about 3.25 fold as compared to a reference sample, reference cell, reference tissue, control sample, control cell, control tissue, or internal control (*e.g*., housekeeping gene).

In some embodiments of any of the methods, reduced expression refers to an overall reduction of about any of 10%, 20%, 30%, 40%, 50%, 60%, 70%, 80%, 90%, 95%, 96%, 97%, 98%, 99% or greater, in the level of biomarker (*e.g.,* protein or nucleic acid (*e.g.,* gene or mRNA)), detected by standard art known methods such as those described herein, as compared to a reference sample, reference cell, reference tissue, control sample, control cell, or control tissue. In certain embodiments, reduced expression refers to the decrease in expression level/amount of a biomarker in the sample wherein the decrease is at least about any of 0.9X, 0.8X, 0.7X, 0.6X, 0.5X, 0.4X, 0.3X, 0.2X, 0.1X, 0.05X, or 0.01X the expression level/amount of the respective biomarker in a reference sample, reference cell, reference tissue, control sample, control cell, or control tissue.

Presence and/or expression level/amount of various biomarkers in a sample can be analyzed by a number of methodologies, many of which are known in the art and understood by the skilled artisan, including, but not limited to, immunohistochemical ("IHC"), Western blot analysis, immunoprecipitation, molecular binding assays, ELISA, ELIFA, fluorescence activated cell sorting ("FACS"), MassARRAY, proteomics, quantitative blood based assays (as for example Serum ELISA), biochemical enzymatic activity assays, in situ hybridization, Southern analysis, Northern analysis, whole genome sequencing, polymerase chain reaction ("PCR") including quantitative real time PCR ("qRT-PCR") and other amplification type detection methods, such as, for example, branched DNA, SISBA, TMA and the like), RNA-Seq, FISH, microarray analysis, gene expression profiling, and/or serial analysis of gene expression ("SAGE"), as well as any one of the wide variety of assays that can be performed by protein, gene, and/or tissue array analysis. Typical protocols for evaluating the status of genes and gene products are found, for example in Ausubel et al., eds., 1995, Current Protocols In Molecular Biology, Units 2 (Northern Blotting), 4 (Southern Blotting), 15 (Immunoblotting) and 18 (PCR Analysis). Multiplexed immunoassays such as those available from Rules Based Medicine or Meso Scale Discovery ("MSD") may also be used.

In some embodiments, presence and/or expression level/amount of a biomarker is determined using a method comprising: (a) performing gene expression profiling, PCR (such as rtPCR), RNA-seq, microarray analysis, SAGE, MassARRAY technique, or FISH on a sample (such as a subject cancer sample); and b) determining presence and/or expression level/amount of a biomarker in the sample. In some embodiments, the microarray method comprises the use of a microarray chip having one or more nucleic acid molecules that can hybridize under stringent conditions to a nucleic acid molecule encoding a gene mentioned above or having one or more polypeptides (such as peptides or antibodies) that can bind to one or more of the proteins encoded by the genes mentioned above. In one embodiment, the PCR method is qRT-PCR. In one embodiment, the PCR method is multiplex-PCR. In some embodiments, gene expression is measured by microarray. In some embodiments, gene expression is measured by qRT-PCR. In some embodiments, expression is measured by multiplex-PCR.

### F. Pharmaceutical Formulations

Pharmaceutical formulations of an anti-RSPO3 antibody as described herein may be prepared by mixing such antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, *e.g*. films, or microcapsules.

The formulations to be used for *in vivo* administration are generally sterile. Sterility may be readily accomplished, *e.g*., by filtration through sterile filtration membranes.

### G. Therapeutic Methods and Compositions

In some embodiments, the anti-RSPO3 antibodies provided herein may be used in therapeutic methods.

In one aspect, an anti-RSPO3 antibody for use as a medicament is provided. In further aspects, an anti-RSPO3 antibody for use in treating tumors, cell proliferative disorders, and/or cancer is provided. In some embodiments, an anti-RSPO3 antibody is provided for use in promoting differentiation of cells including terminal differentiation of cancer cells. In certain embodiments, an anti-RSPO3 antibody for use in a method of treatment is provided. In certain embodiments, the invention provides an anti-RSPO3 antibody for use in a method of treating an individual having tumor, cell proliferative disorder, and/or cancer comprising administering to the individual an effective amount of the anti-RSPO3 antibody. In some embodiments, the cancer is adrenal cancer, bladder cancer, brain cancer, breast cancer, cervix cancer, colon cancer, head and neck cancer, kidney cancer, leukemia, liver cancer, lung cancer (*e.g*., NSCLC), lymphoid cancer, ovarian cancer, pancreatic cancer, prostate cancer, rectum cancer, skin cancer (*e.g*., melanoma), stomach cancer, thyroid cancer, and/or uterine cancer. In some embodiments, the cancer is lung cancer (*e.g*., NSCLC), ovarian cancer, breast cancer, liver cancer, or multiple myeloma. In some embodiments, the cancer is colorectal cancer. In some embodiments, the cancer is gastrointestinal cancer, stomach cancer, colon cancer, colorectal cancer, lung cancer, or rectal cancer. In some embodiments, the method further comprises administering to the individual an effective amount of at least one additional therapeutic agent, *e.g.,* as described below.

In some embodiments, the cancer is characterized by an RSPO3 fusion, such as a PTPRK-RSPO3 fusion, such as a PTPRK(e1)-RSPO3(e2) fusion or a PTPRK(e7)-RSPO3(e2) fusion, such as SEQ ID NO: 105 or 107. In some embodiments, the cancer overexpresses RSPO3 (including, for example, an RSPO3 fusion polypeptide). In further embodiments, the invention provides an anti-RSPO3 antibody for use in inhibiting wnt signaling, inhibiting angiogenesis, inhibiting cell proliferation, inhibiting cancer stem cell proliferation, and/or depleting cancer stem cells. In certain embodiments, the invention provides an anti-RSPO3 antibody for use in a method of inhibiting wnt signaling, inhibiting angiogenesis, inhibiting cell proliferation, inhibiting cancer stem cell proliferation, and/or depleting cancer stem cells in an individual comprising administering to the individual an effective of the anti-RSPPO3 antibody to inhibit wnt signaling, inhibit angiogenesis, inhibit cell proliferation, inhibit cancer stem cell proliferation, and/or deplete cancer stem cells.

An "individual" according to any of the above embodiments is preferably a human. In some embodiments, the individual and/or cancer has one or more biomarkers. In some embodiments, the one or more biomarkers comprise an RSPO translocation. In some embodiments, the RSPO translocation comprises and RSPO2 and/or RSPO3 translocation. In some embodiments, the individual and/or cancer has increased expression of one or more biomarker. In some embodiments, the one or more biomarker comprises RSPO, *e.g*., RSPO2 and/or RSPO3. In some embodiments, the one or more biomarkers comprise an RSPO3 translocation. In some embodiments, the one or more biomarker comprises a stem cell biomarker. In some embodiments, the stem cell biomarker comprises Myc, Axin2, LGR5, TERT, BIRC5, and/or Ascl2. In some embodiments, the individual and/or cancer has decreased expression of one or more biomarkers of differentiation. In some embodiments, the one or more biomarkers of differentiation comprise CEACAM7, SLC26A3, CA1, SYT15, CA4, TFF1, and/or KRT20. In some embodiments, treatment with the anti-RSPO3 antibody reduces expression of one or more stem cell biomarkers, *e.g*., Myc, Axin2, LGR5, TERT, BIRC5, and/or Ascl2. In some embodiments, treatment with the anti-RSPO3 antibody increases expression of one or more biomarkers of differentiation, *e.g*., CEACAM7, SLC26A3, CA1, SYT15, CA4, TFF1, and/or KRT20.

In a further aspect, the invention provides for the use of an anti-RSPO3 antibody in the manufacture or preparation of a medicament. In one embodiment, the medicament is for treatment of tumor, cell proliferative disorder, and/or cancer. In a further embodiment, the medicament is for use in a method of treating tumor, cell proliferative disorder, and/or cancer comprising administering to an individual having tumor, cell proliferative disorder, and/or cancer an effective amount of the medicament. In some embodiments, the cancer is adrenal cancer, bladder cancer, brain cancer, breast cancer, cervix cancer, colon cancer, head and neck cancer, kidney cancer, leukemia, liver cancer, lung cancer (*e.g*., NSCLC), lymphoid cancer, ovarian cancer, pancreatic cancer, prostate cancer, rectum cancer, skin cancer (*e.g*., melanoma), stomach cancer, thyroid cancer, and/or uterine cancer. In some embodiments, the cancer is lung cancer (*e.g*., NSCLC), ovarian cancer, breast cancer, liver cancer, or multiple myeloma. In some embodiments, the cancer is colorectal cancer. In some embodiments, the cancer is gastrointestinal cancer, stomach cancer, colon cancer, colorectal cancer, lung cancer, or rectal cancer. In some embodiments, the method in which the medicament is used further comprises administering to the individual an effective amount of at least one additional therapeutic agent, *e.g.,* as described below.

In a further embodiment, the medicament is for inhibiting wnt signaling, inhibiting angiogenesis, inhibiting cell proliferation, inhibiting cancer stem cell proliferation, and/or depleting cancer stem cells. In a further embodiment, the medicament is for use in a method of inhibiting wnt signaling, inhibiting angiogenesis, inhibiting cell proliferation, inhibiting cancer stem cell proliferation, and/or depleting cancer stem cells in an individual comprising administering to the individual an amount effective of the medicament to inhibit wnt signaling, inhibit angiogenesis, inhibit cell proliferation, inhibit cancer stem cell proliferation, and/or deplete cancer stem cells.

An "individual" according to any of the above embodiments may be a human. In some embodiments, the individual and/or cancer has one or more biomarkers. In some embodiments, the one or more biomarkers comprise an RSPO translocation. In some embodiments, the RSPO translocation comprises and RSPO2 and/or RSPO3 translocation. In some embodiments, the individual and/or cancer has increased expression of one or more biomarker. In some embodiments, the one or more biomarkers comprise RSPO, *e.g*., RSPO2 and/or RSPO3. In some embodiments, the one or more biomarkers comprise an RSPO3 translocation. In some embodiments, the one or more biomarkers comprise a stem cell biomarker. In some embodiments, the stem cell biomarker comprises Myc, Axin2, LGR5, TERT, BIRC5, and/or Ascl2. In some embodiments, the individual and/or cancer has decreased expression of one or more biomarkers of differentiation. In some embodiments, the one or more biomarkers of differentiation comprise CEACAM7, SLC26A3, CA1, SYT15, CA4, TFF1, and/or KRT20. In some embodiments, treatment with the anti-RSPO antibody reduces expression of one or more stem cell biomarkers, *e.g*., Myc, Axin2, LGR5, TERT, BIRC5, and/or Ascl2. In some embodiments, treatment with the anti-RSPO antibody increases expression of one or more biomarkers of differentiation, *e.g*., CEACAM7, SLC26A3, CA1, SYT15, CA4, TFF1, and/or KRT20.

In a further aspect, the invention provides a method for treating a tumor, cell proliferative disorder, and/or cancer. In one embodiment, the method comprises administering to an individual having such tumor, cell proliferative disorder, and/or cancer an effective amount of an anti-RSPO3 antibody. In some embodiments, the cancer is adrenal cancer, bladder cancer, brain cancer, breast cancer, cervix cancer, colon cancer, head and neck cancer, kidney cancer, leukemia, liver cancer, lung cancer (*e.g*., NSCLC), lymphoid cancer, ovarian cancer, pancreatic cancer, prostate cancer, rectum cancer, skin cancer (*e.g*., melanoma), stomach cancer, thyroid cancer, and/or uterine cancer. In some embodiments, the cancer is lung cancer (*e.g.*, NSCLC), ovarian cancer, breast cancer, liver cancer, or multiple myeloma. In some embodiments, the cancer is colorectal cancer. In some embodiments, the cancer is gastrointestinal cancer, stomach cancer, colon cancer, colorectal cancer, lung cancer, or rectal cancer. In some embodiments, the method further comprises administering to the individual an effective amount of at least one additional therapeutic agent, *e.g.,* as described below.

An "individual" according to any of the above embodiments may be a human. In some embodiments, the individual and/or cancer has one or more biomarkers. In some embodiments, the one or more biomarkers comprise an RSPO translocation. In some embodiments, the RSPO translocation comprises and RSPO2 and/or RSPO3 translocation. In some embodiments, the individual and/or cancer has increased expression of one or more biomarker. In some embodiments, the one or more biomarkers comprise RSPO, *e.g*., RSPO2 and/or RSPO3. In some embodiments, the one or more biomarkers comprise an RSPO3 translocation. In some embodiments, the one or more biomarkers comprise a stem cell biomarker. In some embodiments, the stem cell biomarker comprises Myc, Axin2, LGR5, TERT, BIRC5, and/or Ascl2. In some embodiments, the individual and/or cancer has decreased expression of one or more biomarkers of differentiation. In some embodiments, the one or more biomarkers of differentiation comprise CEACAM7, SLC26A3, CA1, SYT15, CA4, TFF1, and/or KRT20. In some embodiments, treatment with the anti-RSPO3 antibody reduces expression of one or more stem cell biomarkers, *e.g*., Myc, Axin2, LGR5, TERT, BIRC5, and/or Ascl2. In some embodiments, treatment with the anti-RSPO antibody increases expression of one or more biomarkers of differentiation, *e.g*., CEACAM7, SLC26A3, CA1, SYT15, CA4, TFF1, and/or KRT20.

In a further aspect, the invention provides a method inhibiting wnt signaling, inhibiting angiogenesis, inhibiting cell proliferation, inhibiting cancer stem cell proliferation, and/or depleting cancer stem cells in an individual. In one embodiment, the method comprises administering to the individual an effective amount of an anti-RSPO3 antibody to inhibit wnt signaling, inhibit angiogenesis, inhibit cell proliferation, inhibit cancer stem cell proliferation, and/or deplete cancer stem cells. In one embodiment, an "individual" is a human. In some embodiments, the individual and/or cancer has one or more biomarker. In some embodiments, the one or more biomarkers comprise an RSPO translocation. In some embodiments, the RSPO translocation comprises and RSPO2 and/or RSPO3 translocation. In some embodiments the RSPO translocation is an RSPO3 translocation. In some embodiments, the individual and/or cancer has increased expression of one or more biomarkers. In some embodiments, the one or more biomarkers comprise RSPO, *e.g*., RSPO2 and/or RSPO3. In some embodiments, the one or more biomarkers comprise a stem cell biomarker. In some embodiments, the stem cell biomarker comprises Myc, Axin2, LGR5, TERT, BIRC5, and/or Ascl2. In some embodiments, the individual and/or cancer has decreased expression of one or more biomarkers of differentiation. In some embodiments, the one or more biomarkers of differentiation comprise CEACAM7, SLC26A3, CA1, SYTI5, CA4, TFF1, and/or KRT20. In some embodiments, treatment with the anti-RSPO3 antibody reduces expression of one or more stem cell biomarkers, *e.g*., Myc, Axin2, LGR5, TERT, BIRC5, and/or Ascl2. In some embodiments, treatment with the anti-RSPO3 antibody increases expression of one or more biomarkers of differentiation, *e.g*., CEACAM7, SLC26A3, CA1, SYTI5, CA4, TFF1, and/or KRT20. In some embodiments, the cancer is adrenal cancer, bladder cancer, brain cancer, breast cancer, cervix cancer, colon cancer, head and neck cancer, kidney cancer, leukemia, liver cancer, lung cancer (*e.g*., NSCLC), lymphoid cancer, ovarian cancer, pancreatic cancer, prostate cancer, rectum cancer, skin cancer (*e.g.*, melanoma), stomach cancer, thyroid cancer, and/or uterine cancer. In some embodiments, the cancer is lung cancer (*e.g.*, NSCLC), ovarian cancer, breast cancer, liver cancer, or multiple myeloma. In some embodiments, the cancer is colorectal cancer.

In a further aspect, the invention provides pharmaceutical formulations comprising any of the anti-RSPO3 antibodies provided herein, *e.g.,* for use in any of the above therapeutic methods. In one embodiment, a pharmaceutical formulation comprises any of the anti-RSPO3 antibodies provided herein and a pharmaceutically acceptable carrier. In another embodiment, a pharmaceutical formulation comprises any of the anti-RSPO3 antibodies provided herein and at least one additional therapeutic agent, *e.g.,* as described below.

In some embodiments, the cancer is adrenal cancer, bladder cancer, brain cancer, breast cancer, cervix cancer, colon cancer, head and neck cancer, kidney cancer, leukemia, liver cancer, lung cancer (*e.g.*, NSCLC), lymphoid cancer, ovarian cancer, pancreatic cancer, prostate cancer, rectum cancer, skin cancer (*e.g*., melanoma), stomach cancer, thyroid cancer, and/or uterine cancer. In some embodiments, the cancer is lung cancer (*e.g*., NSCLC), ovarian cancer, breast cancer, liver cancer, or multiple myeloma. In some embodiments, the cancer is colorectal cancer. In some embodiments, the cancer is gastrointestinal cancer, stomach cancer, colon cancer, colorectal cancer, lung cancer, or rectal cancer. In some embodiments, the method in which the formulation is used further comprises administering to the individual an effective amount of at least one additional therapeutic agent, *e.g.,* as described below. In some embodiments, the pharmaceutical formulation is used in a method inhibiting wnt signaling, inhibiting angiogenesis, inhibiting cell proliferation, inhibiting cancer stem cell proliferation, and/or depleting cancer stem cells in an individual. In one embodiment, the method comprises administering to the individual an effective amount of the pharmaceutical formulation to inhibit wnt signaling, inhibit angiogenesis, inhibit cell proliferation, inhibit cancer stem cell proliferation, and/or deplete cancer stem cells.

In some of the above embodiments, an "individual" is a human. In some embodiments, the pharmaceutical formulation is used in treating an individual and/or cancer having increased expression of one or more biomarker. In some embodiments, the one or more biomarkers comprise an RSPO, *e.g*., RSPO2 and/or RSPO3. In some embodiments, the one or more biomarkers comprise a stem cell biomarker. In some embodiments, the stem cell biomarker comprises Myc, Axin2, LGR5, TERT, BIRC5, and/or Ascl2. In some embodiments, the individual and/or cancer treated with the pharmaceutical formulation has decreased expression of one or more biomarkers of differentiation. In some embodiments, the biomarker of differentiation comprises CEACAM7, SLC26A3, CA1, SYT15, CA4, TFF1, and/or KRT20. In some embodiments, treatment with the pharmaceutical formulation reduces expression of one or more stem cell biomarkers, *e.g*., Myc, Axin2, LGR5, TERT, BIRC5, and/or Ascl2. In some embodiments, treatment with the pharmaceutical formulation increases expression of one or more biomarkers of differentiation, *e.g*., CEACAM7, SLC26A3, CA1, SYT15, CA4, TFF1, and/or KRT20.

Antibodies of the invention can be used either alone or in combination with other agents in a therapy. For instance, an antibody of the invention may be co-administered with at least one additional therapeutic agent. In certain embodiments, an additional therapeutic agent is a cytotoxic agent, chemotherapeutic agent, cytostatic agent, anti-hormonal agent, and/or EGFR inhibitor.

Such combination therapies noted above encompass combined administration (where two or more therapeutic agents are included in the same or separate formulations), and separate administration, in which case, administration of the antibody of the invention can occur prior to, simultaneously, and/or following, administration of the additional therapeutic agent or agents. In one embodiment, administration of the anti-RSPO3 antibody and administration of an additional therapeutic agent occur within about one month, or within about one, two or three weeks, or within about one, two, three, four, five, or six days, of each other. Antibodies of the invention can also be used in combination with radiation therapy.

An antibody of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 µg/kg to 15 mg/kg *(e.g.* 0.1 mg/kg-10 mg/kg) of antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, *e.g.* every week or every three weeks (*e.g.* such that the patient receives from about two to about twenty, or *e.g.* about six doses of the antibody). An initial higher loading dose, followed by one or more lower doses may be administered. However, other dosage regimens may be useful. The progress of this therapy is easily monitored by conventional techniques and assays.

It is understood that any of the above formulations or therapeutic methods may be carried out using an immunoconjugate of the invention in place of or in addition to an anti-RSPO antibody.

### H. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to an anti-RSPO antibody.

### III. WORKING EXAMPLES

The following are examples of methods and compositions of the invention. It is understood that various other embodiments may be practiced, given the general description provided above.

### EXAMPLE I

### Development and Characterization of Rat Anti-Human RSPO3 Hybridoma Antibodies

In effort to generate anti-RSPO3 antibodies, Sprague Dawley rats (Charles River, Hollister, CA) were immunized twice weekly with 20 µg of human RSPO3 protein (Genentech) mixed with MPL+TDM adjuvant (Sigma-Aldrich, St. Louis, MO) divided among sites: intraperitoneal (i.p.), subcutaneous (s.c.) at base of tail, s.c. at nape of neck, and s.c. in both hocks. Multiple lymph nodes were harvested two days after the last immunization. IgM negative B-cells from these rats were purified from lymphocytes using magnetic separation (Miltenyi Biotec, San Diego, CA) and were fused with P3X63-Ag8U.1 mouse myeloma cells (American Type Culture Collection, Rockville, MD) via electrofusion (Harvard Apparatus, Holliston, MA). Fused cells were incubated at 37°C, 7% CO2, overnight in Medium C (StemCell Technologies, Vancouver, BC, Canada), before resuspension in semi-solid Medium D (StemCell Technologies) with anti-rat IgG-FITC (Sigma-Aldrich) and plating into Omniwell trays (Thermo Fisher Scientific, Rochester, NY).

Seven days after plating, fluorescent colonies were selected and transferred into 96-well plates containing Medium E (StemCell Technologies) using a Clonepix2 FL (Molecular Devices, Sunnyvale, CA). Supernatants were screened by ELISA against human RSPO3 protein six days after picking. Human RSPO3 binding hybridoma cell lines were expanded and retested by ELISA. Supernatants from cell lines demonstrating binding to both human and cyno RSPO3 with no cross-reactivity to human RSPO1, RSPO2, or RSPO4 proteins by ELISA were harvested and purified by protein G (GammaBind Plus, GE Healthcare, Pittsburgh, PA).

Three monoclonal antibodies, 4A6, 11C10, and 15F3 were selected for humanization and further study based on their affinities for recombinant RSPO3 proteins and IC50 values in a cell-based assay.

### EXAMPLE II

### Humanization and Binding Affinity of Humanized anti-RSPO3 Antibodies

The 4A6, 11C10 and 15F3 anti-RSPO3 antibodies were humanized as described below. Residue numbers are according to Kabat et al., Sequences of proteins of immunological interest, 5th Ed., Public Health Service, National Institutes of Health, Bethesda, Md. (1991).

Variants constructed during the humanization process were assessed in the form of Fab. The VL and VH domains from rat hybridoma clones were aligned to the closest human germline sequences. For the humanization of 4A6, hypervariable regions were engineered into VLK1-39 and VH3-30 acceptor frameworks. Specifically, from the rat 4A6 VL domain, positions 24-34 (L1), 50-56 (L2) and 89-97 (L3) were grafted into VLK1-39 and from the rat 4A6 VH domain; positions 26-35 (HI), 50-65 (H2) and 95-102 (H3) were grafted into VH3-30. All VL and VH Vernier positions from rat 4A6 were also grafted to the VLK1-39 and VH3-30, respectively. This graft is referred to as hu4A6.L1H1.

For the humanization of 11C10, hypervariable regions were engineered into VLK1D-13 and VH3-30 acceptor frameworks. Specifically, from the rat 11C10 VL domain, positions 24-34 (L1), 50-56 (L2) and 89-97 (L3) were grafted into VLK1-39 and from the rat 11C10 VH domain; positions 26-35 (HI), 50-65 (H2) and 95-102 (H3) were grafted into VH3-30. All VL and VH Vernier positions from rat 11C10 were also grafted to the VLK1D-13 and VH3-30, respectively. This graft is referred to as hu11C10.L1H1.

For the humanization of 15F3, hypervariable regions were engineered into VLK1-39 and VH3-30 acceptor frameworks. Specifically, from the rat 15F3 VL domain, positions 24-34 (L1), 50-56 (L2) and 89-97 (L3) were grafted into VLK1-39 and from the rat 15F3 VH domain; positions 26-35 (HI), 50-65 (H2) and 95-102 (H3) were grafted into VH3-30. All VL and VH Vernier positions from rat 15F3 were also grafted to the VLK1 and VH4, respectively. This graft is referred to as hu15F3.L1H1.

Figures 1 and 2 show examples of humanized 4A6, 11C10 and 15F3 antibody light and heavy chain variable regions. See also the sequences in Table 6 below.

The binding affinity of the humanized antibodies in this section was determined by BIAcore™ T200 format. Briefly, BIAcore™ research grade CM5 chips were activated with 1-ethyl-3-(3-dimethylaminopropyl) carbodiimide (EDC) and N-hydroxysuccinimide (NHS) reagents according to the supplier's instructions. Human RSPO3 (huRSPO3) was immobilized to achieve approximately 30 response units (RU) in each flow cell. Unreacted coupling groups were blocked with 1M ethanolamine. For kinetics measurements, four-fold serial dilutions of variant antibody was injected in HBS-P buffer (0.01M HEPES pH7.4, 0.15M NaCl, 0.005% surfactant P20) at 25°C. with a flow rate of 30 (µl/min. Association rates (kon) and dissociation rates (koff) were calculated using a 1:1 Langmuir binding model (BIAcore™ T200 Evaluation Software version 2.0). The equilibrium dissociation constant (Kd) was calculated as the ratio koff/kon.

The binding affinities of hu4A6.L1H1, hu11C10.L1H1 and hul5F3.L1H1 antibodies to human RSPO3 were compared to the chimeric counterparts 4A6, 11C10, and 15F3. Rat Vernier positions in each of hu4A6.L1H, hul1C10.L1H1 and hul 5F3.L1H1 were converted back to human residues to evaluate the contribution of rat Vernier positions to binding to hRSPO3. For hu4A6, three additional light chains (L2: L1 + Ala43, L3: L1 + Tyr87, L4: CDR graft only) and one additional heavy chain (H2: CDR graft only) were made. For hu11C10, four additional light chains (L2: L1+ Leu4, L3: L1+ Ala43, L4: L1+ Tyr87, L5: CDR graft only) were made. For hu15F3, three additional light chains (L2: L1+ Ala43, L3: L1+ Tyr87, L4: CDR graft only) and one additional heavy chain (H2: CDR graft only) were made. Based on binding affinity evaluation of the variant antibodies described above (data not shown), CDR grafts version of each of clones retains sufficient affinity toward human RSPO3.

Chimeric 4A6 bound to human RSPO3 with a KD of 3.6E-10 M, while hu4A6.L4H2 bound with a KD of 4.3E-10M. Chimeric 11C10 bound with a KD of 2.7E-9 M, while hu11C10.L5H1 bound with a KD of 3.0E-9M. Chimeric 15F3 bound with a KD of 8.5E-10 M, while hu15F3.L4H2 bound with a KD of 8.4E-10M.

The hu4A6.L4H2, hu11C10.L5H1, and hu15F3.L4H2 and their chimeric counterparts were tested for their ability to bind cynomolgus (cyno) and murine RSPO3 as described above except that cyno RSPO3 or murine RSPO3 (R&D Systems Cat. No. 4120-RS/CF) replaced human RSPO3 in the binding assay.

Affinities of two previously identified anti-RSPO3 antibodies 5D6 (see WO2015/058132) and antibody A (see WO 2014/012007; humanized IgG1 antibody 131R010) to human, cyno, and mouse RSPO3 were also tested in this assay system.

Binding properties for these antibodies are shown below in Table 3.

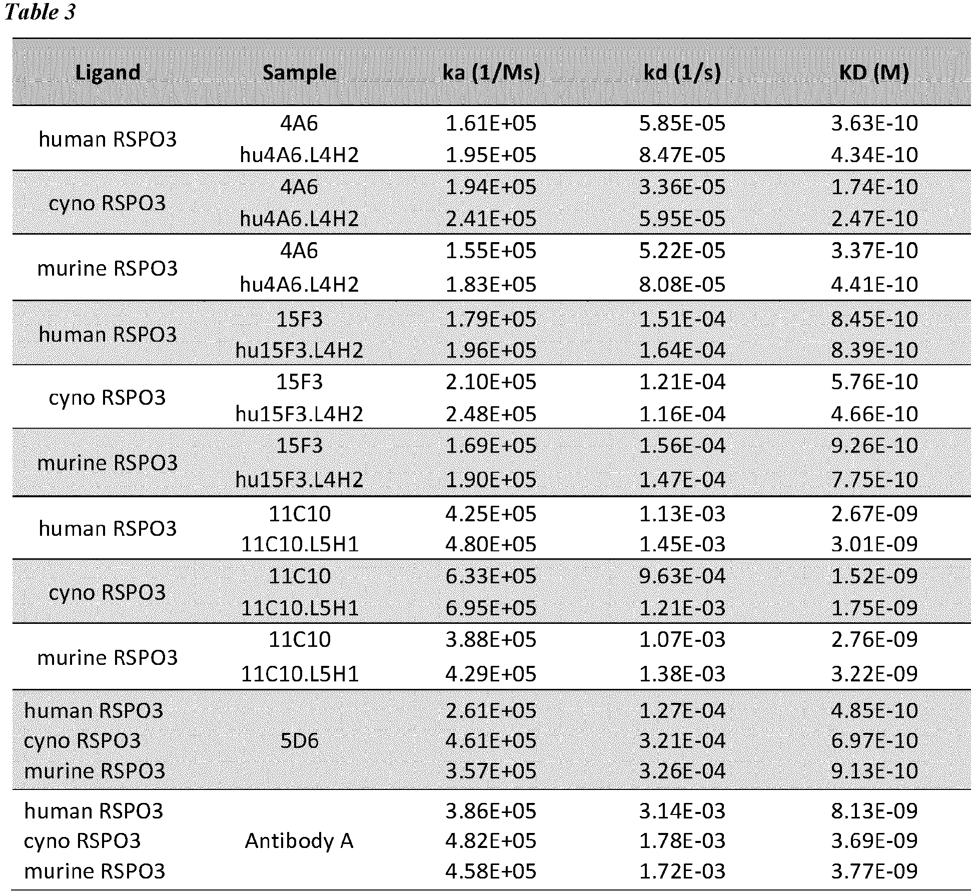

The humanized antibodies were tested under thermal stress (40°C, pH 5.5,2 weeks) and 2,2'-azobis (2-amidinopropane) hydrochloride (AAPH) Analysis. Then samples were thermally stressed to mimic stability over the shelf life of the products. The samples were buffer exchanged into 20mM His Acetate, 240mM sucrose, pH 5.5 and diluted to a concentration of 1 mg/mL. One mL of each sample was stressed at 40°C for 2 weeks and a second was stored at -70°C as a control. Both samples were then digested using trypsin to create peptides that could be analyzed using liquid chromatography(LC) - mass spectrometry(MS) analysis. For each peptide in the sample retention time, from the LC as well as high resolution accurate mass and peptide ion fragmentation information (amino acid sequence information) were acquired in the MS. Extracted ion chromatograms (XIC) were taken for peptides of interest (native and modified peptide ions) from the data sets at a window of +-10 ppm and peaks were integrated to determine area. Relative percentages of modification were calculated for each sample by taking the (area of the modified peptide) divided by (area of the modified peptide plus the area of the native peptide) multiplied by 100. All three humanized antibodies hu4A6.L4H2, hu11C10.LSH1, and hu15F3.L4H2 were found to be stable in the thermal stress and AAPH analyses.

### EXAMPLE III

### In Vivo Efficacy

The effect of anti-RSPO3 antibody administration on tumor growth was evaluated in two colorectal cancer PTPRK-RSPO3 fusion patient-derived tumor models CRC-D (Crown Biosciences model CR3150) and CRC-C (Crown Biosciences model CR2506). Humanized IgG1 antibodies hu4A6.L4H2, hu11C10.L5H1, and hu15F3.L4H2, further comprising an N to G modification at EU position 297 in the heavy chain intended to reduce binding to Fc gamma receptors were tested against a previously identified murine antibody 5D6 also comprising an N to G modification at position 297 (see WO2015/058132). Note that CRC-C exhibits a differentiation phenotype, where mucin production contributes to an overestimation of actual tumor volume. The humanized antibodies 4A6, 15F3, and 11C10, representing a 7-fold range in affinity for human RSPO3, all promoted tumor regression or growth stasis comparable to the previously identified 5D6 antibody in both models (dosing regimen = IP; QWx6). As shown in Figures 3a and 3b, anti-RSPO3 treatment resulted in delayed onset of a significant, yet durable reduction in tumor growth, either resulting in tumor regression as observed in the CRC-D model (Fig. 3a), or growth delay/stasis as seen in the CRC-C model (Fig. 3b). The graphed analysis used Linear Mixed Effect modeling to fit the data.

The activity of the humanized 4A6 antibody, the highest affinity anti-RSPO3 antibody, was then used for a comparison with a previously identified anti-RSPO3 called antibody A (WO2014/012007, humanized IgG1 antibody 131R010) in the CRC-D and CRC-C models. The antibody A does not contain a modification at N297. As exhibited in Figure 3c, both 4A6 and antibody A elicited dose-dependent regression in the CRC-D model. However, 4A6 was ~10-fold more potent. When compared in the CRC-C model (Figure 3d), 4A6 remained superior to antibody A at all tested dose levels (dosing regimen = IV, Q2Wx3).

Treatment of the models with anti-RSPO3 antibodies showed significant reduction in tumor growth or stasis of tumor growth. (Figs. 3a-3d.) In the models, the onset of regression and/or stasis was not immediate upon treatment with the anti-RSPO3 antibodies; there was a delay in the onset of regression or stasis after initiation of treatment. While not wanting to be bound by any particular theory, these efficacy data are consistent with a hierarchical organization of RSPO3 fusion positive tumors in which the proliferation of the cancer stem cells is dependent upon RSPO proteins, and upon treatment with anti-RSPO3 antibody, the cancer stem cells die or differentiate into transit-amplifying (TA) cells. In the absence of a stem cell source to ensure their replenishment, the TA cells undergo a limited number of cell divisions, after which they terminally differentiate, leading to their exhaustion. Therefore, the kinetics and the overall size of the TA cell population may determine the onset of tumor growth inhibition.

Again while not wanting to be bound by any particular theory, based on this theory of hierarchical organization of RSPO3 fusion positive tumors described, combination treatment with a chemotherapeutic agent may reduce the delay in onset of tumor regression and/or stasis by killing the TA cell population and thus, may have increased efficacy compared to treatment with the chemotherapeutic agent alone in PTPRK-RSPO3 fusion patient derived tumor models. By administering an anti-RSPO3 antibody in combination with chemotherapy, both cancer stem cells and TA cells may be targeted for earlier regression or stasis of tumor growth.

### EXAMPLE IV

### Pharmacokinetics of anti-RSPO3 Antibodies in Mice and Cynomolgus Monkeys

The pharmacokinetics of the humanized IgG1, N297G anti-RSPO3 antibodies hu4A6.L4H2, hu15F3.L5H1, and hu11C10.L4H2, were evaluated in female Balb/c nude mice at a single intravenous (IV) dose of 5 mg/kg and in cynomolgus monkeys at a single IV dose of 10 mg/kg. The previously identified anti-RSPO3 antibody 5D6 was also included in a mouse study of the same design and in a separate monkey study at doses of 3 mg/kg and 30 mg/kg. A control anti-gD antibody was also included in the mouse study.

The mice (n=9 per group; Charles River Laboratories (Hollister, CA)) were 6 to 11 weeks old and weighed approximately 14.0-20.5 g at the initiation of the study. The monkeys (n=4 per group; Chinese origin; Covance Research Products Inc. (Alice, Texas)) were 3 to 4 years old and weighed approximately 2.6-3.4 kg at the initiation of the study. At selected times throughout the study, blood samples were collected from each animal and used to derive total antibody concentrations in serum by ELISA. Specifically, the concentrations of all antibodies in serum were assayed using a GRIP ELISA. GRIP ELISA used a sheep anti-human IgG as the capturing reagent and a goat anti-human IgG conjugated to horseradish peroxidase (HRP) as the detection reagent. The assay sensitivity is 0.98 ng/mL and 9.8 ng/mL in mouse and monkey serum, respectively. The presence of anti-therapeutic antibodies (ATAs) in monkey serum samples was analyzed using a colorimetric assay and animals that developed detectable ATA responses were not included in the PK analysis.

A naive, pooled approach was used in mouse with a non-compartmental model while individual PK parameters were estimated in monkey with a non-compartmental model for 5D6 and using a 2-compartmental elimination model for 4A6, 15F3, and 11C10 (Phoenix™ WinNonlin®, Version 6.4; Pharsight Corporation; Mountain View, CA). Nominal sample collection time and nominal dose concentrations were used in the data analysis.

In the mouse PK study, at earlier time points, the humanized 4A6, 15F3, 11C10 antibodies demonstrated biphasic disposition profiles typical of IgG1 antibodies and were indistinguishable from the control anti-gD antibody (Figure 4). At later time points, anti-RSPO3 antibodies showed a rapid decrease in concentrations compared to anti-gD, which is likely due to the dominance of target-mediated clearance at lower concentration ranges at the 5 mg/kg dose level. Total exposures measured by area under the curve up to the last measurable concentration (AUCₗₐₛₜ) for the 4A6, 15F3, and 11C10 antibodies were 364±9.39, 300±14.6, and 377±13.6, respectively. The AUCₗₐₛₜ for the 4A6, 15F3, 11C10 and 5D6 antibodies were approximately 20-50% lower than anti-gD control (AUCₗₐₛₜ=450±17.3 day^{∗}µg/mL) (Table 4). 5D6 showed rapid clearance compared to the other antibodies with AUCₗₐₛₜ of 280±16.8 day^{∗}µg/mL, more than 60% lower than the AUCₗₐₛₜ of the anti-gD control (Figure 4 and Table 4).

**Table 4: Pharmacokinetic Parameter Estimates (Mean ± SE) of anti-RSPO3 and anti-gD Antibodies after IV Administration in Balb/c Nude mice**

| **Dose (mg/kg)** | **Test Material** | **Cₘₐₓ (µg/mL)** | **AUCₗₐₛₜ (day^{∗}µg/mL)** | **CL (mL/day/kg)** | **Vₛₛ (mL/kg)** |
|---|---|---|---|---|---|
| 5 | 4A6 | 94.0±3.34 | 364±9.39 | 13.7 | 88.1 |
| 5 | 15F3 | 77.2±2.92 | 300±14.6 | 16.6 | 97.7 |
| 5 | 11C10 | 91.4±1.37 | 377±13.6 | 13.0 | 103 |
| 5 | 5D6 | 78.0±6.99 | 280±16.8 | 17.8 | 103 |
| 5 | anti-gD | 82.0±2.93 | 450±17.3 | 9.92 | 128 |
| AUCₗₐₛₜ = area under the serum concentration up to the last measurable concentration; CL = clearance; Cₘₐₓ = maximum concentration; Vss = volume of distribution at steady state. | | | | | |

In cynomolgus monkeys, the humanized anti-RSPO3 antibodies 4A6, 15F3, and 11C10 exhibited similar biphasic PK profiles with a short distribution phase followed by a long elimination phase following a single IV bolus dose of 10 mg/kg (Figure 5a). The PK parameters were comparable for the three anti-RSPO3 antibodies, with mean clearance (CL) ranging from 4.60 to 5.22 mL/day/kg and t_{1/2} ranging from 8.47 to 10.7 days. These data are consistent with those of typical IgG1 antibodies (Deng et al. 2011). A separate monkey PK study with 5D6 was conducted at dose levels of 3 and 30 mg/kg. 5D6, in contrast to 4A6, 11C10, and 15F3, demonstrated a distinctly different PK profile: following a single IV bolus dose of 3 and 30 mg/kg, the concentration-time profiles showed a fast decline throughout the time course of the study with large variability (Figure 5b). The mean clearance (CL) for the two doses ranged from 13.7 to 17.1±4.73 mL/day/kg, which was 3-6 times higher than expected for typical IgG1 antibodies (CL range: ∼3-6 mL/day/kg; Deng et al. 2011).

**Table 5: Pharmacokinetic Parameter Estimates (Mean ± SD) of anti-RSPO3 Antibody after IV Administration in Cynomolgus Monkeys**

| **Dose (mg/kg)** | **Test Material** | **Cmax (µg/mL)** | **AUC (day*µg/mL)** | **CL (mL/day/kg)** | **t_{1/2,} β (day)** | **Vₛₛ (mL/kg)** |
|---|---|---|---|---|---|---|
| 10 | *4A6 (n=2) | 277; 288 | 1870; 2360 | 4.24; 5.35 | 9.37; 12.1 | 69.0; 71.3 |
| 10 | * 15F3 (n=2) | 232; 270 | 1460;2780 | 3.60; 6.83 | 6.68; 10.3 | 52.1; 62.7 |
| 10 | 11C10 (n=3) | 316±30.4 | 2230±439 | 4.60±0.814 | 8.47±1.41 | 52.9±1.97 |
| 3 | 5D6 (n=3) | 89.7±3.52 | 172+50.9 | 18.4±5.22 | NR | 41.6±4.01 |
| 30 | *5D6 (n=2) | 1060; 970 | 1760; 2800 | 17.1; 10.7 | 4.32; 3.81 | 33.5; 35.1 |
| 10 (normalized from 30 mg/kg) | *5D6 (n=2) | 353; 323 | 587; 933 | 17.1; 10.7 | 4.32; 3.81 | 33.5; 35.1 |
| Cₘₐₓ = maximum concentration; AUC = area under the serum concentration versus time curve; CL = clearance; NR = not reported; t_{1/2,β} = beta-phase half-life; Vₛₛ = volume of distribution at steady state. | | | | | | |
| *Animals tested positive for anti-therapeutic antibody were excluded from the mean and SD calculations. Individual values were reported if n < 3. n=2 for 4A6, 15F3 and 30 mg/kg 5D6; n=3 for 11C10 and 3 mg/kg 5D6. | | | | | | |
| Note: 4A6, 15F3, and 11C10 were analyzed by a 2-compartmental model while 5D6 was analyzed with a non-compartmental model. | | | | | | |

Overall, the three humanized anti-RSPO3 antibodies 4A6, 15F3, and 11C10 demonstrated profiles typical of IgG1 antibodies and showed significant improvement over the previous antibody 5D6 in cynomolgus monkeys.

### EXAMPLE V

### Competition ELISA Assays Show Effect of Different anti-RSPO3 Antibodies on Blocking RSPO3 Binding to Each of LGR4, LGR5, and RNF43

To measure the activity of anti-RSPO3 antibodies in blocking the binding of LGR4 and LGR5 extracellular domains (ECDs) to RSPO3, MaxiSorp® 384-well microwell plates (Thermo Scientific Nunc, Roskilde, Denmark) were coated with 25 ul/well of 0.5 µg/ml hRSPO3 (Genentech) in 50 mM carbonate buffer, pH 9.6, overnight at 4°C. Plates were blocked with at 80 ul/well of 0.5% bovine serum albumin, 15 parts per million Proclin™ 300 in phosphate buffered saline (PBS), pH 7.4, for 1 hour. Serially diluted murine IgG2a anti-RSPO3 antibodies (0.078-10 ng/ml in 3-fold serial dilution plus buffer blank) containing 0.1 µg/ml LGR4-Fc or 0.015 µg/ml LGR5-Fc in assay buffer (0.5% BSA, 0.05% polysorbate 20, 15 parts per million Proclin™ 300 in PBS) were added to the plates at 25 ul/well. The following antibodies were tested along with a buffer control: mouse anti-Ragweed mIgG2a control, 5D6 mIgG2a, 4A6 mIgG2a with L234A, L235A, and P329G substitutions (LALAPG), which reduce effector function, 11C10 mIgG2 LALAPG, 15F3 mIgG2 LALAPG, and antibody A mIgG2 LALAPG (see WO 2014/012007; humanized antibody 131R010 and its parental 131R003 antibody, for the variable region sequences of antibody A).

After a 2-hour incubation, LGR4-Fc and LGR5-Fc bound to the plates were detected using peroxidase labeled goat F(ab')2 anti-human Fc (Jackson ImmunoResearch, West Grove, PA). After a 1 hour incubation, the substrate 3,3',5,5'-tetramethyl benzidine (Moss Inc., Pasadena, Maryland) was added to the plates and the reaction was stopped by adding 1 M phosphoric acid. Plates were washed with PBS, pH 7.4, containing 0.05% Tween® 20, between steps and all the incubation steps following the coating step were performed at room temperature on an orbital shaker. Absorbance was read at 450 nm on a multiscan Ascent® reader (Thermo Scientific, Hudson, NH).

The activities of the above anti-RSPO3 antibodies in blocking binding of RNF43 to RSPO3 were measured similarly using 20 ng/ml biotinylated RNF43-Flag on RSPO3 coated plates. Bound biotinylated RNF43-Flag was detected using peroxidase labeled streptavidin (GE Healthcare, Piscataway, NJ) followed by the substrate as described above.

Results of the three competition ELISA assays are shown in **Figs. 6a-6c** (LGR4 in **Fig. 6a****,** LGR5 in **Fig. 6b****,** and RNF43 in **Fig. 6c**). As can be seen in **Fig. 6a****,** the 4A6, 11C10, and 15F3 antibodies were superior to both the 5D6 and antibody A in blocking LGR4 binding to RSPO3. The IC50 for 4A6, 11C10, and 15F3 in this experiment are 0.046 µg/ml, 0.035 µg/ml, and 0.045 µg/ml, respectively, while the IC50 for 5D6 is 0.07 µg/ml and that of antibody A is 0.105 µg/ml. Similar results are shown in **Fig. 6b****,** where the IC50 for 4A6, 11C10, and 15F3 are 0.083, 0.063, and 0.079 µg/ml respectively, while the IC50 for 5D6 is 0.110 µg/ml and that for antibody A is 0.143 µg/ml. With respect to RNF43 binding, shown in **Fig. 6c****,** all of 5D6, 4A6, 11C10, and 15F3 effectively blocked RNF43 binding to RSPO3 with similar IC50 of 0.050, 0.042, 0.035, and 0.047 µg/ml, respectively, while antibody A did not block RNF43 binding to RSPO3 and thus, no IC50 value was obtained for antibody A.

Similar results to those shown in **Fig. 6c** have been obtained using 5D6, 4A6, 11C10, 15F3, and antibody A constructs having human IgG1 heavy chains with an N297G mutation to reduce effector function in place of the murine IgG2a LALAPG mutant heavy chain used for the experiments shown here (data not shown). Specifically, in that experiment, all of 5D6, 4A6, 11C10, and 15F3 effectively blocked RNF43 binding to RSPO3 with similar IC50 of about 0.038 to 0.050 µg/ml, while antibody A did not block RNF43 binding to RSPO3 and thus, no IC50 value was obtained for antibody A.

Collectively, the experimental data on RNF43 binding described in this example indicate that antibody A may bind to a different epitope on RSPO3 than the 5D6, 4A6, 11C10, and 15F3 antibodies.

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

### ADDITIONAL SEQUENCES:

SEQ ID NO: 1 >sp|Q6UXX9|RSPO2_HUMAN R-spondin-2 OS=Homo sapiens GN=RSPO2
MQFRLFSFALIIILNCMDYSHCQGNRWRRSKRASYVSNPICKGCLSCSKDNGCSRCQQKLF
FFLRREGMRQYGECLHSCPSGYYGHRAPDMNRCARCRIENCDSCFSKDFCTKCKVGFYLH
RGRCFDECPDGFAPLEETMECVEGCEVGHWSEWGTCSRNNRTCGFKWGLETRTRQIVKKP
VKDTILCPTIAESRRCKMTMRHCPGGKRTPKAKEKRNKKKKRKLIERAQEQHSVFLATDR
ANQ

SEQ ID NO:2 >sp|Q9BXY4|RSPO3_HUMAN R-spondin-3 OS=Homo sapiens GN=RSPO3
MHLRLISWLFIILNFMEYIGSQNASRGRRQRRMHPNVSQGCQGGCATCSDYNGCLSCKPR
LFFALERIGMKQIGVCLSSCPSGYYGTRYPDINKCTKCKADCDTCFNKNFCTKCKSGFYL
HLGKCLDNCPEGLEANNHTMECVSIVHCEVSEWNPWSPCTKKGKTCGFKRGTETRVREII
QHPSAKGNLCPPTNETRKCTVQRKKCQKGERGKKGRERKRKKPNKGESKEAIPDSKSLES
SKEIPEQRENKQQQKKRKVQDKQKSVSVSTVH

SEQ ID NO:3 >sp|Q2MKA7|RSPO1_HUMAN R-spondin-1 OS=Homo sapiens GN=RSPO1
MRLGLCVVALVLSWTHLTISSRGIKGKRQRRISAEGSQACAKGCELCSEVNGCLKCSPKL
FILLERNDIRQVGVCLPSCPPGYFDARNPDMNKCIKCKIEHCEACFSHNFCTKCKEGLYL
HKGRCYPACPEGSSAANGTMECSSPAQCEMSEWSPWGPCSKKQQLCGFRRGSEERTRRVL
HAPVGDHAACSDTKETRRCTVRRVPCPEGQKRRKGGQGRRENANRNLARKESKEAGAGSR
RRKGQQQQQQGTVGPLTSAGPA

SEQ ID NO:4 >sp|Q2I0M5|RSPO4_HUMAN R-spondin-4 OS=Homo sapiens GN=RSPO4
MRAPLCLLLLVAHAVDMLALNRRKKQVGTGLGGNCTGCIICSEENGCSTCQQRLFLFIRR
EGIRQYGKCLHDCPPGYFGIRGQEVNRCKKCGATCESCFSQDFCIRCKRQFYLYKGKCLP
TCPPGTLAHQNTRECQGECELGPWGGWSPCTHNGKTCGSAWGLESRVREAGRAGHEEAAT
CQVLSESRKCPIQRPCPGERSPGQKKGRKDRRPRKDRKLDRRLDVRPRQPGLQP

**Table 6 - Antibody Sequences**

| NAME | SEQUENCE | SEQ ID NO |
|---|---|---|
| 4A6-HVR L1 | LASEDISNDLV | 5 |
| 4A6-HVR L2 | AASRLQD | 6 |
| 4A6-HVR L3 | QQSYKYLPT | 7 |
| 4A6-HVR H1 | DYDMA | 8 |
| 4A6-HVR H2 | TIIYDGSRTYYRDSVKG | 9 |
| 4A6-HVR H3 | HDRSFDY | 10 |
| 11C10-HVR L1 | RASEDIYSDLA | 11 |
| 11C10-HVR L2 | DVNSLIH | 12 |
| 11C10-HVR L3 | QQYDNYPNT | 13 |
| 11C10-HVR H1 | DYDMA | 14 |
| 11C10-HVR H2 | TIIYDGSRTYYRDSVKG | 15 |
| 11C10-HVR H3 | HDKTFDY | 16 |
| 15F3-HVR L1 | LVSEDISNDFV | 17 |
| 15F3-HVR L2 | AASRLQD | 18 |
| 15F3-HVR L3 | QQSYKYPPT | 19 |
| 15F3-HVR H1 | DYDMA | 20 |
| 15F3-HVR H2 | TIIYDGSRAYFGDSVRG | 21 |
| 15F3-HVR H3 | HDRSFDY | 22 |
| 4A6 V_{L} | DIQMTQSPASLSASLGETVSIECLASEDISNDLVWYQQKSGKSPQLLIYAASRL QDGVPSRFSGSGFGTRFSLKISGMQPEDEADYFCQQSYKYLPTFGAGTKLGLK | 23 |
| 4A6 V_{H} | EVQLVESGGGSVQPGRSLKVSCAASGFTFSDYDMAWVRQAPKKGLEWVATIIYD GSRTYYRDSVKGRFTLSRDNTKSTLCLQMDSLRSEDTATYYCAAHDRSFDYWGQ GIMVTVSS | 24 |
| hu4A6.L1H1 V_{L} | DIQMTQSPSSLSASVGDRVTITCLASEDISNDLVWYQQKPGKSPKLLIYAASRL QDGVPSRFSGSGSGTDFTLTISSLQPEDFATYFCQQSYKYLPTFGQGTKLEIK | 25 |
| hu4A6.L1H1 V_{H} | EVQLVESGGGVVQPGRSLRLSCAASGFTFSDYDMAWVRQAPGKGLEWVATIIYD GSRTYYRDSVKGRFTLSRDNSKNTLYLQMNSLRAEDTAVYYCAAHDRSFDYWGQ GTMVTVSS | 26 |
| hu4A6.L1H2 V_{L} | DIQMTQSPSSLSASVGDRVTITCLASEDISNDLVWYQQKPGKSPKLLIYAASRL QDGVPSRFSGSGSGTDFTLTISSLQPEDFATYFCQQSYKYLPTFGQGTKLEIK | 27 |
| hu4A6.L1H2 V_{H} | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCAAHDRSFDYWGQ GTMVTVSS | 28 |
| hu4A6.L2H1 V_{L} | DIQMTQSPSSLSASVGDRVTITCLASEDISNDLVWYQQKPGKAPKLLIYAASRL QDGVPSRFSGSGSGTDFTLTISSLQPEDFATYFCQQSYKYLPTFGQGTKLEIK | 29 |
| hu4A6.L2H1 V_{H} | EVQLVESGGGVVQPGRSLRLSCAASGFTFSDYDMAWVRQAPGKGLEWVATIIYD GSRTYYRDSVKGRFTLSRDNSKNTLYLQMNSLRAEDTAVYYCAAHDRSFDYWGQ GTMVTVSS | 30 |
| hu4A6.L3H1 V_{L} | DIQMTQSPSSLSASVGDRVTITCLASEDISNDLVWYQQKPGKSPKLLIYAASRL QDGVPSRFSGSGSGTDFTLTISSLQPEDFATYYCQQSYKYLPTFGQGTKLEIK | 31 |
| hu4A6.L3H1 V_{H} | EVQLVESGGGVVQPGRSLRLSCAASGFTFSDYDMAWVRQAPGKGLEWVATIIYD GSRTYYRDSVKGRFTLSRDNSKNTLYLQMNSLRAEDTAVYYCAAHDRSFDYWGQ GTMVTVSS | 32 |
| hu4A6.L4H1 V_{L} | DIQMTQSPSSLSASVGDRVTITCLASEDISNDLVWYQQKPGKAPKLLIYAASRL QDGVPSRFSGSGFGTDFTLTISSLQPEDFATYYCQQSYKYLPTFGQGTKLEIK | 33 |
| hu4A6.L4H1 V_{H} | EVQLVESGGGVVQPGRSLRLSCAASGFTFSDYDMAWVRQAPGKGLEWVATIIYD GSRTYYRDSVKGRFTLSRDNSKNTLYLQMNSLRAEDTAVYYCAAHDRSFDYWGQ GTMVTVSS | 34 |
| hu4A6.L4H2 V_{L} | DIQMTQSPSSLSASVGDRVTITCLASEDISNDLVWYQQKPGKAPKLLIYAASRL QDGVPSRFSGSGFGTDFTLTISSLQPEDFATYYCQQSYKYLPTFGQGTKLEIK | 35 |
| hu4A6.L4H2 V_{H} | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCAAHDRSFDYWGQ GTMVTVSS | 36 |
| 11C10 V_{L} | DIQMTQSPASLSASLGETVTIECRASEDIYSDLAWYQQKPGNSPQLLIYDVNSL IHGVPSRFSGSGSGTQFSLKINNLQSEDVASYFCQQYDNYPNTFGAGTKLELK | 37 |
| 11C10 V_{H} | EVQLVESGGGLVQPGRSLKLSCAASGFTFSDYDMAWVRQAPKKGLEWVATIIYD GSRTYYRDSVKGRFTISRANAKSTLYLQMDSLRSEDTATYYCATHDKTFDYWGQ GVMVTVSS | 38 |
| hu11C10.L1H1 V_{L} | DIQMTQSPSSLSASVGDRVTITCRASEDIYSDLAWYQQKPGKSPKLLIYDVNSL IHGVPSRFSGSGSGTDFTLTISSLQPEDFATYFCQQYDNYPNTFGQGTKLEIK | 39 |
| hu11C10.L1H1 V_{H} | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCATHDKTFDYWGQ GTMVTVSS | 40 |
| hu11C10.L2H1 V_{L} | DIQLTQSPSSLSASVGDRVTITCRASEDIYSDLAWYQQKPGKSPKLLIYDVNSL IHGVPSRFSGSGSGTDFTLTISSLQPEDFATYFCQQYDNYPNTFGQGTKLEIK | 41 |
| hu11C10.L2H1 V_{H} | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCATHDKTFDYWGQ GTMVTVSS | 42 |
| hu11C10.L3H1 V_{L} | DIQMTQSPSSLSASVGDRVTITCRASEDIYSDLAWYQQKPGKAPKLLIYDVNSL IHGVPSRFSGSGSGTDFTLTISSLQPEDFATYFCQQYDNYPNTFGQGTKLEIK | 43 |
| hu11C10.L3H1 V_{H} | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCATHDKTFDYWGQ GTMVTVSS | 44 |
| hu11C10.L4H1 V_{L} | DIQMTQSPSSLSASVGDRVTITCRASEDIYSDLAWYQQKPGKSPKLLIYDVNSL IHGVPSRFSGSGSGTDFTLTISSLQPEDFATYYCQQYDNYPNTFGQGTKLEIK | 45 |
| hu11C10.L4H1 V_{H} | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCATHDKTFDYWGQ GTMVTVSS | 46 |
| hu11C10.L5H1 V_{L} | DIQLTQSPSSLSASVGDRVTITCRASEDIYSDLAWYQQKPGKAPKLLIYDVNSL IHGVPSRFSGSGSGTDFTLTISSLQPEDFATYYCQQYDNYPNTFGQGTKLEIK | 47 |
| hu11C10.L5H1 V_{H} | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCATHDKTFDYWGQ GTMVTVSS | 48 |
| 15F3 V_{L} | DIQMTQSPASLSASLGETVSIECLVSEDISNDFVWYQQKSGKSPQLLIYAASRL QDGVPSRFSGSGSGTRFSLRISGMQPEDEAEYFCQQSYKYPPTFGAGTKLELK | 49 |
| 15F3 V_{H} | EVQLVESGGGLVQPGRSLKLSCAASGFTFSDYDMAWVRQAPKKGLEWVATIIYD GSRAYFGDSVRGRFTVSRDNTKSTLYLQMDSLRSEDTATYYCTAHDRSFDYWGQ GVMVTVSS | 50 |
| hu15F3.L1H1 V_{L} | DIQMTQSPSSLSASVGDRVTITCLVSEDISNDFVWYQQKPGKSPKLLIYAASRL QDGVPSRFSGSGSGTDFTLTISSLQPEDFATYFCQQSYKYPPTFGQGTKLEIK | 51 |
| hu15F3.L1H1 V_{H} | EVQLVESGGGVVQPGRSLRLSCAASGFTFSDYDMAWVRQAPGKGLEWVATIIYD GSRAYFGDSVRGRFTVSRDNSKNTLYLQMNSLRAEDTAVYYCTAHDRSFDYWGQ GTMVTVSS | 52 |
| hu15F3.L1H2 V_{L} | DIQMTQSPSSLSASVGDRVTITCLVSEDISNDFVWYQQKPGKSPKLLIYAASRL QDGVPSRFSGSGSGTDFTLTISSLQPEDFATYFCQQSYKYPPTFGQGTKLEIK | 53 |
| hu15F3.L1H2 V_{H} | EVQLVESGGGVVQPGRSLRLSCAASGFTFSDYDMAWVRQAPGKGLEWVATIIYD GSRAYFGDSVRGRFTISRDNSKNTLYLQMNSLRAEDTAVYYCTAHDRSFDYWGQ GTMVTVSS | 54 |
| hu15F3.L2H1 V_{L} | DIQMTQSPSSLSASVGDRVTITCLVSEDISNDFVWYQQKPGKAPKLLIYAASRL QDGVPSRFSGSGSGTDFTLTISSLQPEDFATYFCQQSYKYPPTFGQGTKLEIK | 55 |
| hu15F3.L2H1 V_{H} | EVQLVESGGGVVQPGRSLRLSCAASGFTFSDYDMAWVRQAPGKGLEWVATIIYD GSRAYFGDSVRGRFTVSRDNSKNTLYLQMNSLRAEDTAVYYCTAHDRSFDYWGQ GTMVTVSS | 56 |
| hu15F3.L3H1 V_{L} | DIQMTQSPSSLSASVGDRVTITCLVSEDISNDFVWYQQKPGKSPKLLIYAASRL QDGVPSRFSGSGSGTDFTLTISSLQPEDFATYYCQQSYKYPPTFGQGTKLEIK | 57 |
| hu15F3.L3H1 V_{H} | EVQLVESGGGVVQPGRSLRLSCAASGFTFSDYDMAWVRQAPGKGLEWVATIIYD GSRAYFGDSVRGRFTVSRDNSKNTLYLQMNSLRAEDTAVYYCTAHDRSFDYWGQ GTMVTVSS | 58 |
| hu15F3.L4H1 V_{L} | DIQMTQSPSSLSASVGDRVTITCLVSEDISNDFVWYQQKPGKAPKLLIYAASRL QDGVPSRFSGSGSGTDFTLTISSLQPEDFATYYCQQSYKYPPTFGQGTKLEIK | 59 |
| hu15F3.L4H1 V_{H} | EVQLVESGGGVVQPGRSLRLSCAASGFTFSDYDMAWVRQAPGKGLEWVATIIYD GSRAYFGDSVRGRFTVSRDNSKNTLYLQMNSLRAEDTAVYYCTAHDRSFDYWGQ GTMVTVSS | 60 |
| hu15F3.L4H2 V_{L} | DIQMTQSPSSLSASVGDRVTITCLVSEDISNDFVWYQQKPGKAPKLLIYAASRL QDGVPSRFSGSGSGTDFTLTISSLQPEDFATYYCQQSYKYPPTFGQGTKLEIK | 61 |
| hu15F3.L4H2 V_{H} | EVQLVESGGGVVQPGRSLRLSCAASGFTFSDYDMAWVRQAPGKGLEWVATIIYD GSRAYFGDSVRGRFTISRDNSKNTLYLQMNSLRAEDTAVYYCTAHDRSFDYWGQ GTMVTVSS | 62 |
| 4A6 Light Chain | DIQMTQSPASLSASLGETVSIECLASEDISNDLVWYQQKSGKSPQLLIYAASRL QDGVPSRFSGSGFGTRFSLKISGMQPEDEADYFCQQSYKYLPTFGAGTKLGLKR ADAAPTVSIFPPSMEQLTSGGATVVCFVNNFYPRDISVKWKIDGSEQRDGVLDS VTDQDSKDSTYSMSSTL | 63 |
| 4A6 Heavy Chain | EVQLVESGGGSVQPGRSLKVSCAASGFTFSDYDMAWVRQAPKKGLEWVATIIYD GSRTYYRDSVKGRFTLSRDNTKSTLCLQMDSLRSEDTATYYCAAHDRSFDYWGQ GIMVTVSSAETTAPSVYPL | 64 |
| hu4A6.L1H1 Light Chain | DIQMTQSPSSLSASVGDRVTITCLASEDISNDLVWYQQKPGKSPKLLIYAASRL QDGVPSRFSGSGSGTDFTLTISSLQPEDFATYFCQQSYKYLPTFGQGTKLEIK | 65 |
| hu4A6.L1H1 Heavy Chain | EVQLVESGGGVVQPGRSLRLSCAASGFTFSDYDMAWVRQAPGKGLEWVATIIYD GSRTYYRDSVKGRFTLSRDNSKNTLYLQMNSLRAEDTAVYYCAAHDRSFDYWGQ GTMVTVSS | 66 |
| hu4A6.L1H2 Light Chain | DIQMTQSPSSLSASVGDRVTITCLASEDISNDLVWYQQKPGKSPKLLIYAASRL QDGVPSRFSGSGSGTDFTLTISSLQPEDFATYFCQQSYKYLPTFGQGTKLEIK | 67 |
| hu4A6.L1H2 Heavy Chain | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCAAHDRSFDYWGQ GTMVTVSS | 68 |
| hu4A6.L2H1 Light Chain | DIQMTQSPSSLSASVGDRVTITCLASEDISNDLVWYQQKPGKAPKLLIYAASRL QDGVPSRFSGSGSGTDFTLTISSLQPEDFATYFCQQSYKYLPTFGQGTKLEIK | 69 |
| hu4A6.L2H1 Heavy Chain | EVQLVESGGGVVQPGRSLRLSCAASGFTFSDYDMAWVRQAPGKGLEWVATIIYD GSRTYYRDSVKGRFTLSRDNSKNTLYLQMNSLRAEDTAVYYCAAHDRSFDYWGQ GTMVTVSS | 70 |
| hu4A6.L3H1 Light Chain | DIQMTQSPSSLSASVGDRVTITCLASEDISNDLVWYQQKPGKSPKLLIYAASRL QDGVPSRFSGSGSGTDFTLTISSLQPEDFATYYCQQSYKYLPTFGQGTKLEIK | 71 |
| hu4A6.L3H1 Heavy Chain | EVQLVESGGGVVQPGRSLRLSCAASGFTFSDYDMAWVRQAPGKGLEWVATIIYD GSRTYYRDSVKGRFTLSRDNSKNTLYLQMNSLRAEDTAVYYCAAHDRSFDYWGQ GTMVTVSS | 72 |
| hu4A6.L4H1 Light Chain | DIQMTQSPSSLSASVGDRVTITCLASEDISNDLVWYQQKPGKAPKLLIYAASRL QDGVPSRFSGSGFGTDFTLTISSLQPEDFATYYCQQSYKYLPTFGQGTKLEIK | 73 |
| hu4A6.L4H1 Heavy Chain | EVQLVESGGGVVQPGRSLRLSCAASGFTFSDYDMAWVRQAPGKGLEWVATIIYD GSRTYYRDSVKGRFTLSRDNSKNTLYLQMNSLRAEDTAVYYCAAHDRSFDYWGQ GTMVTVSS | 74 |
| hu4A6.L4H2 Light Chain | DIQMTQSPSSLSASVGDRVTITCLASEDISNDLVWYQQKPGKAPKLLIYAASRL QDGVPSRFSGSGFGTDFTLTISSLQPEDFATYYCQQSYKYLPTFGQGTKLEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC | 75 |
| hu4A6.L4H2 Heavy Chain (wild type human IgGl; position N297 in bold) | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCAAHDRSFDYWGQ GTMVTVSSASTKGPSVFPLAPSSKSTSGGTAALGCLVKDYFPEPVTVSWNSGAL TSGVHTFPAVLQSSGLYSLSSVVTVPSSSLGTQTYI CNVNHKPSNTKVDKKVEP KSCDKTHTCPPCPAPELLGGPSVFLFPPKPKDTLMI SRTPEVTCVVVDVSHEDP EVKFNWYVDGVEVHNAKTKPREEQYNSTYRVVSVLTVLHQDWLNGKEYKCKVSN KALPAPIEKTISKAKGQPREPQVYTLPPSREEMTKNQVSLTCLVKGFYPSDIAV EWESNGQPENNYKTTPPVLDSDGSFFLYSKLTVDKS RWQQGNVFSCSVMHEALH NHYTQKSLSLSPGK | 76 |
| hu4A6.L4H2 Heavy Chain (human IgGl N297G) | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCAAHDRSFDYWGQ GTMVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDP EVKFNWYVDGVEVHNAKTKPREEQYGSTYRVVSVLTVLHQDWLNGKEYKCKVSN KALPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK | 171 |
| 11C10 Light Chain | DIQMTQSPASLSASLGETVTIECRASEDIYSDLAWYQQKPGNSPQLLIYDVNSL IHGVPSRFSGSGSGTQFSLKINNLQSEDVASYFCQQYDNYPNTFGAGTKLELKR ADAAPTVSIFPPSMEQLTSGGATVVCFVNNFYPRDISVKWKIDGSEQRDGVLDS VTDQDSKDSTYSMSSTL | 77 |
| 11C10 Heavy Chain | EVQLVESGGGLVQPGRSLKLSCAASGFTFSDYDMAWVRQAPKKGLEWVATIIYD GSRTYYRDSVKGRFTISRANAKSTLYLQMDSLRSEDTATYYCATHDKTFDYWGQ GVMVTVSSAETTAPSVYPL | 78 |
| hu11C10.L1H1 Light Chain | DIQMTQSPSSLSASVGDRVTITCRASEDIYSDLAWYQQKPGKSPKLLIYDVNSL IHGVPSRFSGSGSGTDFTLTISSLQPEDFATYFCQQYDNYPNTFGQGTKLEIK | 79 |
| hu11C10.L1H1 Heavy Chain | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCATHDKTFDYWGQ GTMVTVSS | 80 |
| hu11C10.L2H1 Light Chain | DIQLTQSPSSLSASVGDRVTITCRASEDIYSDLAWYQQKPGKSPKLLIYDVNSL IHGVPSRFSGSGSGTDFTLTISSLQPEDFATYFCQQYDNYPNTFGQGTKLEIK | 81 |
| hu11C10.L2H1 Heavy Chain | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCATHDKTFDYWGQ GTMVTVSS | 82 |
| hu11C10.L3H1 Light Chain | DIQMTQSPSSLSASVGDRVTITCRASEDIYSDLAWYQQKPGKAPKLLIYDVNSL IHGVPSRFSGSGSGTDFTLTISSLQPEDFATYFCQQYDNYPNTFGQGTKLEIK | 83 |
| hu11C10.L3H1 Heavy Chain | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCATHDKTFDYWGQ GTMVTVSS | 84 |
| hu11C10.L4H1 Light Chain | DIQMTQSPSSLSASVGDRVTITCRASEDIYSDLAWYQQKPGKSPKLLIYDVNSL IHGVPSRFSGSGSGTDFTLTISSLQPEDFATYYCQQYDNYPNTFGQGTKLEIK | 85 |
| hu11C10.L4H1 Heavy Chain | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCATHDKTFDYWGQ GTMVTVSS | 86 |
| hu11C10.L5H1 Light Chain | DIQLTQSPSSLSASVGDRVTITCRASEDIYSDLAWYQQKPGKAPKLLIYDVNSL IHGVPSRFSGSGSGTDFTLTISSLQPEDFATYYCQQYDNYPNTFGQGTKLEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC | 87 |
| hu11C10. L5H1 Heavy Chain (human IgGl wild-type; N297 in bold) | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCATHDKTFDYWGQ GTMVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSN KALPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK | 88 |
| hu11C10. L5H1 Heavy Chain (human IgGl N297G) | EVQLVESGGGVVQPGRSLRLSCAASGFTFSDYDMAWVRQAPGKGLEWVATIIYD GSRTYYRDSVKGRFTISRDNSKNTLYLQMNSLRAEDTAVYYCATHDKTFDYWGQ GTMVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDP EVKFNWYVDGVEVHNAKTKPREEQYGSTYRVVSVLTVLHQDWLNGKEYKCKVSN KALPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK | 172 |
| 15F3 Light Chain | DIQMTQSPASLSASLGETVSIECLVSEDISNDFVWYQQKSGKSPQLLIYAASRL QDGVPSRFSGSGSGTRFSLRISGMQPEDEAEYFCQQSYKYPPTFGAGTKLELK | 89 |
| 15F3 Heavy Chain | EVQLVESGGGLVQPGRSLKLSCAASGFTFSDYDMAWVRQAPKKGLEWVATIIYD GSRAYFGDSVRGRFTVSRDNTKSTLYLQMDSLRSEDTATYYCTAHDRSFDYWGQ GVMVTVSSAETTAPSVYPL | 90 |
| hu15F3.L1H1 Light Chain | DIQMTQSPSSLSASVGDRVTITCLVSEDISNDFVWYQQKPGKSPKLLIYAASRL QDGVPSRFSGSGSGTDFTLTISSLQPEDFATYFCQQSYKYPPTFGQGTKLEIK | 91 |
| hu15F3.L1H1 Heavy Chain | EVQLVESGGGVVQPGRSLRLSCAASGFTFSDYDMAWVRQAPGKGLEWVATIIYD GSRAYFGDSVRGRFTVSRDNSKNTLYLQMNSLRAEDTAVYYCTAHDRSFDYWGQ GTMVTVSS | 92 |
| hu15F3.L1H2 Light Chain | DIQMTQSPSSLSASVGDRVTITCLVSEDISNDFVWYQQKPGKSPKLLIYAASRL QDGVPSRFSGSGSGTDFTLTISSLQPEDFATYFCQQSYKYPPTFGQGTKLEIK | 93 |
| hu15F3.L1H2 Heavy Chain | EVQLVESGGGVVQPGRSLRLSCAASGFTFSDYDMAWVRQAPGKGLEWVATIIYD GSRAYFGDSVRGRFTISRDNSKNTLYLQMNSLRAEDTAVYYCTAHDRSFDYWGQ GTMVTVSS | 94 |
| hu15F3.L2H1 Light Chain | DIQMTQSPSSLSASVGDRVTITCLVSEDISNDFVWYQQKPGKAPKLLIYAASRL QDGVPSRFSGSGSGTDFTLTISSLQPEDFATYFCQQSYKYPPTFGQGTKLEIK | 95 |
| hu15F3.L2H1 Heavy Chain | EVQLVESGGGVVQPGRSLRLSCAASGFTFSDYDMAWVRQAPGKGLEWVATIIYD GSRAYFGDSVRGRFTVSRDNSKNTLYLQMNSLRAEDTAVYYCTAHDRSFDYWGQ GTMVTVSS | 96 |
| hu15F3.L3H1 Light Chain | DIQMTQSPSSLSASVGDRVTITCLVSEDISNDFVWYQQKPGKSPKLLIYAASRL QDGVPSRFSGSGSGTDFTLTISSLQPEDFATYYCQQSYKYPPTFGQGTKLEIK | 97 |
| hu15F3.L3H1 Heavy Chain | EVQLVESGGGVVQPGRSLRLSCAASGFTFSDYDMAWVRQAPGKGLEWVATIIYD GSRAYFGDSVRGRFTVSRDNSKNTLYLQMNSLRAEDTAVYYCTAHDRSFDYWGQ GTMVTVSS | 98 |
| hu15F3.L4H1 Light Chain | DIQMTQSPSSLSASVGDRVTITCLVSEDISNDFVWYQQKPGKAPKLLIYAASRL QDGVPSRFSGSGSGTDFTLTISSLQPEDFATYYCQQSYKYPPTFGQGTKLEIK | 99 |
| hu15F3.L4H1 Heavy Chain | EVQLVESGGGVVQPGRSLRLSCAASGFTFSDYDMAWVRQAPGKGLEWVATIIYD GSRAYFGDSVRGRFTVSRDNSKNTLYLQMNSLRAEDTAVYYCTAHDRSFDYWGQ GTMVTVSS | 100 |
| hu15F3.L4H2 Light Chain | DIQMTQSPSSLSASVGDRVTITCLVSEDISNDFVWYQQKPGKAPKLLIYAASRL QDGVPSRFSGSGSGTDFTLTISSLQPEDFATYYCQQSYKYPPTFGQGTKLEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC | 101 |
| hu15F3.L4H2 Heavy Chain (human IgGl; N297 in bold) | EVQLVESGGGVVQPGRSLRLSCAASGFTFSDYDMAWVRQAPGKGLEWVATIIYD GSRAYFGDSVRGRFTISRDNSKNTLYLQMNSLRAEDTAVYYCTAHDRSFDYWGQ GTMVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSN KALPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK | 102 |
| hu15F3.L4H2 Heavy Chain (human IgGl N297G) | EVQLVESGGGVVQPGRSLRLSCAASGFTFSDYDMAWVRQAPGKGLEWVATIIYD GSRAYFGDSVRGRFTISRDNSKNTLYLQMNSLRAEDTAVYYCTAHDRSFDYWGQ GTMVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDP EVKFNWYVDGVEVHNAKTKPREEQYGSTYRVVSVLTVLHQDWLNGKEYKCKVSN KALPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK | 173 |

EIF3E(e1)-RSPO2(e2) translocation fusion polynucleotide (SEQ ID NO:103) 
GAGCACAGACTCCCTTTCTTTGGCAAGATGGCGGAGTACGACTTGACTACTCGCATCGCGCACTTTTTGGATCGGCAT
CTAGTCTTTCCGCTTCTTGAATTTCTCTCTGTAAAGGAGGTTCGTGGCGGAGAGATGCTGATCGCGCTGAACTGACCGG
TGCGGCCCGGGGGTGAGTGGCGAGTCTCCCTCTGAGTCCTCCCCAGCAGCGCGGCCGGCGCCGGCTCTTTGGGCGAACC
CTCCAGTTCCTAGACTTTGAGAGGCGTCTCTCCCCCGCCCGACCGCCCAGATGCAGTTCGCCTTTTCTCCTTTGCCCT
CATCATTCTGAACTGCATGGATTACAGCCACTGCCAAGGCAACCGATGGAGACGCAGTAAGCGAGCTAGTTATGTATCA
AATCCCATTTGCAAGGGTTGTTTGTCTTGTTCAAAGGACAATGGGTGTAGCCGATGTCAACAGAAGTTGTTCTTCTTCC
TTCGAAGAGAAGGATGCGCCAGTATGGAGAGTGCCTGCATTCCTGCCCATCCGGGTACTATGGACACCGAGCCCCAGA
TATGAACAGATGTGCAAGATGCAGAATAGAAAACTGTGATTCTTGCTTTAGCAAAGACTTTTGTACCAAGTGCAAAGTA
GGCTTTTATTTGCATAGAGGCCGTTGCTTTGATGAATGTCCAGATGGTTTTGCACCATTAGAAGAAACCATGGAATGTG
TGGAAGGATGTGAAGTTGGTCATTGGAGCGAATGGGGAACTTGTAGCAGAAATAATCGCACATGTGGATTTAAATGGGG
TCTGGAAACCAGAACACGGCAAATTGTTAAAAAGCCAGTGAAAGACACAATACTGTGTCCAACCATTGCTGAATCCAGG
AGATGCAAGATGACAATGAGGCATTGTCCAGGAGGGAAGAGAACACCAAAGGCGAAGGAGAAGAGGAACAAGAAAAAGA
AAAGGAAGCTGATAGAAAGGGCCCAGGAGCAACACAGCGTCTTCCTAGCTACAGACAGAGCTAACCAATAA
EIF3E(e1)-RSPO2(e2) translocation fusion polypeptide sequence (SEQ ID NO:104) 
MAEYDLTTRIAHFLDRHLVFPLLEFLSVKEVRGGEMLIALNMQFRLFSFALIIILNCMDYSHCQGNRWRRSKRASYVSNP
ICKGCLSCSKDNGCSRCQQKLFFFLRREGMRQYGECLHSCPSGYYGHRAPDMNRCARCRIENCDSCFSKDFCTKCKVGF
YLHRGRCFDECPDGFAPLEETMECVEGCEVGHWSEWGTCSRNNRTCGFKWGLETRTRQIVKKPVKDTILCPTIAESRRC
KMTMRHCPGGKRTPKAKEKRNKKKKRKLIERAQEQHSVFLATDRANQ
PTPRK(e1)-RSPO3(e2) translocation fusion polynucleotide sequence (SEQ ID NO:105) 
ATGGATACGACTGCGGCGGCGGCGCTGCCTGCTTTTGTGGCGCTCTTGCTCCTCTCTCCTTGGCCTCTCCTGGGATCGG
CCCAAGGCCAGTTCTCCGCAGTGCATCCTAACGTTAGTCAAGGCTGCCAAGGAGGCTGTGCAACATGCTCAGATTACAA
TGGATGTTTGTCATGTAAGCCCAGACTATTTTTTGCTCTGGAAAGAATTGGCATGAAGCAGATTGGAGTATGTCTCTCT
TCATGTCCAAGTGGATATTATGGAACTCGATATCCAGATATAAATAAGTGTACAAAATGCAAAGCTGACTGTGATACCT
GTTTCAACAAAAATTTCTGCACAAAATGTAAAAGTGGATTTTACTTACACCTTGGAAAGTGCCTTGACAATTGCCCAGA
AGGGTTGGAAGCCAACAACCATACTATGGAGTGTGTCAGTATTGTGCACTGTGAGGTCAGTGAATGGAATCCTTGGAGT
 
CCATGCACGAAGAAGGAAAAACATGTGGCTTCAAAAGAGGGACTGAAACACGGGTCCGAGAAATAATACAGCATCCTT
CAGCAAAGGGTAACCTGTGTCCCCCAACAAATGAGACAAGAAAGTGTACAGTGCAAAGGAAGAAGTGTCAGAAGGAGA
ACGAGGAAAAAAAGGAAGGGAGAGGAAAAGAAAAAAACCTAATAAAGGAGAAAGTAAAGAAGCAATACCTGACAGCAAA
AGTCTGGAATCCAGCAAAGAAATCCCAGAGCAACGAGAAAACAAACAGCAGCAGAAGAAGCGAAAAGTCCAAGATAAAC
AGAAATCGGTATCAGTCAGCACTGTACACTAG
PTPRK(e1)-RSPO3(e2) translocation fusion polypeptide sequence (SEQ ID NO:106) 
MDTTAAAALPAFVALLLLSPWPLLGSAQGQFSAVHPNVSQGCQGGCATCSDYNGCLSCKPRLFFALERIGMKQIGVCLS
SCPSGYYGTRYPDINKCTKCKADCDTCFNKNFCTKCKSGFYLHLGKCLDNCPEGLEANNHTMECVSIVHCEVSEWNPWS
PCTKKGKTCGFKRGTETRVREIIQHPSAKGNLCPPTNETRKCTVQRKKCQKGERGKKGR
PTPRK(e7)-RSPO3(e2) translocation fusion polynucleotide sequence (SEQ ID NO:107) 
ATGGATACGACTGCGGCGGCGGCGCTGCCTGCTTTTGTGGCGCTCTTGCTCCTCTCTCCTTGGCCTCTCCTGGGATCGG
CCCAAGGCCAGTTCTCCGCAGGTGGCTGTACTTTTGATGATGGTCCAGGGGCCTGTGATTACCACCAGGATCTGTATGA
TGACTTTGAATGGGTGCATGTTAGTGCTCAAGAGCCTCATTATCTACCACCCGAGATGCCCCAAGGTTCCTATATGATA
GTGGACTCTTCAGATCACGACCCTGGAGAAAAAGCCAGACTTCAGCTGCCTACAATGAAGGAGAACGACACTCACTGCA
TTGATTTCAGTTACCTATTATATAGCCAGAAAGGACTGAATCCTGGCACTTTGAACATATTAGTTAGGGTGAATAAAGG
ACCTCTTGCCAATCCAATTTGGAATGTGACTGGATTCACGGGTAGAGATTGGCTTCGGGCTGAGCTAGCAGTGAGCACC
TTTTGGCCCAATGAATATCAGGTAATATTTGAAGCTGAAGTCTCAGGAGGGAGAAGTGGTTATATTGCCATTGATGACA
TCCAAGTACTGAGTTATCCTTGTGATAAATCTCCTCATTTCCTCCGTCTAGGGGATGTAGAGGTGAATGCAGGGCAAAA
CGCTACATTTCAGTGCATTGCCACAGGGAGAGATGCTGTGCATAACAAGTTATGGCTCCAGAGACGAAATGGAGAAGAT
ATACCAGTAGCCCAGACTAAGAACATCAATCATAGAAGGTTTGCCGCTTCCTTCAGATTGCAAGAAGTGACAAAAACTG
ACCAGGATTTGTATCGCTGTGTAACTCAGTCAGAACGAGGTTCCGGTGTGTCCAATTTTGCTCAACTTATTGTGAGAGA
ACCGCCAAGACCCATTGCTCCTCCTCAGCTTCTTGGTGTTGGGCCTACATATTTGCTGATCCAACTAAATGCCAACTCG
ATCATTGGCGATGGTCCTATCATCCTGAAAGAAGTAGAGTACCGAATGACATCAGGATCCTGGACAGAAACCCATGCAG
TCAATGCTCCAACTTACAAATTATGGCATTTAGATCCAGATACCGAATATGAGATCCGAGTTCTACTTACAAGACCTGG
TGAAGGTGGAACGGGGCTCCCAGGACCTCCACTAATCACCAGAACAAAATGTGCAGTGCATCCTAACGTTAGTCAAGGC
TGCCAAGGAGGCTGTGCAACATGCTCAGATTACAATGGATGTTTGTCATGTAAGCCCAGACTATTTTTTGCTCTGGAAA
GAATTGGCATGAAGCAGATTGGAGTATGTCTCTCTTCATGTCCAAGTGGATATTATGGAACTCGATATCCAGATATAAA
TAAGTGTACAAAATGCAAAGCTGACTGTGATACCTGTTTCAACAAAAATTTCTGCACAAAATGTAAAAGTGGATTTTAC
TTACACCTTGGAAAGTGCCTTGACAATTGCCCAGAAGGGTTGGAAGCCAACAACCATACTATGGAGTGTGTCAGTATTG
TGCACTGTGAGGTCAGTGAATGGAATCCTTGGAGTCCATGCACGAAGAAGGGAAAAACATGTGGCTTCAAAAGAGGGAC
TGAAACACGGGTCCGAGAAATAATACAGCATCCTTCAGCAAAGGGTAACCTGTGTCCCCCAACAAATGAGACAAGAAAG
TGTACAGTGCAAAGGAAGAAGTGTCAGAAGGGAGAACGAGGAAAAAAAGGAAGGGAGAGGAAAAGAAAAAAACCTAATA
AAGGAGAAAGTAAAGAAGCAATACCTGACAGCAAAAGTCTGGAATCCAGCAAAGAAATCCCAGAGCAACGAGAAAACAA
ACAGCAGCAGAAGAAGCGAAAAGTCCAAGATAAACAGAAATCGGTATCAGTCAGCACTGTACACTAG
PTPRK(e7)-RSPO3(e2) translocation fusion polypeptide sequence (SEQ ID NO:108)

MDTTAAAALPAFVALLLLSPWPLLGSAQGQFSAGGCTFDDGPGACDYHQDLYDDFEWVHVSAQEPHYLPPEMPQGSYMI
VDSSDHDPGEKARLQLPTMKENDTHCIDFSYLLYSQKGLNPGTLNILVRVNKGPLANPIWNVTGFTGRDWLRAELAVST
FWPNEYQVIFEAEVSGGRSGYIAIDDIQVLSYPCDKSPHFLRLGDVEVNAGQNATFQCIATGRDAVHNKLWLQRRNGED
IPVAQTKNINHRRFAASFRLQEVTKTDQDLYRCVTQSERGSGVSNFAQLIVREPPRPIAPPQLLGVGPTYLLIQLNANS
IIGDGPIILKEVEYRMTSGSWTETHAVNAPTYKLWHLDPDTEYEIRVLLTRPGEGGTGLPGPPLITRTKCAVHPNVSQG
CQGGCATCSDYNGCLSCKPRLFFALERIGMKQIGVCLSSCPSGYYGTRYPDINKCTKCKADCDTCFNKNFCTKCKSGFY
LHLGKCLDNCPEGLEANNHTMECVSIVHCEVSEWNPWSPCTKKGKTCGFKRGTETRVREIIQHPSAKGNLCPPTNETRK
CTVQRKKCQKGERGKKGRERKRKKPNKGESKEAIPDSKSLESSKEIPEQRENKQQQKKRKVQDKQKSVSVSTVH

### WHAT IS DISCLOSED INCLUDES:

1. An isolated antibody that binds to human R-spondin 3 (RSPO3), wherein the antibody comprises:
   (a) a light chain variable region (VL) comprising (i) a first light chain hypervariable region (HVR-L1) comprising the amino acid sequence of SEQ ID NO:5, (ii) HVR-L2 comprising the amino acid sequence of SEQ ID NO:6, and (iii) HVR-L3 comprising the amino acid sequence of SEQ ID NO:7; and a heavy chain variable region (VH) comprising (i) a first heavy chain hypervariable region (HVR-H1) comprising the amino acid sequence of SEQ ID NO:8, (ii) HVR-H2 comprising the amino acid sequence of SEQ ID NO:9, and (iii) HVR-H3 comprising the amino acid sequence of SEQ ID NO:10;
   (b) a VL comprising (i) HVR-L1 comprising the amino acid sequence of SEQ ID NO:11, (ii) HVR-L2 comprising the amino acid sequence of SEQ ID NO: 12, and (iii) HVR-L3 comprising the amino acid sequence of SEQ ID NO: 13; and a VH comprising (i) HVR-H1 comprising the amino acid sequence of SEQ ID NO: 14, (ii) HVR-H2 comprising the amino acid sequence of SEQ ID NO: 15, and (iii) HVR-H3 comprising the amino acid sequence of SEQ ID NO: 16; or
   (c) a VL comprising (i) HVR-L1 comprising the amino acid sequence of SEQ ID NO: 17, (ii) HVR-L2 comprising the amino acid sequence of SEQ ID NO: 18, and (iii) HVR-L3 comprising the amino acid sequence of SEQ ID NO: 19; and a VH comprising (i) HVR-H1 comprising the amino acid sequence of SEQ ID NO:20, (ii) HVR-H2 comprising the amino acid sequence of SEQ ID NO:21, and (iii) HVR-H3 comprising the amino acid sequence of SEQ ID NO:22.
2. The isolated antibody of 1, wherein the antibody comprises
   (a) a VL sequence comprising SEQ ID NO:23 and a VH sequence comprising SEQ ID NO:24;
   (b) a VL sequence comprising SEQ ID NO:25 and a VH sequence comprising SEQ ID NO:26;
   (c) a VL sequence comprising SEQ ID NO:27 and a VH sequence comprising SEQ ID NO:28;
   (d) a VL sequence comprising SEQ ID NO:29 and a VH sequence comprising SEQ ID NO:30;
   (e) a VL sequence comprising SEQ ID NO:31 and a VH sequence comprising SEQ ID NO:32;
   (f) a VL sequence comprising SEQ ID NO:33 and a VH sequence comprising SEQ ID NO:34;
   (g) a VL sequence comprising SEQ ID NO:35 and a VH sequence comprising SEQ ID NO:36;
   (h) a VL sequence comprising SEQ ID NO:37 and a VH sequence comprising SEQ ID NO:38;
   (i) a VL sequence comprising SEQ ID NO:39 and a VH sequence comprising SEQ ID NO:40;
   (j) a VL sequence comprising SEQ ID NO:41 and a VH sequence comprising SEQ ID NO:42;
   (k) a VL sequence comprising SEQ ID NO:43 and a VH sequence comprising SEQ ID NO:44;
   (l) a VL sequence comprising SEQ ID NO:45 and a VH sequence comprising SEQ ID NO:46;
   (m) a VL sequence comprising SEQ ID NO:47 and a VH sequence comprising SEQ ID NO:48;
   (n) a VL sequence comprising SEQ ID NO:49 and a VH sequence comprising SEQ ID NO:50;
   (o) a VL sequence comprising SEQ ID NO:51 and a VH sequence comprising SEQ ID NO:52;
   (p) a VL sequence comprising SEQ ID NO:53 and a VH sequence comprising SEQ ID NO:54;
   (q) a VL sequence comprising SEQ ID NO:55 and a VH sequence comprising SEQ ID NO:56;
   (r) a VL sequence comprising SEQ ID NO:57 and a VH sequence comprising SEQ ID NO:58;
   (w) a VL sequence comprising SEQ ID NO:59 and a VH sequence comprising SEQ ID NO:60; or
   (x) a VL sequence comprising SEQ ID NO:61 and a VH sequence comprising SEQ ID NO:62.
3. The isolated antibody of 1 or 2, wherein the antibody comprises
   (a) a light chain sequence comprising SEQ ID NO:63 and a heavy chain sequence comprising SEQ ID NO:64;
   (b) a light chain sequence comprising SEQ ID NO:65 and a heavy chain sequence comprising SEQ ID NO:66;
   (c) a light chain sequence comprising SEQ ID NO:67 and a heavy chain sequence comprising SEQ ID NO:68;
   (d) a light chain sequence comprising SEQ ID NO:69 and a heavy chain sequence comprising SEQ ID NO:70;
   (e) a light chain sequence comprising SEQ ID NO:71 and a heavy chain sequence comprising SEQ ID NO:72;
   (f) a light chain sequence comprising SEQ ID NO:73 and a heavy chain sequence comprising SEQ ID NO:74;
   (g) a light chain sequence comprising SEQ ID NO:75 and a heavy chain sequence comprising SEQ ID NO:76 or 171;
   (h) a light chain sequence comprising SEQ ID NO:77 and a heavy chain sequence comprising SEQ ID NO:78;
   (i) a light chain sequence comprising SEQ ID NO:79 and a heavy chain sequence comprising SEQ ID NO:80;
   (j) a light chain sequence comprising SEQ ID NO:81 and a heavy chain sequence comprising SEQ ID NO:82;
   (k) a light chain sequence comprising SEQ ID NO:83 and a heavy chain sequence comprising SEQ ID NO:84;
   (l) a light chain sequence comprising SEQ ID NO:85 and a heavy chain sequence comprising SEQ ID NO:86;
   (m) a light chain sequence comprising SEQ ID NO:87 and a heavy chain sequence comprising SEQ ID NO:88 or 172;
   (n) a light chain sequence comprising SEQ ID NO:89 and a heavy chain sequence comprising SEQ ID NO:90;
   (o) a light chain sequence comprising SEQ ID NO:91 and a heavy chain sequence comprising SEQ ID NO:92;
   (p) a light chain sequence comprising SEQ ID NO:93 and a heavy chain sequence comprising SEQ ID NO:94;
   (q) a light chain sequence comprising SEQ ID NO:95 and a heavy chain sequence comprising SEQ ID NO:96;
   (r) a light chain sequence comprising SEQ ID NO:97 and a heavy chain sequence comprising SEQ ID NO:98;
   (w) a light chain sequence comprising SEQ ID NO:99 and a heavy chain sequence comprising SEQ ID NO:100; or
   (x) a light chain sequence comprising SEQ ID NO:101 and a heavy chain sequence comprising SEQ ID NO: 102 or 173.
4. An isolated antibody that binds to human RSPO3, wherein the antibody has one or more of the following characteristics:
   (a) binds human RSPO3 but not human RSPO1, human RSPO2, or human RSPO4;
   (b) binds to human RSPO3 with a Kd of less than 0.5 nM;
   (c) binds to cynomolgus RSPO3 with a Kd of less than 0.5 nM;
   (d) binds to murine RSPO3 with a Kd of less than 0.5 nM;
   (e) binds human RSPO3 with a Kd of less than 1.0 nM, binds cyno RSPO3 with a Kd of less than 1.0 nM, and binds murine RSPO3 with a Kd of less than 1.0 nM;
   (f) binds human RSPO3 with a Kd of less than 0.5 nM, binds cyno RSPO3 with a Kd of less than 0.5 nM, and binds murine RSPO3 with a Kd of less than 0.5 nM;
   (g) has a half-life of at least 6 days following a single 10 mg/kg intravenous administration in cynomolgus monkeys; and
   (h) reduces tumor growth in a patient derived mouse xenograft RSPO3 fusion model, such as a PTPRK-RSPO3 fusion model, such as a PTPRK(exon1)-RSPO3(exon2) fusion or a PTPRK(exon7)-RSPO3(exon2) fusion model.
5. The isolated antibody of 4, wherein the antibody inhibits the interaction of human RSPO3 with one or more of transmembrane E3 ubiquitinase, ZNRF3, RNF43, LGR4, LGR5, and LGR6.
6. The isolated antibody of 4 or 5, wherein the antibody does not bind to human RSPO2.
7. The isolated antibody of any one of 4 to 6, wherein the antibody binds to human RSPO3 with a Kd of less than 4 nM, such as less than 3.5 nM, such as 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, or less than 0.2 nM.
8. The isolated antibody of any one of 4 to 7, wherein the antibody binds to cynomolgus RSPO3 with a Kd of less than 4 nM, such as less than 3.5 nM, such as less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, or less than 0.2 nM.
9. The isolated antibody of any one of 4 to 7, wherein the antibody binds to murine RSPO3 with a Kd of less than 4 nM, such as less than 3.5 nM, such as less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, or less than 0.2 nM.
10. The isolated antibody of any one of 4 to 9, wherein the antibody binds human RSPO3 with a Kd of less than 1.0 nM, binds cyno RSPO3 with a Kd of less than 1.0 nM, and binds murine RSPO3 with a Kd of less than 1.0 nM.
11. The isolated antibody of 10, wherein the antibody binds human RSPO3 with a Kd of less than 1.0 nM, binds cyno RSPO3 with a Kd of less than 0.5 nM, and binds murine RSPO3 with a Kd of less than 1.0 nM.
12. The isolated antibody of 10, wherein the antibody binds human RSPO3 with a Kd of less than 0.5 nM, binds cyno RSPO3 with a Kd of less than 0.5 nM, and binds murine RSPO3 with a Kd of less than 0.5 nM.
13. The isolated antibody of 10, wherein the antibody binds human RSPO3 with a Kd of less than 0.5 nM, binds cyno RSPO3 with a Kd of less than 0.3 nM, and binds murine RSPO3 with a Kd of less than 0.5 nM.
14. The isolated antibody of any one of 4 to 13, wherein binding to RSPO3 is determined by surface plasmon resonance.
15. The isolated antibody of any one of 4 to 14, wherein the antibody has a half-life of at least 6 days, such as at least 8 days, at least 9 days, at least 10 days, at least 11 days, or at least 12 days following a single 10 mg/kg intravenous administration in cynomolgus monkeys.
16. The isolated antibody of any one of 4 to 6, wherein the antibody
   binds human RSPO3 with a Kd of less than 4 nM, binds cyno RSPO3 with a Kd of less than 2 nM, and binds murine RSPO3 with a Kd of less than 4 nM; and
   has a half-life of at least 6 days, such as at least 8 days, at least 9 days, at least 10 days, at least 11 days, or at least 12 days following a single 10 mg/kg intravenous administration in cynomolgus monkeys.
17. The antibody of any one of 4 to 8 or 15 to 16, wherein the antibody comprises:
   (a) VL comprising (i) HVR-L1 comprising the amino acid sequence of SEQ ID NO:5, (ii) HVR-L2 comprising the amino acid sequence of SEQ ID NO:6, and (iii) HVR-L3 comprising the amino acid sequence of SEQ ID NO:7; and a VH comprising (i) HVR-H1 comprising the amino acid sequence of SEQ ID NO:8, (ii) HVR-H2 comprising the amino acid sequence of SEQ ID NO:9, and (iii) HVR-H3 comprising the amino acid sequence of SEQ ID NO:10;
   (b) a VL comprising (i) HVR-L1 comprising the amino acid sequence of SEQ ID NO:11, (ii) HVR-L2 comprising the amino acid sequence of SEQ ID NO: 12, and (iii) HVR-L3 comprising the amino acid sequence of SEQ ID NO: 13; and a VH comprising (i) HVR-H1 comprising the amino acid sequence of SEQ ID NO: 14, (ii) HVR-H2 comprising the amino acid sequence of SEQ ID NO:15, and (iii) HVR-H3 comprising the amino acid sequence of SEQ ID NO: 16; or
   (c) a VL comprising (i) HVR-L1 comprising the amino acid sequence of SEQ ID NO:17, (ii) HVR-L2 comprising the amino acid sequence of SEQ ID NO: 18, and (iii) HVR-L3 comprising the amino acid sequence of SEQ ID NO: 19; and a VH comprising (i) HVR-H1 comprising the amino acid sequence of SEQ ID NO:20, (ii) HVR-H2 comprising the amino acid sequence of SEQ ID NO:21, and (iii) HVR-H3 comprising the amino acid sequence of SEQ ID NO:22.
18. The isolated antibody of 17, wherein the antibody comprises
   (a) a VL sequence comprising SEQ ID NO:23 and a VH sequence comprising SEQ ID NO:24;
   (b) a VL sequence comprising SEQ ID NO:25 and a VH sequence comprising SEQ ID NO:26;
   (c) a VL sequence comprising SEQ ID NO:27 and a VH sequence comprising SEQ ID NO:28;
   (d) a VL sequence comprising SEQ ID NO:29 and a VH sequence comprising SEQ ID NO:30;
   (e) a VL sequence comprising SEQ ID NO:31 and a VH sequence comprising SEQ ID NO:32;
   (f) a VL sequence comprising SEQ ID NO:33 and a VH sequence comprising SEQ ID NO:34;
   (g) a VL sequence comprising SEQ ID NO:35 and a VH sequence comprising SEQ ID NO:36;
   (h) a VL sequence comprising SEQ ID NO:37 and a VH sequence comprising SEQ ID NO:38;
   (i) a VL sequence comprising SEQ ID NO:39 and a VH sequence comprising SEQ ID NO:40;
   (j) a VL sequence comprising SEQ ID NO:41 and a VH sequence comprising SEQ ID NO:42;
   (k) a VL sequence comprising SEQ ID NO:43 and a VH sequence comprising SEQ ID NO:44;
   (l) a VL sequence comprising SEQ ID NO:45 and a VH sequence comprising SEQ ID NO:46;
   (m) a VL sequence comprising SEQ ID NO:47 and a VH sequence comprising SEQ ID NO:48;
   (n) a VL sequence comprising SEQ ID NO:49 and a VH sequence comprising SEQ ID NO:50;
   (o) a VL sequence comprising SEQ ID NO:51 and a VH sequence comprising SEQ ID NO:52;
   (p) a VL sequence comprising SEQ ID NO:53 and a VH sequence comprising SEQ ID NO:54;
   (q) a VL sequence comprising SEQ ID NO:55 and a VH sequence comprising SEQ ID NO:56;
   (r) a VL sequence comprising SEQ ID NO:57 and a VH sequence comprising SEQ ID NO:58;
   (w) a VL sequence comprising SEQ ID NO:59 and a VH sequence comprising SEQ ID NO:60; or
   (x) a VL sequence comprising SEQ ID NO:61 and a VH sequence comprising SEQ ID NO:62.
19. The isolated antibody of 17, wherein the antibody comprises
   (a) a light chain sequence comprising SEQ ID NO:63 and a heavy chain sequence comprising SEQ ID NO:64;
   (b) a light chain sequence comprising SEQ ID NO:65 and a heavy chain sequence comprising SEQ ID NO:66;
   (c) a light chain sequence comprising SEQ ID NO:67 and a heavy chain sequence comprising SEQ ID NO:68;
   (d) a light chain sequence comprising SEQ ID NO:69 and a heavy chain sequence comprising SEQ ID NO:70;
   (e) a light chain sequence comprising SEQ ID NO:71 and a heavy chain sequence comprising SEQ ID NO:72;
   (f) a light chain sequence comprising SEQ ID NO:73 and a heavy chain sequence comprising SEQ ID NO:74;
   (g) a light chain sequence comprising SEQ ID NO:75 and a heavy chain sequence comprising SEQ ID NO:76 or 171;
   (h) a light chain sequence comprising SEQ ID NO:77 and a heavy chain sequence comprising SEQ ID NO:78;
   (i) a light chain sequence comprising SEQ ID NO:79 and a heavy chain sequence comprising SEQ ID NO:80;
   (j) a light chain sequence comprising SEQ ID NO:81 and a heavy chain sequence comprising SEQ ID NO:82;
   (k) a light chain sequence comprising SEQ ID NO:83 and a heavy chain sequence comprising SEQ ID NO:84;
   (l) a light chain sequence comprising SEQ ID NO:85 and a heavy chain sequence comprising SEQ ID NO:86;
   (m) a light chain sequence comprising SEQ ID NO:87 and a heavy chain sequence comprising SEQ ID NO:88 or 172;
   (n) a light chain sequence comprising SEQ ID NO:89 and a heavy chain sequence comprising SEQ ID NO:90;
   (o) a light chain sequence comprising SEQ ID NO:91 and a heavy chain sequence comprising SEQ ID NO:92;
   (p) a light chain sequence comprising SEQ ID NO:93 and a heavy chain sequence comprising SEQ ID NO:94;
   (q) a light chain sequence comprising SEQ ID NO:95 and a heavy chain sequence comprising SEQ ID NO:96;
   (r) a light chain sequence comprising SEQ ID NO:97 and a heavy chain sequence comprising SEQ ID NO:98;
   (w) a light chain sequence comprising SEQ ID NO:99 and a heavy chain sequence comprising SEQ ID NO:100; or
   (x) a light chain sequence comprising SEQ ID NO:101 and a heavy chain sequence comprising SEQ ID NO: 102 or 173.
20. The isolated antibody of any one of 4 to 19, wherein the antibody inhibits the interaction of human RSPO3 with human ZNRF3 and/or human RNF43.
21. An isolated antibody that binds the same epitope on RSPO3 as the antibody according to any one of 1-3.
22. An isolated antibody that binds an epitope on RSPO3 that overlaps with the epitope on RSPO3 bound by the antibody according to any one of 1-3.
23. The isolated antibody of any one of 1-22, wherein the antibody inhibits RSPO3 mediated wnt signaling and/or induces differentiation of tumor cells in a mouse xenograft model comprising an RSPO3 fusion, such as a PTPRK/RSPO3 fusion, such as a PTPRK(e1)/RSPO3(e2) fusion or a PTPRK (e7)/RSPO3(e2) fusion, such as a fusion chosen from SEQ ID NOs: 105 and 107.
24. The antibody of any one of 1-23, which is a monoclonal antibody.
25. The antibody of any one of 1-24, which is a human, humanized, or chimeric antibody or a bi-specific or multi-specific antibody.
26. The antibody of any one of 1-25, which is a full length IgG1 or IgG2a antibody.
27. The antibody of any one of 1-26, wherein the antibody is a glycosylation variant or Fc variant antibody, such as a fucose deficient antibody, such as an antibody with an amino acid modification at EU positions 294 to 300, such as a modification at EU position 297.
28. The antibody of any one of 1-26, which is an antigen binding fragment, such as comprising a Fab, F(ab)₂, Fv, or scFv fragment.
29. An isolated nucleic acid or set of isolated nucleic acids encoding the heavy and light chain variable regions of the antibody of any one of 1-28.
30. A host cell comprising the nucleic acid or set of nucleic acids of 29.
31. A method of producing an antibody comprising culturing the host cell of 30 so that the antibody is produced.
32. The method of 31, further comprising recovering the antibody from the host cell.
33. An immunoconjugate comprising the antibody of any one of 1-28 and a cytotoxic agent.
34. A pharmaceutical formulation comprising the antibody of any one of 1-28 and a pharmaceutically acceptable carrier.
35. The pharmaceutical formulation of 34, further comprising an additional therapeutic agent.
36. The antibody of any one of 1-28 or the pharmaceutical formulation of 34 or 35 for use as a medicament.
37. The antibody of any one of 1-28 or the pharmaceutical formulation of 34 or 35 for use in treating cancer.
38. The antibody or pharmaceutical formulation of 37, wherein the cancer is gastrointestinal cancer, stomach cancer, colon cancer, colorectal cancer, lung cancer, or rectal cancer.
39. The antibody of any one of 1-28 or the pharmaceutical formulation of 34 or 35 for use in inhibiting wnt signaling, inhibiting angiogenesis and/or vasculogenesis, and/or inhibiting cell proliferation.
40. Use of the antibody of any one of 1-28 in the manufacture of a medicament for treatment of cancer.
41. Use of 40, wherein the cancer is gastrointestinal cancer, stomach cancer, colon cancer, colorectal cancer, lung cancer, or rectal cancer.
42. Use of the antibody of any one of 1-28 in the manufacture of a medicament for inhibiting wnt signaling, inhibiting angiogenesis and/or vasculogenesis, and/or inhibiting cell proliferation.
43. A method of treating an individual having cancer comprising administering to the individual an effective amount of the antibody of any one of 1-28 or the pharmaceutical formulation of 34 or 35.
44. The method of 43, wherein the cancer is gastrointestinal cancer, stomach cancer, colon cancer, colorectal cancer, lung cancer, or rectal cancer.
45. A method of inhibiting wnt signaling, inhibiting angiogenesis and/or vasculogenesis, and/or inhibiting cell proliferation in an individual comprising administering to the individual an effective amount of the antibody of any one of 1-28 or the pharmaceutical formulation of 34 or 35 to inhibit wnt signaling, inhibit angiogenesis and/or vasculogenesis, and/or inhibit cell proliferation.
46. The antibody, pharmaceutical formulation, use, or method of any one of 36 to 45, further comprising administering an additional therapeutic agent to the individual.
47. The antibody, pharmaceutical formulation, use or method of any one of 36-38, 40-41, 43-44, or 46, wherein the cancer is characterized by increased expression of RSPO3 compared to a reference.
48. The antibody, pharmaceutical formulation, use or method of any one of 36-38, 40-41, 43-44, or 46-47, wherein the cancer is characterized by presence of an RSPO3 fusion.
49. The antibody, pharmaceutical formulation, use or method of 48, wherein the RSPO3 fusion is a PTPRK/RSPO3 fusion, such as a PTPRK(e1)/RSPO3(e2) fusion or a PTPRK (e7)/RSPO3(e2) fusion, such as a fusion chosen from SEQ ID NOs: 105 and 107.

## Claims

1. An isolated antibody that binds to human R-spondin 3 (RSPO3), wherein the antibody comprises:
a light chain variable region (VL) comprising (i) a first light chain hypervariable region (HVR-L1) comprising the amino acid sequence of SEQ ID NO:5, (ii) HVR-L2 comprising the amino acid sequence of SEQ ID NO:6, and (iii) HVR-L3 comprising the amino acid sequence of SEQ ID NO:7; and a heavy chain variable region (VH) comprising (i) a first heavy chain hypervariable region (HVR-H1) comprising the amino acid sequence of SEQ ID NO:8, (ii) HVR-H2 comprising the amino acid sequence of SEQ ID NO:9, and (iii) HVR-H3 comprising the amino acid sequence of SEQ ID NO:10, wherein the antibody has a half-life of at least 6 days following a single 10 mg/kg intravenous administration in cynomolgus monkeys.

2. The isolated antibody of claim 1, wherein the antibody comprises
(a) a VL sequence comprising SEQ ID NO:23 and a VH sequence comprising SEQ ID NO:24;
(b) a VL sequence comprising SEQ ID NO:25 and a VH sequence comprising SEQ ID NO:26;
(c) a VL sequence comprising SEQ ID NO:27 and a VH sequence comprising SEQ ID NO:28;
(d) a VL sequence comprising SEQ ID NO:29 and a VH sequence comprising SEQ ID NO:30;
(e) a VL sequence comprising SEQ ID NO:31 and a VH sequence comprising SEQ ID NO:32;
(f) a VL sequence comprising SEQ ID NO:33 and a VH sequence comprising SEQ ID NO:34; or
(g) a VL sequence comprising SEQ ID NO:35 and a VH sequence comprising SEQ ID NO:36.

3. The isolated antibody of claim 1 or 2, wherein the antibody comprises
(a) a light chain sequence comprising SEQ ID NO:63 and a heavy chain sequence comprising SEQ ID NO:64;
(b) a light chain sequence comprising SEQ ID NO:65 and a heavy chain sequence comprising SEQ ID NO:66;
(c) a light chain sequence comprising SEQ ID NO:67 and a heavy chain sequence comprising SEQ ID NO:68;
(d) a light chain sequence comprising SEQ ID NO:69 and a heavy chain sequence comprising SEQ ID NO:70;
(e) a light chain sequence comprising SEQ ID NO:71 and a heavy chain sequence comprising SEQ ID NO:72;
(f) a light chain sequence comprising SEQ ID NO:73 and a heavy chain sequence comprising SEQ ID NO:74; or
(g) a light chain sequence comprising SEQ ID NO:75 and a heavy chain sequence comprising SEQ ID NO:76 or 171.

4. The isolated antibody of any one of claims 1-3, wherein the antibody has the following characteristics:
(a) binds human RSPO3 but not human RSPO1, human RSPO2, or human RSPO4;
(b) binds to human RSPO3 with a Kd of less than 0.5 nM;
(c) binds to cynomolgus RSPO3 with a Kd of less than 0.5 nM;
(d) binds to murine RSPO3 with a Kd of less than 0.5 nM;
(e) binds human RSPO3 with a Kd of less than 1.0 nM, binds cyno RSPO3 with a Kd of less than 1.0 nM, and binds murine RSPO3 with a Kd of less than 1.0 nM;
(f) binds human RSPO3 with a Kd of less than 0.5 nM, binds cyno RSPO3 with a Kd of less than 0.5 nM, and binds murine RSPO3 with a Kd of less than 0.5 nM; and
(g) reduces tumor growth in a patient derived mouse xenograft RSPO3 fusion model, such as a PTPRK-RSPO3 fusion model, such as a PTPRK(exon1)-RSPO3(exon2) fusion or a PTPRK(exon7)-RSPO3(exon2) fusion model.

5. The antibody of any one of claims 1-4, which is a monoclonal antibody.

6. The antibody of any one of claims 1-5, which is a human, humanized, or chimeric antibody or a bi-specific or multi-specific antibody.

7. The antibody of any one of claims 1-6, which is a full length IgG1 or IgG2a antibody.

8. The antibody of any one of claims 1-7, wherein the antibody is a glycosylation variant or Fc variant antibody, such as a fucose deficient antibody, such as an antibody with an amino acid modification at EU positions 294 to 300, such as a modification at EU position 297.

9. The antibody of any one of claims 1-8, which is an antigen binding fragment, such as comprising a Fab, F(ab)₂, Fv, or scFv fragment.

10. An isolated nucleic acid or set of isolated nucleic acids encoding the heavy and light chain variable regions of the antibody of any one of claims 1-9.

11. A host cell comprising the nucleic acid or set of nucleic acids of claim 10.

12. A method of producing an antibody comprising culturing the host cell of claim 11 so that the antibody is produced.

13. An immunoconjugate comprising the antibody of any one of claims 1-9 and a cytotoxic agent.

14. A pharmaceutical formulation comprising the antibody of any one of claims 1-9 and a pharmaceutically acceptable carrier.

15. The antibody of any one of claims 1-9 or the pharmaceutical formulation of claim 14 for use as a medicament.


SEQUENCE LISTING

<110>  GENENTECH, INC.
       F. HOFFMANN-LA ROCHE AG

<120>  ANTI-RSPO3 ANTIBODIES AND METHODS OF USE

<130>  01146-0055-00PCT

<150>  US 62/322,717
<151>  2016-04-14

<160>  173

<170>  PatentIn version 3.5

<210>  1
<211>  243
<212>  PRT
<213>  Homo sapiens

<400>  1

Met Gln Phe Arg Leu Phe Ser Phe Ala Leu Ile Ile Leu Asn Cys Met
1               5                   10                  15

Asp Tyr Ser His Cys Gln Gly Asn Arg Trp Arg Arg Ser Lys Arg Ala
            20                  25                  30

Ser Tyr Val Ser Asn Pro Ile Cys Lys Gly Cys Leu Ser Cys Ser Lys
        35                  40                  45

Asp Asn Gly Cys Ser Arg Cys Gln Gln Lys Leu Phe Phe Phe Leu Arg
    50                  55                  60

Arg Glu Gly Met Arg Gln Tyr Gly Glu Cys Leu His Ser Cys Pro Ser
65                  70                  75                  80

Gly Tyr Tyr Gly His Arg Ala Pro Asp Met Asn Arg Cys Ala Arg Cys
                85                  90                  95

Arg Ile Glu Asn Cys Asp Ser Cys Phe Ser Lys Asp Phe Cys Thr Lys
            100                 105                 110

Cys Lys Val Gly Phe Tyr Leu His Arg Gly Arg Cys Phe Asp Glu Cys
        115                 120                 125

Pro Asp Gly Phe Ala Pro Leu Glu Glu Thr Met Glu Cys Val Glu Gly
    130                 135                 140

Cys Glu Val Gly His Trp Ser Glu Trp Gly Thr Cys Ser Arg Asn Asn
145                 150                 155                 160

Arg Thr Cys Gly Phe Lys Trp Gly Leu Glu Thr Arg Thr Arg Gln Ile
                165                 170                 175

Val Lys Lys Pro Val Lys Asp Thr Ile Leu Cys Pro Thr Ile Ala Glu
            180                 185                 190

Ser Arg Arg Cys Lys Met Thr Met Arg His Cys Pro Gly Gly Lys Arg
        195                 200                 205

Thr Pro Lys Ala Lys Glu Lys Arg Asn Lys Lys Lys Lys Arg Lys Leu
    210                 215                 220

Ile Glu Arg Ala Gln Glu Gln His Ser Val Phe Leu Ala Thr Asp Arg
225                 230                 235                 240

Ala Asn Gln

<210>  2
<211>  272
<212>  PRT
<213>  Homo sapiens

<400>  2

Met His Leu Arg Leu Ile Ser Trp Leu Phe Ile Ile Leu Asn Phe Met
1               5                   10                  15

Glu Tyr Ile Gly Ser Gln Asn Ala Ser Arg Gly Arg Arg Gln Arg Arg
            20                  25                  30

Met His Pro Asn Val Ser Gln Gly Cys Gln Gly Gly Cys Ala Thr Cys
        35                  40                  45

Ser Asp Tyr Asn Gly Cys Leu Ser Cys Lys Pro Arg Leu Phe Phe Ala
    50                  55                  60

Leu Glu Arg Ile Gly Met Lys Gln Ile Gly Val Cys Leu Ser Ser Cys
65                  70                  75                  80

Pro Ser Gly Tyr Tyr Gly Thr Arg Tyr Pro Asp Ile Asn Lys Cys Thr
                85                  90                  95

Lys Cys Lys Ala Asp Cys Asp Thr Cys Phe Asn Lys Asn Phe Cys Thr
            100                 105                 110

Lys Cys Lys Ser Gly Phe Tyr Leu His Leu Gly Lys Cys Leu Asp Asn
        115                 120                 125

Cys Pro Glu Gly Leu Glu Ala Asn Asn His Thr Met Glu Cys Val Ser
    130                 135                 140

Ile Val His Cys Glu Val Ser Glu Trp Asn Pro Trp Ser Pro Cys Thr
145                 150                 155                 160

Lys Lys Gly Lys Thr Cys Gly Phe Lys Arg Gly Thr Glu Thr Arg Val
                165                 170                 175

Arg Glu Ile Ile Gln His Pro Ser Ala Lys Gly Asn Leu Cys Pro Pro
            180                 185                 190

Thr Asn Glu Thr Arg Lys Cys Thr Val Gln Arg Lys Lys Cys Gln Lys
        195                 200                 205

Gly Glu Arg Gly Lys Lys Gly Arg Glu Arg Lys Arg Lys Lys Pro Asn
    210                 215                 220

Lys Gly Glu Ser Lys Glu Ala Ile Pro Asp Ser Lys Ser Leu Glu Ser
225                 230                 235                 240

Ser Lys Glu Ile Pro Glu Gln Arg Glu Asn Lys Gln Gln Gln Lys Lys
                245                 250                 255

Arg Lys Val Gln Asp Lys Gln Lys Ser Val Ser Val Ser Thr Val His
            260                 265                 270

<210>  3
<211>  263
<212>  PRT
<213>  Homo sapiens

<400>  3

Met Arg Leu Gly Leu Cys Val Val Ala Leu Val Leu Ser Trp Thr His
1               5                   10                  15

Leu Thr Ile Ser Ser Arg Gly Ile Lys Gly Lys Arg Gln Arg Arg Ile
            20                  25                  30

Ser Ala Glu Gly Ser Gln Ala Cys Ala Lys Gly Cys Glu Leu Cys Ser
        35                  40                  45

Glu Val Asn Gly Cys Leu Lys Cys Ser Pro Lys Leu Phe Ile Leu Leu
    50                  55                  60

Glu Arg Asn Asp Ile Arg Gln Val Gly Val Cys Leu Pro Ser Cys Pro
65                  70                  75                  80

Pro Gly Tyr Phe Asp Ala Arg Asn Pro Asp Met Asn Lys Cys Ile Lys
                85                  90                  95

Cys Lys Ile Glu His Cys Glu Ala Cys Phe Ser His Asn Phe Cys Thr
            100                 105                 110

Lys Cys Lys Glu Gly Leu Tyr Leu His Lys Gly Arg Cys Tyr Pro Ala
        115                 120                 125

Cys Pro Glu Gly Ser Ser Ala Ala Asn Gly Thr Met Glu Cys Ser Ser
    130                 135                 140

Pro Ala Gln Cys Glu Met Ser Glu Trp Ser Pro Trp Gly Pro Cys Ser
145                 150                 155                 160

Lys Lys Gln Gln Leu Cys Gly Phe Arg Arg Gly Ser Glu Glu Arg Thr
                165                 170                 175

Arg Arg Val Leu His Ala Pro Val Gly Asp His Ala Ala Cys Ser Asp
            180                 185                 190

Thr Lys Glu Thr Arg Arg Cys Thr Val Arg Arg Val Pro Cys Pro Glu
        195                 200                 205

Gly Gln Lys Arg Arg Lys Gly Gly Gln Gly Arg Arg Glu Asn Ala Asn
    210                 215                 220

Arg Asn Leu Ala Arg Lys Glu Ser Lys Glu Ala Gly Ala Gly Ser Arg
225                 230                 235                 240

Arg Arg Lys Gly Gln Gln Gln Gln Gln Gln Gln Gly Thr Val Gly Pro
                245                 250                 255

Leu Thr Ser Ala Gly Pro Ala
            260

<210>  4
<211>  234
<212>  PRT
<213>  Homo sapiens

<400>  4

Met Arg Ala Pro Leu Cys Leu Leu Leu Leu Val Ala His Ala Val Asp
1               5                   10                  15

Met Leu Ala Leu Asn Arg Arg Lys Lys Gln Val Gly Thr Gly Leu Gly
            20                  25                  30

Gly Asn Cys Thr Gly Cys Ile Ile Cys Ser Glu Glu Asn Gly Cys Ser
        35                  40                  45

Thr Cys Gln Gln Arg Leu Phe Leu Phe Ile Arg Arg Glu Gly Ile Arg
    50                  55                  60

Gln Tyr Gly Lys Cys Leu His Asp Cys Pro Pro Gly Tyr Phe Gly Ile
65                  70                  75                  80

Arg Gly Gln Glu Val Asn Arg Cys Lys Lys Cys Gly Ala Thr Cys Glu
                85                  90                  95

Ser Cys Phe Ser Gln Asp Phe Cys Ile Arg Cys Lys Arg Gln Phe Tyr
            100                 105                 110

Leu Tyr Lys Gly Lys Cys Leu Pro Thr Cys Pro Pro Gly Thr Leu Ala
        115                 120                 125

His Gln Asn Thr Arg Glu Cys Gln Gly Glu Cys Glu Leu Gly Pro Trp
    130                 135                 140

Gly Gly Trp Ser Pro Cys Thr His Asn Gly Lys Thr Cys Gly Ser Ala
145                 150                 155                 160

Trp Gly Leu Glu Ser Arg Val Arg Glu Ala Gly Arg Ala Gly His Glu
                165                 170                 175

Glu Ala Ala Thr Cys Gln Val Leu Ser Glu Ser Arg Lys Cys Pro Ile
            180                 185                 190

Gln Arg Pro Cys Pro Gly Glu Arg Ser Pro Gly Gln Lys Lys Gly Arg
        195                 200                 205

Lys Asp Arg Arg Pro Arg Lys Asp Arg Lys Leu Asp Arg Arg Leu Asp
    210                 215                 220

Val Arg Pro Arg Gln Pro Gly Leu Gln Pro
225                 230

<210>  5
<211>  11
<212>  PRT
<213>  Artificial Sequence

<220>
<223>  Antibody Sequence 4A6-HVR L1

<400>  5

Leu Ala Ser Glu Asp Ile Ser Asn Asp Leu Val

1                   5                       10

<210>  6
<211>  7
<212>  PRT
<213>  Artificial Sequence

<220>
<223>  Antibody Sequence 4A6-HVR L2

<400>  6

Ala Ala Ser Arg Leu Gln Asp
1               5

<210>  7
<211>  9
<212>  PRT
<213>  Artificial Sequence

<220>
<223>  Antibody Sequence 4A6-HVR L3

<400>  7

Gln Gln Ser Tyr Lys Tyr Leu Pro Thr
1               5

<210>  8
<211>  5
<212>  PRT
<213>  Artificial Sequence

<220>
<223>  Antibody Sequence 4A6-HVR H1

<400>  8

Asp Tyr Asp Met Ala
1               5

<210>  9
<211>  17
<212>  PRT
<213>  Artificial Sequence

<220>
<223>  Antibody Sequence 4A6-HVR H2

<400>  9

Thr Ile Ile Tyr Asp Gly Ser Arg Thr Tyr Tyr Arg Asp Ser Val Lys
1               5                   10                  15

Gly

<210>  10
<211>  7
<212>  PRT
<213>  Artificial Sequence

<220>
<223>  Antibody Sequence 4A6-HVR H3

<400>  10

His Asp Arg Ser Phe Asp Tyr
1               5


<210>  11
<211>  11
<212>  PRT
<213>  Artificial Sequence

<220>
<223>  Antibody Sequence 11C10-HVR L1

<400>  11

Arg Ala Ser Glu Asp Ile Tyr Ser Asp Leu Ala
1               5                   10


<210>  12
<211>  7
<212>  PRT
<213>  Artificial Sequence

<220>
<223>  Antibody Sequence 11C10-HVR L2

<400>  12

Asp Val Asn Ser Leu Ile His
1               5


<210>  13
<211>  9
<212>  PRT
<213>  Artificial Sequence

<220>
<223>  Antibody Sequence 11C10-HVR L3

<400>  13

Gln Gln Tyr Asp Asn Tyr Pro Asn Thr
1               5


<210>  14
<211>  5
<212>  PRT
<213>  Artificial Sequence

<220>
<223>  Antibody Sequence 11C10-HVR H1

<400>  14

Asp Tyr Asp Met Ala
1               5



<210>  15
<211>  17
<212>  PRT
<213>  Artificial Sequence

<220>
<223>  Antibody Sequence 11C10-HVR H2

<400>  15

Thr Ile Ile Tyr Asp Gly Ser Arg Thr Tyr Tyr Arg Asp Ser Val Lys
1               5                   10                  15


Gly




<210>  16
<211>  7
<212>  PRT
<213>  Artificial Sequence

<220>
<223>  Antibody Sequence 11C10-HVR H3

<400>  16

His Asp Lys Thr Phe Asp Tyr
1               5



<210>  17
<211>  11
<212>  PRT
<213>  Artificial Sequence

<220>
<223>  Antibody Sequence 15F3-HVR L1

<400>  17

Leu Val Ser Glu Asp Ile Ser Asn Asp Phe Val
1               5                   10



<210>  18
<211>  7
<212>  PRT
<213>  Artificial Sequence

<220>
<223>  Antibody Sequence 15F3-HVR L2

<400>  18

Ala Ala Ser Arg Leu Gln Asp
1               5

<210>  19
<211>  9
<212>  PRT
<213>  Artificial Sequence

<220>
<223>  Antibody Sequence 15F3-HVR L3

<400>  19

Gln Gln Ser Tyr Lys Tyr Pro Pro Thr
1               5

<210>  20
<211>  5
<212>  PRT
<213>  Artificial Sequence

<220>
<223>  Antibody Sequence 15F3-HVR H1

<400>  20

Asp Tyr Asp Met Ala
1               5

<210>  21
<211>  17
<212>  PRT
<213>  Artificial Sequence

<220>
<223>  Antibody Sequence 15F3-HVR H2

<400>  21

Thr Ile Ile Tyr Asp Gly Ser Arg Ala Tyr Phe Gly Asp Ser Val Arg
1               5                   10                  15

Gly

<210>  22
<211>  7
<212>  PRT
<213>  Artificial Sequence

<220>
<223>  Antibody Sequence 15F3-HVR H3

<400>  22

His Asp Arg Ser Phe Asp Tyr
1               5

<210>  23
<211>  107
<212>  PRT
<213>  Artificial Sequence

<220>
<223>  Antibody Sequence 4A6 VL

<400>  23

Asp Ile Gln Met Thr Gln Ser Pro Ala Ser Leu Ser Ala Ser Leu Gly
1               5                   10                  15

Glu Thr Val Ser Ile Glu Cys Leu Ala Ser Glu Asp Ile Ser Asn Asp
            20                  25                  30

Leu Val Trp Tyr Gln Gln Lys Ser Gly Lys Ser Pro Gln Leu Leu Ile
        35                  40                  45

Tyr Ala Ala Ser Arg Leu Gln Asp Gly Val Pro Ser Arg Phe Ser Gly
    50                  55                  60

Ser Gly Phe Gly Thr Arg Phe Ser Leu Lys Ile Ser Gly Met Gln Pro
65                  70                  75                  80

Glu Asp Glu Ala Asp Tyr Phe Cys Gln Gln Ser Tyr Lys Tyr Leu Pro
                85                  90                  95

Thr Phe Gly Ala Gly Thr Lys Leu Gly Leu Lys
            100                 105


<210>  24
<211>  116
<212>  PRT
<213>  Artificial Sequence

<220>
<223>  Antibody Sequence 4A6 VH

<400>  24

Glu Val Gln Leu Val Glu Ser Gly Gly Gly Ser Val Gln Pro Gly Arg
1               5                   10                  15

Ser Leu Lys Val Ser Cys Ala Ala Ser Gly Phe Thr Phe Ser Asp Tyr
            20                  25                  30

Asp Met Ala Trp Val Arg Gln Ala Pro Lys Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Leu Ser Arg Asp Asn Thr Lys Ser Thr Leu Cys
65                  70                  75                  80

Leu Gln Met Asp Ser Leu Arg Ser Glu Asp Thr Ala Thr Tyr Tyr Cys
                85                  90                  95

Ala Ala His Asp Arg Ser Phe Asp Tyr Trp Gly Gln Gly Ile Met Val
            100                 105                 110

Thr Val Ser Ser
        115

<210>  25
<211>  107
<212>  PRT
<213>  Artificial Sequence

<220>
<223>  Antibody Sequence hu4A6.L1H1 VL

<400>  25

Asp Ile Gln Met Thr Gln Ser Pro Ser Ser Leu Ser Ala Ser Val Gly
1               5                   10                  15

Asp Arg Val Thr Ile Thr Cys Leu Ala Ser Glu Asp Ile Ser Asn Asp
            20                  25                  30

Leu Val Trp Tyr Gln Gln Lys Pro Gly Lys Ser Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Lys Tyr Leu Pro
                85                  90                  95

Thr Phe Gly Gln Gly Thr Lys Leu Glu Ile Lys
            100                 105

<210>  26
<211>  116
<212>  PRT
<213>  Artificial Sequence

<220>
<223>  Antibody Sequence hu4A6.L1H1 VH

<400>  26

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Leu Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser
        115

<210>  27
<211>  107
<212>  PRT
<213>  Artificial Sequence

<220>
<223>  Antibody Sequence hu4A6.L1H2 VL

<400>  27

Asp Ile Gln Met Thr Gln Ser Pro Ser Ser Leu Ser Ala Ser Val Gly
1               5                   10                  15

Asp Arg Val Thr Ile Thr Cys Leu Ala Ser Glu Asp Ile Ser Asn Asp
            20                  25                  30

Leu Val Trp Tyr Gln Gln Lys Pro Gly Lys Ser Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Lys Tyr Leu Pro
                85                  90                  95

Thr Phe Gly Gln Gly Thr Lys Leu Glu Ile Lys
            100                 105

<210>  28
<211>  116
<212>  PRT
<213>  Artificial Sequence

<220>
<223>  Antibody Sequence hu4A6.L1H2 VH

<400>  28

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser
        115

<210>  29
<211>  107
<212>  PRT
<213>  Artificial Sequence

<220>
<223>  Antibody Sequence hu4A6.L2H1 VL

<400>  29

Asp Ile Gln Met Thr Gln Ser Pro Ser Ser Leu Ser Ala Ser Val Gly

1               5                   10                  15

Asp Arg Val Thr Ile Thr Cys Leu Ala Ser Glu Asp Ile Ser Asn Asp
            20                  25                  30

Leu Val Trp Tyr Gln Gln Lys Pro Gly Lys Ala Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Lys Tyr Leu Pro
                85                  90                  95

Thr Phe Gly Gln Gly Thr Lys Leu Glu Ile Lys
            100                 105

<210>  30
<211>  116
<212>  PRT
<213>  Artificial Sequence

<220>
<223>  Antibody Sequence hu4A6.L2H1 VH

<400>  30

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Leu Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser
        115

<210>  31
<211>  107
<212>  PRT
<213>  Artificial Sequence

<220>
<223>  Antibody Sequence hu4A6.L3H1 VL

<400>  31

Asp Ile Gln Met Thr Gln Ser Pro Ser Ser Leu Ser Ala Ser Val Gly
1               5                   10                  15

Asp Arg Val Thr Ile Thr Cys Leu Ala Ser Glu Asp Ile Ser Asn Asp
            20                  25                  30

Leu Val Trp Tyr Gln Gln Lys Pro Gly Lys Ser Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Lys Tyr Leu Pro
                85                  90                  95

Thr Phe Gly Gln Gly Thr Lys Leu Glu Ile Lys
            100                 105

<210>  32
<211>  116
<212>  PRT
<213>  Artificial Sequence

<220>
<223>  Antibody Sequence hu4A6.L3H1 VH

<400>  32

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val

35                  40                  45

Ala Thr Ile Ile Tyr Asp Gly Ser Arg Thr Tyr Tyr Arg Asp Ser Val
    50                  55                  60

Lys Gly Arg Phe Thr Leu Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser
        115

<210>  33
<211>  107
<212>  PRT
<213>  Artificial Sequence

<220>
<223>  Antibody Sequence hu4A6.L4H1 VL

<400>  33

Asp Ile Gln Met Thr Gln Ser Pro Ser Ser Leu Ser Ala Ser Val Gly
1               5                   10                  15

Asp Arg Val Thr Ile Thr Cys Leu Ala Ser Glu Asp Ile Ser Asn Asp
            20                  25                  30

Leu Val Trp Tyr Gln Gln Lys Pro Gly Lys Ala Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Phe Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Lys Tyr Leu Pro
                85                  90                  95

Thr Phe Gly Gln Gly Thr Lys Leu Glu Ile Lys
            100                 105

<210>  34
<211>  116

<212>  PRT
<213>  Artificial Sequence

<220>
<223>  Antibody Sequence hu4A6.L4H1 VH

<400>  34

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Leu Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser
        115


<210>  35
<211>  107
<212>  PRT
<213>  Artificial Sequence

<220>
<223>  Antibody Sequence hu4A6.L4H2 VL

<400>  35

Asp Ile Gln Met Thr Gln Ser Pro Ser Ser Leu Ser Ala Ser Val Gly
1               5                   10                  15

Asp Arg Val Thr Ile Thr Cys Leu Ala Ser Glu Asp Ile Ser Asn Asp
            20                  25                  30

Leu Val Trp Tyr Gln Gln Lys Pro Gly Lys Ala Pro Lys Leu Leu Ile
        35                  40                  45

Tyr Ala Ala Ser Arg Leu Gln Asp Gly Val Pro Ser Arg Phe Ser Gly

50                  55                  60

Ser Gly Phe Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Lys Tyr Leu Pro
                85                  90                  95

Thr Phe Gly Gln Gly Thr Lys Leu Glu Ile Lys
            100                 105

<210>  36
<211>  116
<212>  PRT
<213>  Artificial Sequence

<220>
<223>  Antibody Sequence hu4A6.L4H2 VH

<400>  36

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser
        115

<210>  37
<211>  107
<212>  PRT
<213>  Artificial Sequence

<220>

<223>  Antibody Sequence 11C10 VL

<400>  37

Asp Ile Gln Met Thr Gln Ser Pro Ala Ser Leu Ser Ala Ser Leu Gly
1               5                   10                  15

Glu Thr Val Thr Ile Glu Cys Arg Ala Ser Glu Asp Ile Tyr Ser Asp
            20                  25                  30

Leu Ala Trp Tyr Gln Gln Lys Pro Gly Asn Ser Pro Gln Leu Leu Ile
        35                  40                  45

Tyr Asp Val Asn Ser Leu Ile His Gly Val Pro Ser Arg Phe Ser Gly
    50                  55                  60

Ser Gly Ser Gly Thr Gln Phe Ser Leu Lys Ile Asn Asn Leu Gln Ser
65                  70                  75                  80

Glu Asp Val Ala Ser Tyr Phe Cys Gln Gln Tyr Asp Asn Tyr Pro Asn
                85                  90                  95

Thr Phe Gly Ala Gly Thr Lys Leu Glu Leu Lys
            100                 105

<210>  38
<211>  116
<212>  PRT
<213>  Artificial Sequence

<220>
<223>  Antibody Sequence 11C10 VH

<400>  38

Glu Val Gln Leu Val Glu Ser Gly Gly Gly Leu Val Gln Pro Gly Arg
1               5                   10                  15

Ser Leu Lys Leu Ser Cys Ala Ala Ser Gly Phe Thr Phe Ser Asp Tyr
            20                  25                  30

Asp Met Ala Trp Val Arg Gln Ala Pro Lys Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Ala Asn Ala Lys Ser Thr Leu Tyr
65                  70                  75                  80

Leu Gln Met Asp Ser Leu Arg Ser Glu Asp Thr Ala Thr Tyr Tyr Cys

85                  90                  95

Ala Thr His Asp Lys Thr Phe Asp Tyr Trp Gly Gln Gly Val Met Val
            100                 105                 110

Thr Val Ser Ser
        115

<210>  39
<211>  107
<212>  PRT
<213>  Artificial Sequence

<220>
<223>  Antibody Sequence hu11C10.L1H1 VL

<400>  39

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asp Ile Tyr Ser Asp
            20                  25                  30

Leu Ala Trp Tyr Gln Gln Lys Pro Gly Lys Ser Pro Lys Leu Leu Ile
        35                  40                  45

Tyr Asp Val Asn Ser Leu Ile His Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Asp Asn Tyr Pro Asn
                85                  90                  95

Thr Phe Gly Gln Gly Thr Lys Leu Glu Ile Lys
            100                 105

<210>  40
<211>  116
<212>  PRT
<213>  Artificial Sequence

<220>
<223>  Antibody Sequence hu11C10.L1H1 VH

<400>  40

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr His Asp Lys Thr Phe Asp Tyr Trp Gly Gln Gly Thr Met Val
            100                 105                 110

Thr Val Ser Ser
        115

<210> 41
<211> 107
<212> PRT
<213> Artificial Sequence

<220>
<223> Antibody Sequence hu11C10.L2H1 VL

<400> 41

Asp Ile Gln Leu Thr Gln Ser Pro Ser Ser Leu Ser Ala Ser Val Gly
1               5                   10                  15

Asp Arg Val Thr Ile Thr Cys Arg Ala Ser Glu Asp Ile Tyr Ser Asp
            20                  25                  30

Leu Ala Trp Tyr Gln Gln Lys Pro Gly Lys Ser Pro Lys Leu Leu Ile
        35                  40                  45

Tyr Asp Val Asn Ser Leu Ile His Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Asp Asn Tyr Pro Asn
                85                  90                  95

Thr Phe Gly Gln Gly Thr Lys Leu Glu Ile Lys

100                 105

<210>  42
<211>  116
<212>  PRT
<213>  Artificial Sequence

<220>
<223>  Antibody Sequence hu11C10.L2H1 VH

<400>  42

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr His Asp Lys Thr Phe Asp Tyr Trp Gly Gln Gly Thr Met Val
            100                 105                 110

Thr Val Ser Ser
        115

<210>  43
<211>  107
<212>  PRT
<213>  Artificial Sequence

<220>
<223>  Antibody Sequence hu11C10.L3H1 VL

<400>  43

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asp Ile Tyr Ser Asp
            20                  25                  30

Leu Ala Trp Tyr Gln Gln Lys Pro Gly Lys Ala Pro Lys Leu Leu Ile
        35                  40                  45

Tyr Asp Val Asn Ser Leu Ile His Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Asp Asn Tyr Pro Asn
                85                  90                  95

Thr Phe Gly Gln Gly Thr Lys Leu Glu Ile Lys
            100                 105

<210>  44
<211>  116
<212>  PRT
<213>  Artificial Sequence

<220>
<223>  Antibody Sequence hu11C10.L3H1 VH

<400>  44

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr His Asp Lys Thr Phe Asp Tyr Trp Gly Gln Gly Thr Met Val
            100                 105                 110

Thr Val Ser Ser
        115

<210>  45

<211>  107
<212>  PRT
<213>  Artificial Sequence

<220>
<223>  Antibody Sequence hu11C10.L4H1 VL

<400>  45

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asp Ile Tyr Ser Asp
            20                  25                  30

Leu Ala Trp Tyr Gln Gln Lys Pro Gly Lys Ser Pro Lys Leu Leu Ile
        35                  40                  45

Tyr Asp Val Asn Ser Leu Ile His Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asp Asn Tyr Pro Asn
                85                  90                  95

Thr Phe Gly Gln Gly Thr Lys Leu Glu Ile Lys
            100                 105


<210>  46
<211>  116
<212>  PRT
<213>  Artificial Sequence

<220>
<223>  Antibody Sequence hu11C10.L4H1 VH

<400>  46

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr His Asp Lys Thr Phe Asp Tyr Trp Gly Gln Gly Thr Met Val
            100                 105                 110

Thr Val Ser Ser
        115

<210>  47
<211>  107
<212>  PRT
<213>  Artificial Sequence

<220>
<223>  Antibody Sequence hu11C10.L5H1 VL

<400>  47

Asp Ile Gln Leu Thr Gln Ser Pro Ser Ser Leu Ser Ala Ser Val Gly
1               5                   10                  15

Asp Arg Val Thr Ile Thr Cys Arg Ala Ser Glu Asp Ile Tyr Ser Asp
            20                  25                  30

Leu Ala Trp Tyr Gln Gln Lys Pro Gly Lys Ala Pro Lys Leu Leu Ile
        35                  40                  45

Tyr Asp Val Asn Ser Leu Ile His Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asp Asn Tyr Pro Asn
                85                  90                  95

Thr Phe Gly Gln Gly Thr Lys Leu Glu Ile Lys
            100                 105

<210>  48
<211>  116
<212>  PRT
<213>  Artificial Sequence

<220>
<223>  Antibody Sequence hu11C10.L5H1 VH

<400>  48

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr His Asp Lys Thr Phe Asp Tyr Trp Gly Gln Gly Thr Met Val
            100                 105                 110

Thr Val Ser Ser
        115

<210>  49
<211>  107
<212>  PRT
<213>  Artificial Sequence

<220>
<223>  Antibody Sequence 15F3 VL

<400>  49

Asp Ile Gln Met Thr Gln Ser Pro Ala Ser Leu Ser Ala Ser Leu Gly
1               5                   10                  15

Glu Thr Val Ser Ile Glu Cys Leu Val Ser Glu Asp Ile Ser Asn Asp
            20                  25                  30

Phe Val Trp Tyr Gln Gln Lys Ser Gly Lys Ser Pro Gln Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Arg Phe Ser Leu Arg Ile Ser Gly Met Gln Pro
65                  70                  75                  80

Glu Asp Glu Ala Glu Tyr Phe Cys Gln Gln Ser Tyr Lys Tyr Pro Pro
                85                  90                  95

Thr Phe Gly Ala Gly Thr Lys Leu Glu Leu Lys
            100                 105

<210>  50
<211>  116
<212>  PRT
<213>  Artificial Sequence

<220>
<223>  Antibody Sequence 15F3 VH

<400>  50

Glu Val Gln Leu Val Glu Ser Gly Gly Gly Leu Val Gln Pro Gly Arg
1               5                   10                  15

Ser Leu Lys Leu Ser Cys Ala Ala Ser Gly Phe Thr Phe Ser Asp Tyr
            20                  25                  30

Asp Met Ala Trp Val Arg Gln Ala Pro Lys Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Val Ser Arg Asp Asn Thr Lys Ser Thr Leu Tyr
65                  70                  75                  80

Leu Gln Met Asp Ser Leu Arg Ser Glu Asp Thr Ala Thr Tyr Tyr Cys
                85                  90                  95

Thr Ala His Asp Arg Ser Phe Asp Tyr Trp Gly Gln Gly Val Met Val
            100                 105                 110

Thr Val Ser Ser
        115

<210>  51
<211>  107
<212>  PRT
<213>  Artificial Sequence

<220>
<223>  Antibody Sequence hu15F3.L1H1 VL

<400>  51

Asp Ile Gln Met Thr Gln Ser Pro Ser Ser Leu Ser Ala Ser Val Gly
1               5                   10                  15

Asp Arg Val Thr Ile Thr Cys Leu Val Ser Glu Asp Ile Ser Asn Asp
            20                  25                  30

Phe Val Trp Tyr Gln Gln Lys Pro Gly Lys Ser Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Lys Tyr Pro Pro
                85                  90                  95

Thr Phe Gly Gln Gly Thr Lys Leu Glu Ile Lys
            100                 105

<210>  52
<211>  116
<212>  PRT
<213>  Artificial Sequence

<220>
<223>  Antibody Sequence hu15F3.L1H1 VH

<400>  52

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Val Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser
        115

<210>  53
<211>  107
<212>  PRT
<213>  Artificial Sequence

<220>
<223>  Antibody Sequence hu15F3.L1H2 VL

<400>  53

Asp Ile Gln Met Thr Gln Ser Pro Ser Ser Leu Ser Ala Ser Val Gly
1               5                   10                  15

Asp Arg Val Thr Ile Thr Cys Leu Val Ser Glu Asp Ile Ser Asn Asp
            20                  25                  30

Phe Val Trp Tyr Gln Gln Lys Pro Gly Lys Ser Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Lys Tyr Pro Pro
                85                  90                  95

Thr Phe Gly Gln Gly Thr Lys Leu Glu Ile Lys
            100                 105

<210>  54
<211>  116
<212>  PRT
<213>  Artificial Sequence

<220>
<223>  Antibody Sequence hu15F3.L1H2 VH

<400>  54

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser
        115

<210>  55
<211>  107
<212>  PRT
<213>  Artificial Sequence

<220>
<223>  Antibody Sequence hu15F3.L2H1 VL

<400>  55

Asp Ile Gln Met Thr Gln Ser Pro Ser Ser Leu Ser Ala Ser Val Gly
1               5                   10                  15

Asp Arg Val Thr Ile Thr Cys Leu Val Ser Glu Asp Ile Ser Asn Asp
            20                  25                  30

Phe Val Trp Tyr Gln Gln Lys Pro Gly Lys Ala Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Lys Tyr Pro Pro
                85                  90                  95

Thr Phe Gly Gln Gly Thr Lys Leu Glu Ile Lys
            100                 105

<210>  56
<211>  116
<212>  PRT
<213>  Artificial Sequence

<220>
<223>  Antibody Sequence hu15F3.L2H1 VH

<400>  56

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Val Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser
        115

<210>  57
<211>  107
<212>  PRT
<213>  Artificial Sequence

<220>
<223>  Antibody Sequence hu15F3.L3H1 VL

<400>  57

Asp Ile Gln Met Thr Gln Ser Pro Ser Ser Leu Ser Ala Ser Val Gly
1               5                   10                  15

Asp Arg Val Thr Ile Thr Cys Leu Val Ser Glu Asp Ile Ser Asn Asp
            20                  25                  30

Phe Val Trp Tyr Gln Gln Lys Pro Gly Lys Ser Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Lys Tyr Pro Pro
                85                  90                  95

Thr Phe Gly Gln Gly Thr Lys Leu Glu Ile Lys
            100                 105

<210>  58
<211>  116
<212>  PRT
<213>  Artificial Sequence

<220>
<223>  Antibody Sequence hu15F3.L3H1 VH

<400>  58

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Val Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser
        115

<210>  59
<211>  107
<212>  PRT
<213>  Artificial Sequence

<220>
<223>  Antibody Sequence hu15F3.L4H1 VL

<400>  59

Asp Ile Gln Met Thr Gln Ser Pro Ser Ser Leu Ser Ala Ser Val Gly
1               5                   10                  15

Asp Arg Val Thr Ile Thr Cys Leu Val Ser Glu Asp Ile Ser Asn Asp
            20                  25                  30

Phe Val Trp Tyr Gln Gln Lys Pro Gly Lys Ala Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Lys Tyr Pro Pro
                85                  90                  95

Thr Phe Gly Gln Gly Thr Lys Leu Glu Ile Lys
            100                 105


<210>  60
<211>  116
<212>  PRT
<213>  Artificial Sequence

<220>
<223>  Antibody Sequence hu15F3.L4H1 VH

<400>  60

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Val Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser
        115

<210>  61
<211>  107
<212>  PRT
<213>  Artificial Sequence

<220>
<223>  Antibody Sequence hu15F3.L4H2 VL

<400>  61

Asp Ile Gln Met Thr Gln Ser Pro Ser Ser Leu Ser Ala Ser Val Gly
1               5                   10                  15

Asp Arg Val Thr Ile Thr Cys Leu Val Ser Glu Asp Ile Ser Asn Asp
            20                  25                  30

Phe Val Trp Tyr Gln Gln Lys Pro Gly Lys Ala Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Lys Tyr Pro Pro
                85                  90                  95

Thr Phe Gly Gln Gly Thr Lys Leu Glu Ile Lys
            100                 105

<210>  62
<211>  116
<212>  PRT
<213>  Artificial Sequence

<220>
<223>  Antibody Sequence hu15F3.L4H2 VH

<400>  62

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser
        115

<210> 63
<211> 179
<212> PRT
<213> Artificial Sequence

<220>
<223> Antibody Sequence 4A6 Light Chain

<400> 63

Asp Ile Gln Met Thr Gln Ser Pro Ala Ser Leu Ser Ala Ser Leu Gly
1               5                   10                  15

Glu Thr Val Ser Ile Glu Cys Leu Ala Ser Glu Asp Ile Ser Asn Asp
            20                  25                  30

Leu Val Trp Tyr Gln Gln Lys Ser Gly Lys Ser Pro Gln Leu Leu Ile
        35                  40                  45

Tyr Ala Ala Ser Arg Leu Gln Asp Gly Val Pro Ser Arg Phe Ser Gly
    50                  55                  60

Ser Gly Phe Gly Thr Arg Phe Ser Leu Lys Ile Ser Gly Met Gln Pro
65                  70                  75                  80

Glu Asp Glu Ala Asp Tyr Phe Cys Gln Gln Ser Tyr Lys Tyr Leu Pro
                85                  90                  95

Thr Phe Gly Ala Gly Thr Lys Leu Gly Leu Lys Arg Ala Asp Ala Ala
            100                 105                 110

Pro Thr Val Ser Ile Phe Pro Pro Ser Met Glu Gln Leu Thr Ser Gly
        115                 120                 125

Gly Ala Thr Val Val Cys Phe Val Asn Asn Phe Tyr Pro Arg Asp Ile
    130                 135                 140

Ser Val Lys Trp Lys Ile Asp Gly Ser Glu Gln Arg Asp Gly Val Leu
145                 150                 155                 160

Asp Ser Val Thr Asp Gln Asp Ser Lys Asp Ser Thr Tyr Ser Met Ser
                165                 170                 175

Ser Thr Leu

<210>  64
<211>  127
<212>  PRT
<213>  Artificial Sequence

<220>
<223>  Antibody Sequence 4A6 Heavy Chain

<400>  64

Glu Val Gln Leu Val Glu Ser Gly Gly Gly Ser Val Gln Pro Gly Arg
1               5                   10                  15

Ser Leu Lys Val Ser Cys Ala Ala Ser Gly Phe Thr Phe Ser Asp Tyr
            20                  25                  30

Asp Met Ala Trp Val Arg Gln Ala Pro Lys Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Leu Ser Arg Asp Asn Thr Lys Ser Thr Leu Cys
65                  70                  75                  80

Leu Gln Met Asp Ser Leu Arg Ser Glu Asp Thr Ala Thr Tyr Tyr Cys
                85                  90                  95

Ala Ala His Asp Arg Ser Phe Asp Tyr Trp Gly Gln Gly Ile Met Val
            100                 105                 110

Thr Val Ser Ser Ala Glu Thr Thr Ala Pro Ser Val Tyr Pro Leu
        115                 120                 125

<210>  65
<211>  107
<212>  PRT
<213>  Artificial Sequence

<220>
<223>  Antibody Sequence hu4A6.L1H1 Light Chain

<400>  65

Asp Ile Gln Met Thr Gln Ser Pro Ser Ser Leu Ser Ala Ser Val Gly
1               5                   10                  15

Asp Arg Val Thr Ile Thr Cys Leu Ala Ser Glu Asp Ile Ser Asn Asp
            20                  25                  30

Leu Val Trp Tyr Gln Gln Lys Pro Gly Lys Ser Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Lys Tyr Leu Pro
                85                  90                  95

Thr Phe Gly Gln Gly Thr Lys Leu Glu Ile Lys
            100                 105


<210>  66
<211>  116
<212>  PRT
<213>  Artificial Sequence

<220>
<223>  Antibody Sequence hu4A6.L1H1 Heavy Chain

<400>  66

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Leu Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser
        115

<210>  67
<211>  107
<212>  PRT
<213>  Artificial Sequence

<220>
<223>  Antibody Sequence hu4A6.L1H2 Light Chain

<400>  67

Asp Ile Gln Met Thr Gln Ser Pro Ser Ser Leu Ser Ala Ser Val Gly
1               5                   10                  15

Asp Arg Val Thr Ile Thr Cys Leu Ala Ser Glu Asp Ile Ser Asn Asp
            20                  25                  30

Leu Val Trp Tyr Gln Gln Lys Pro Gly Lys Ser Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Lys Tyr Leu Pro
                85                  90                  95

Thr Phe Gly Gln Gly Thr Lys Leu Glu Ile Lys
            100                 105

<210>  68
<211>  116
<212>  PRT
<213>  Artificial Sequence

<220>
<223>  Antibody Sequence hu4A6.L1H2 Heavy Chain

<400>  68

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser
        115

<210>  69
<211>  107
<212>  PRT
<213>  Artificial Sequence

<220>
<223>  Antibody Sequence hu4A6.L2H1 Light Chain

<400>  69

Asp Ile Gln Met Thr Gln Ser Pro Ser Ser Leu Ser Ala Ser Val Gly
1               5                   10                  15

Asp Arg Val Thr Ile Thr Cys Leu Ala Ser Glu Asp Ile Ser Asn Asp
            20                  25                  30

Leu Val Trp Tyr Gln Gln Lys Pro Gly Lys Ala Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Lys Tyr Leu Pro
                85                  90                  95

Thr Phe Gly Gln Gly Thr Lys Leu Glu Ile Lys
            100                 105

<210>  70
<211>  116
<212>  PRT
<213>  Artificial Sequence

<220>
<223>  Antibody Sequence hu4A6.L2H1 Heavy Chain

<400>  70

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Leu Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser
        115

<210>  71
<211>  107
<212>  PRT
<213>  Artificial Sequence

<220>
<223>  Antibody Sequence hu4A6.L3H1 Light Chain

<400>  71

Asp Ile Gln Met Thr Gln Ser Pro Ser Ser Leu Ser Ala Ser Val Gly
1               5                   10                  15

Asp Arg Val Thr Ile Thr Cys Leu Ala Ser Glu Asp Ile Ser Asn Asp
            20                  25                  30

Leu Val Trp Tyr Gln Gln Lys Pro Gly Lys Ser Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Lys Tyr Leu Pro
                85                  90                  95

Thr Phe Gly Gln Gly Thr Lys Leu Glu Ile Lys
            100                 105

<210>  72
<211>  116
<212>  PRT
<213>  Artificial Sequence

<220>
<223>  Antibody Sequence hu4A6.L3H1 Heavy Chain

<400>  72

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Leu Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser
        115

<210>  73
<211>  107
<212>  PRT
<213>  Artificial Sequence

<220>
<223>  Antibody Sequence hu4A6.L4H1 Light Chain

<400>  73

Asp Ile Gln Met Thr Gln Ser Pro Ser Ser Leu Ser Ala Ser Val Gly
1               5                   10                  15

Asp Arg Val Thr Ile Thr Cys Leu Ala Ser Glu Asp Ile Ser Asn Asp
            20                  25                  30

Leu Val Trp Tyr Gln Gln Lys Pro Gly Lys Ala Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Phe Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Lys Tyr Leu Pro
                85                  90                  95

Thr Phe Gly Gln Gly Thr Lys Leu Glu Ile Lys
            100                 105

<210>  74
<211>  116
<212>  PRT
<213>  Artificial Sequence

<220>
<223>  Antibody Sequence hu4A6.L4H1 Heavy Chain

<400>  74

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Leu Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser
        115

<210>  75
<211>  214
<212>  PRT
<213>  Artificial Sequence

<220>
<223>  Antibody Sequence hu4A6.L4H2 Light Chain

<400>  75

Asp Ile Gln Met Thr Gln Ser Pro Ser Ser Leu Ser Ala Ser Val Gly
1               5                   10                  15

Asp Arg Val Thr Ile Thr Cys Leu Ala Ser Glu Asp Ile Ser Asn Asp
            20                  25                  30

Leu Val Trp Tyr Gln Gln Lys Pro Gly Lys Ala Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Phe Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Lys Tyr Leu Pro
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  76
<211>  446
<212>  PRT
<213>  Artificial Sequence

<220>
<223>  Antibody Sequence hu4A6.L4H2 Heavy Chain (wild type human IgG1)

<400>  76

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  77
<211>  179
<212>  PRT
<213>  Artificial Sequence

<220>
<223>  Antibody Sequence 11C10 Light Chain

<400>  77

Asp Ile Gln Met Thr Gln Ser Pro Ala Ser Leu Ser Ala Ser Leu Gly
1               5                   10                  15

Glu Thr Val Thr Ile Glu Cys Arg Ala Ser Glu Asp Ile Tyr Ser Asp
            20                  25                  30

Leu Ala Trp Tyr Gln Gln Lys Pro Gly Asn Ser Pro Gln Leu Leu Ile
        35                  40                  45

Tyr Asp Val Asn Ser Leu Ile His Gly Val Pro Ser Arg Phe Ser Gly
    50                  55                  60

Ser Gly Ser Gly Thr Gln Phe Ser Leu Lys Ile Asn Asn Leu Gln Ser
65                  70                  75                  80

Glu Asp Val Ala Ser Tyr Phe Cys Gln Gln Tyr Asp Asn Tyr Pro Asn
                85                  90                  95

Thr Phe Gly Ala Gly Thr Lys Leu Glu Leu Lys Arg Ala Asp Ala Ala
            100                 105                 110

Pro Thr Val Ser Ile Phe Pro Pro Ser Met Glu Gln Leu Thr Ser Gly
        115                 120                 125

Gly Ala Thr Val Val Cys Phe Val Asn Asn Phe Tyr Pro Arg Asp Ile
    130                 135                 140

Ser Val Lys Trp Lys Ile Asp Gly Ser Glu Gln Arg Asp Gly Val Leu
145                 150                 155                 160

Asp Ser Val Thr Asp Gln Asp Ser Lys Asp Ser Thr Tyr Ser Met Ser
                165                 170                 175

Ser Thr Leu

<210>  78
<211>  127
<212>  PRT
<213>  Artificial Sequence

<220>
<223>  Antibody Sequence 11C10 Heavy Chain

<400>  78

Glu Val Gln Leu Val Glu Ser Gly Gly Gly Leu Val Gln Pro Gly Arg
1               5                   10                  15

Ser Leu Lys Leu Ser Cys Ala Ala Ser Gly Phe Thr Phe Ser Asp Tyr
            20                  25                  30

Asp Met Ala Trp Val Arg Gln Ala Pro Lys Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Ala Asn Ala Lys Ser Thr Leu Tyr
65                  70                  75                  80

Leu Gln Met Asp Ser Leu Arg Ser Glu Asp Thr Ala Thr Tyr Tyr Cys
                85                  90                  95

Ala Thr His Asp Lys Thr Phe Asp Tyr Trp Gly Gln Gly Val Met Val
            100                 105                 110

Thr Val Ser Ser Ala Glu Thr Thr Ala Pro Ser Val Tyr Pro Leu
        115                 120                 125

<210>  79
<211>  107
<212>  PRT
<213>  Artificial Sequence

<220>
<223>  Antibody Sequence hu11C10.L1H1 Light Chain

<400>  79

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asp Ile Tyr Ser Asp
            20                  25                  30

Leu Ala Trp Tyr Gln Gln Lys Pro Gly Lys Ser Pro Lys Leu Leu Ile
        35                  40                  45

Tyr Asp Val Asn Ser Leu Ile His Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Asp Asn Tyr Pro Asn
                85                  90                  95

Thr Phe Gly Gln Gly Thr Lys Leu Glu Ile Lys
            100                 105


<210>  80
<211>  116
<212>  PRT
<213>  Artificial Sequence

<220>
<223>  Antibody Sequence hu11C10.L1H1 Heavy Chain

<400>  80

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr His Asp Lys Thr Phe Asp Tyr Trp Gly Gln Gly Thr Met Val
            100                 105                 110

Thr Val Ser Ser
        115

<210>  81
<211>  107
<212>  PRT
<213>  Artificial Sequence

<220>
<223>  Antibody Sequence hu11C10.L2H1 Light Chain

<400>  81

Asp Ile Gln Leu Thr Gln Ser Pro Ser Ser Leu Ser Ala Ser Val Gly
1               5                   10                  15

Asp Arg Val Thr Ile Thr Cys Arg Ala Ser Glu Asp Ile Tyr Ser Asp
            20                  25                  30

Leu Ala Trp Tyr Gln Gln Lys Pro Gly Lys Ser Pro Lys Leu Leu Ile
        35                  40                  45

Tyr Asp Val Asn Ser Leu Ile His Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Asp Asn Tyr Pro Asn
                85                  90                  95

Thr Phe Gly Gln Gly Thr Lys Leu Glu Ile Lys
            100                 105

<210>  82
<211>  116
<212>  PRT
<213>  Artificial Sequence

<220>
<223>  Antibody Sequence hu11C10.L2H1 Heavy Chain

<400>  82

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr His Asp Lys Thr Phe Asp Tyr Trp Gly Gln Gly Thr Met Val
            100                 105                 110

Thr Val Ser Ser
        115

<210> 83
<211> 107
<212> PRT
<213> Artificial Sequence

<220>
<223> Antibody Sequence hu11C10.L3H1 Light Chain

<400> 83

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asp Ile Tyr Ser Asp
            20                  25                  30

Leu Ala Trp Tyr Gln Gln Lys Pro Gly Lys Ala Pro Lys Leu Leu Ile
        35                  40                  45

Tyr Asp Val Asn Ser Leu Ile His Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Asp Asn Tyr Pro Asn
                85                  90                  95

Thr Phe Gly Gln Gly Thr Lys Leu Glu Ile Lys
            100                 105

<210>  84
<211>  116
<212>  PRT
<213>  Artificial Sequence

<220>
<223>  Antibody Sequence hu11C10.L3H1 Heavy Chain

<400>  84

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr His Asp Lys Thr Phe Asp Tyr Trp Gly Gln Gly Thr Met Val
            100                 105                 110

Thr Val Ser Ser
        115

<210>  85
<211>  107
<212>  PRT
<213>  Artificial Sequence

<220>
<223>  Antibody Sequence hu11C10.L4H1 Light Chain

<400>  85

Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asp Ile Tyr Ser Asp
            20                  25                  30

Leu Ala Trp Tyr Gln Gln Lys Pro Gly Lys Ser Pro Lys Leu Leu Ile
        35                  40                  45

Tyr Asp Val Asn Ser Leu Ile His Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asp Asn Tyr Pro Asn
                85                  90                  95

Thr Phe Gly Gln Gly Thr Lys Leu Glu Ile Lys
            100                 105

<210>  86
<211>  116
<212>  PRT
<213>  Artificial Sequence

<220>
<223>  Antibody Sequence hu11C10.L4H1 Heavy Chain

<400>  86

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr His Asp Lys Thr Phe Asp Tyr Trp Gly Gln Gly Thr Met Val
            100                 105                 110

Thr Val Ser Ser
        115

<210>  87
<211>  214
<212>  PRT
<213>  Artificial Sequence

<220>
<223>  Antibody Sequence hu11C10.L5H1 Light Chain

<400>  87

Asp Ile Gln Leu Thr Gln Ser Pro Ser Ser Leu Ser Ala Ser Val Gly
1               5                   10                  15

Asp Arg Val Thr Ile Thr Cys Arg Ala Ser Glu Asp Ile Tyr Ser Asp
            20                  25                  30

Leu Ala Trp Tyr Gln Gln Lys Pro Gly Lys Ala Pro Lys Leu Leu Ile
        35                  40                  45

Tyr Asp Val Asn Ser Leu Ile His Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asp Asn Tyr Pro Asn
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys

    210

<210>  88
<211>  446
<212>  PRT
<213>  Artificial Sequence

<220>
<223>  Antibody Sequence hu11C10.L5H1 Heavy Chain (human IgG1 wild-type)

<400>  88

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr His Asp Lys Thr Phe Asp Tyr Trp Gly Gln Gly Thr Met Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  89

<211>  107
<212>  PRT
<213>  Artificial Sequence

<220>
<223>  Antibody Sequence 15F3 Light Chain

<400>  89

Asp Ile Gln Met Thr Gln Ser Pro Ala Ser Leu Ser Ala Ser Leu Gly
1               5                   10                  15

Glu Thr Val Ser Ile Glu Cys Leu Val Ser Glu Asp Ile Ser Asn Asp
            20                  25                  30

Phe Val Trp Tyr Gln Gln Lys Ser Gly Lys Ser Pro Gln Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Arg Phe Ser Leu Arg Ile Ser Gly Met Gln Pro
65                  70                  75                  80

Glu Asp Glu Ala Glu Tyr Phe Cys Gln Gln Ser Tyr Lys Tyr Pro Pro
                85                  90                  95

Thr Phe Gly Ala Gly Thr Lys Leu Glu Leu Lys
            100                 105


<210>  90
<211>  127
<212>  PRT
<213>  Artificial Sequence

<220>
<223>  Antibody Sequence 15F3 Heavy Chain

<400>  90

Glu Val Gln Leu Val Glu Ser Gly Gly Gly Leu Val Gln Pro Gly Arg
1               5                   10                  15

Ser Leu Lys Leu Ser Cys Ala Ala Ser Gly Phe Thr Phe Ser Asp Tyr
            20                  25                  30

Asp Met Ala Trp Val Arg Gln Ala Pro Lys Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Val Ser Arg Asp Asn Thr Lys Ser Thr Leu Tyr
65                  70                  75                  80

Leu Gln Met Asp Ser Leu Arg Ser Glu Asp Thr Ala Thr Tyr Tyr Cys
                85                  90                  95

Thr Ala His Asp Arg Ser Phe Asp Tyr Trp Gly Gln Gly Val Met Val
            100                 105                 110

Thr Val Ser Ser Ala Glu Thr Thr Ala Pro Ser Val Tyr Pro Leu
        115                 120                 125

<210>  91
<211>  107
<212>  PRT
<213>  Artificial Sequence

<220>
<223>  Antibody Sequence hu15F3.L1H1 Light Chain

<400>  91

Asp Ile Gln Met Thr Gln Ser Pro Ser Ser Leu Ser Ala Ser Val Gly
1               5                   10                  15

Asp Arg Val Thr Ile Thr Cys Leu Val Ser Glu Asp Ile Ser Asn Asp
            20                  25                  30

Phe Val Trp Tyr Gln Gln Lys Pro Gly Lys Ser Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Lys Tyr Pro Pro
                85                  90                  95

Thr Phe Gly Gln Gly Thr Lys Leu Glu Ile Lys
            100                 105

<210>  92
<211>  116
<212>  PRT
<213>  Artificial Sequence

<220>
<223>  Antibody Sequence hu15F3.L1H1 Heavy Chain

<400>  92

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Val Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser
        115

<210>  93
<211>  107
<212>  PRT
<213>  Artificial Sequence

<220>
<223>  Antibody Sequence hu15F3.L1H2 Light Chain

<400>  93

Asp Ile Gln Met Thr Gln Ser Pro Ser Ser Leu Ser Ala Ser Val Gly
1               5                   10                  15

Asp Arg Val Thr Ile Thr Cys Leu Val Ser Glu Asp Ile Ser Asn Asp
            20                  25                  30

Phe Val Trp Tyr Gln Gln Lys Pro Gly Lys Ser Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Lys Tyr Pro Pro
                85                  90                  95

Thr Phe Gly Gln Gly Thr Lys Leu Glu Ile Lys
            100                 105

<210>  94
<211>  116
<212>  PRT
<213>  Artificial Sequence

<220>
<223>  Antibody Sequence hu15F3.L1H2 Heavy Chain

<400>  94

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser
        115

<210>  95
<211>  107
<212>  PRT
<213>  Artificial Sequence

<220>
<223>  Antibody Sequence hu15F3.L2H1 Light Chain

<400>  95

Asp Ile Gln Met Thr Gln Ser Pro Ser Ser Leu Ser Ala Ser Val Gly
1               5                   10                  15

Asp Arg Val Thr Ile Thr Cys Leu Val Ser Glu Asp Ile Ser Asn Asp
            20                  25                  30

Phe Val Trp Tyr Gln Gln Lys Pro Gly Lys Ala Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Lys Tyr Pro Pro
                85                  90                  95

Thr Phe Gly Gln Gly Thr Lys Leu Glu Ile Lys
            100                 105

<210>  96
<211>  116
<212>  PRT
<213>  Artificial Sequence

<220>
<223>  Antibody Sequence hu15F3.L2H1 Heavy Chain

<400>  96

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Val Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser
        115

<210>  97
<211>  107
<212>  PRT
<213>  Artificial Sequence

<220>
<223>  Antibody Sequence hu15F3.L3H1 Light Chain

<400>  97

Asp Ile Gln Met Thr Gln Ser Pro Ser Ser Leu Ser Ala Ser Val Gly
1               5                   10                  15

Asp Arg Val Thr Ile Thr Cys Leu Val Ser Glu Asp Ile Ser Asn Asp
            20                  25                  30

Phe Val Trp Tyr Gln Gln Lys Pro Gly Lys Ser Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Lys Tyr Pro Pro
                85                  90                  95

Thr Phe Gly Gln Gly Thr Lys Leu Glu Ile Lys
            100                 105

<210>  98
<211>  116
<212>  PRT
<213>  Artificial Sequence

<220>
<223>  Antibody Sequence hu15F3.L3H1 Heavy Chain

<400>  98

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Val Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser
        115

<210>  99
<211>  107
<212>  PRT
<213>  Artificial Sequence

<220>
<223>  Antibody Sequence hu15F3.L4H1 Light Chain

<400>  99

Asp Ile Gln Met Thr Gln Ser Pro Ser Ser Leu Ser Ala Ser Val Gly
1               5                   10                  15

Asp Arg Val Thr Ile Thr Cys Leu Val Ser Glu Asp Ile Ser Asn Asp
            20                  25                  30

Phe Val Trp Tyr Gln Gln Lys Pro Gly Lys Ala Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Lys Tyr Pro Pro
                85                  90                  95

Thr Phe Gly Gln Gly Thr Lys Leu Glu Ile Lys
            100                 105

<210>  100
<211>  116
<212>  PRT
<213>  Artificial Sequence

<220>
<223>  Antibody Sequence hu15F3.L4H1 Heavy Chain

<400>  100

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Val Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser
        115


<210>  101
<211>  214
<212>  PRT
<213>  Artificial Sequence

<220>
<223>  Antibody Sequence hu15F3.L4H2 Light Chain

<400>  101

Asp Ile Gln Met Thr Gln Ser Pro Ser Ser Leu Ser Ala Ser Val Gly
1               5                   10                  15

Asp Arg Val Thr Ile Thr Cys Leu Val Ser Glu Asp Ile Ser Asn Asp
            20                  25                  30

Phe Val Trp Tyr Gln Gln Lys Pro Gly Lys Ala Pro Lys Leu Leu Ile
        35                  40                  45

Tyr Ala Ala Ser Arg Leu Gln Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Lys Tyr Pro Pro
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  102
<211>  446
<212>  PRT
<213>  Artificial Sequence

<220>
<223>  Antibody Sequence hu15F3.L4H2 Heavy Chain (human IgG1)

<400>  102

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  103
<211>  1019
<212>  DNA
<213>  Artificial Sequence

<220>
<223>  EIF3E(e1)-RSPO2(e2) translocation fusion polynucleotide

<400>  103
gagcacagac tccctttctt ttggcaagat ggcggagtac gacttgacta ctcgcatcgc      60

gcactttttg gatcggcatc tagtctttcc gcttcttgaa tttctctctg taaaggaggt     120

tcgtggcgga gagatgctga tcgcgctgaa ctgaccggtg cggcccgggg gtgagtggcg     180

agtctccctc tgagtcctcc ccagcagcgc ggccggcgcc ggctctttgg gcgaaccctc     240

cagttcctag actttgagag gcgtctctcc cccgcccgac cgcccagatg cagtttcgcc     300

ttttctcctt tgccctcatc attctgaact gcatggatta cagccactgc caaggcaacc     360

gatggagacg cagtaagcga gctagttatg tatcaaatcc catttgcaag ggttgtttgt     420

cttgttcaaa ggacaatggg tgtagccgat gtcaacagaa gttgttcttc ttccttcgaa     480

gagaagggat gcgccagtat ggagagtgcc tgcattcctg cccatccggg tactatggac     540

accgagcccc agatatgaac agatgtgcaa gatgcagaat agaaaactgt gattcttgct 600

ttagcaaaga cttttgtacc aagtgcaaag taggctttta tttgcataga ggccgttgct 660

ttgatgaatg tccagatggt tttgcaccat tagaagaaac catggaatgt gtggaaggat 720

gtgaagttgg tcattggagc gaatggggaa cttgtagcag aaataatcgc acatgtggat 780

ttaaatgggg tctggaaacc agaacacggc aaattgttaa aaagccagtg aaagacacaa 840

tactgtgtcc aaccattgct gaatccagga gatgcaagat gacaatgagg cattgtccag 900

gagggaagag aacaccaaag gcgaaggaga agaggaacaa gaaaaagaaa aggaagctga 960

tagaaagggc ccaggagcaa cacagcgtct tcctagctac agacagagct aaccaataa 1019

<210> 104
<211> 284
<212> PRT
<213> Artificial Sequence

<220>
<223> EIF3E(e1)-RSPO2(e2) translocation fusion polypeptide

<400> 104

Met Ala Glu Tyr Asp Leu Thr Thr Arg Ile Ala His Phe Leu Asp Arg
1               5                   10                  15

His Leu Val Phe Pro Leu Leu Glu Phe Leu Ser Val Lys Glu Val Arg
            20                  25                  30

Gly Gly Glu Met Leu Ile Ala Leu Asn Met Gln Phe Arg Leu Phe Ser
        35                  40                  45

Phe Ala Leu Ile Ile Leu Asn Cys Met Asp Tyr Ser His Cys Gln Gly
    50                  55                  60

Asn Arg Trp Arg Arg Ser Lys Arg Ala Ser Tyr Val Ser Asn Pro Ile
65                  70                  75                  80

Cys Lys Gly Cys Leu Ser Cys Ser Lys Asp Asn Gly Cys Ser Arg Cys
                85                  90                  95

Gln Gln Lys Leu Phe Phe Phe Leu Arg Arg Glu Gly Met Arg Gln Tyr
            100                 105                 110

Gly Glu Cys Leu His Ser Cys Pro Ser Gly Tyr Tyr Gly His Arg Ala
        115                 120                 125

Pro Asp Met Asn Arg Cys Ala Arg Cys Arg Ile Glu Asn Cys Asp Ser
    130                 135                 140

Cys Phe Ser Lys Asp Phe Cys Thr Lys Cys Lys Val Gly Phe Tyr Leu
145                 150                 155                 160

His Arg Gly Arg Cys Phe Asp Glu Cys Pro Asp Gly Phe Ala Pro Leu
                165                 170                 175

Glu Glu Thr Met Glu Cys Val Glu Gly Cys Glu Val Gly His Trp Ser
            180                 185                 190

Glu Trp Gly Thr Cys Ser Arg Asn Asn Arg Thr Cys Gly Phe Lys Trp
        195                 200                 205

Gly Leu Glu Thr Arg Thr Arg Gln Ile Val Lys Lys Pro Val Lys Asp
    210                 215                 220

Thr Ile Leu Cys Pro Thr Ile Ala Glu Ser Arg Arg Cys Lys Met Thr
225                 230                 235                 240

Met Arg His Cys Pro Gly Gly Lys Arg Thr Pro Lys Ala Lys Glu Lys
                245                 250                 255

Arg Asn Lys Lys Lys Lys Arg Lys Leu Ile Glu Arg Ala Gln Glu Gln
            260                 265                 270

His Ser Val Phe Leu Ala Thr Asp Arg Ala Asn Gln
        275                 280

<210>  105
<211>  822
<212>  DNA
<213>  Artificial Sequence

<220>
<223>  PTPRK(e1)-RSPO3(e2) translocation fusion polynucleotide

<400>  105
atggatacga ctgcggcggc ggcgctgcct gcttttgtgg cgctcttgct cctctctcct      60

tggcctctcc tgggatcggc ccaaggccag ttctccgcag tgcatcctaa cgttagtcaa     120

ggctgccaag gaggctgtgc aacatgctca gattacaatg gatgtttgtc atgtaagccc     180

agactatttt ttgctctgga aagaattggc atgaagcaga ttggagtatg tctctcttca     240

tgtccaagtg gatattatgg aactcgatat ccagatataa ataagtgtac aaaatgcaaa     300

gctgactgtg atacctgttt caacaaaaat ttctgcacaa aatgtaaaag tggattttac     360

ttacaccttg gaaagtgcct tgacaattgc ccagaagggt tggaagccaa caaccatact     420

atggagtgtg tcagtattgt gcactgtgag gtcagtgaat ggaatccttg gagtccatgc     480

acgaagaagg gaaaaacatg tggcttcaaa agagggactg aaacacgggt ccgagaaata     540

atacagcatc cttcagcaaa gggtaacctg tgtcccccaa caaatgagac aagaaagtgt 600

acagtgcaaa ggaagaagtg tcagaaggga gaacgaggaa aaaaaggaag ggagaggaaa 660

agaaaaaaac ctaataaagg agaaagtaaa gaagcaatac ctgacagcaa aagtctggaa 720

tccagcaaag aaatcccaga gcaacgagaa aacaaacagc agcagaagaa gcgaaaagtc 780

caagataaac agaaatcggt atcagtcagc actgtacact ag 822

<210> 106
<211> 217
<212> PRT
<213> Artificial Sequence

<220>
<223> PTPRK(e1)-RSPO3(e2) translocation fusion polypeptide

<400> 106

Met Asp Thr Thr Ala Ala Ala Ala Leu Pro Ala Phe Val Ala Leu Leu
1               5                   10                  15

Leu Leu Ser Pro Trp Pro Leu Leu Gly Ser Ala Gln Gly Gln Phe Ser
            20                  25                  30

Ala Val His Pro Asn Val Ser Gln Gly Cys Gln Gly Gly Cys Ala Thr
        35                  40                  45

Cys Ser Asp Tyr Asn Gly Cys Leu Ser Cys Lys Pro Arg Leu Phe Phe
    50                  55                  60

Ala Leu Glu Arg Ile Gly Met Lys Gln Ile Gly Val Cys Leu Ser Ser
65                  70                  75                  80

Cys Pro Ser Gly Tyr Tyr Gly Thr Arg Tyr Pro Asp Ile Asn Lys Cys
                85                  90                  95

Thr Lys Cys Lys Ala Asp Cys Asp Thr Cys Phe Asn Lys Asn Phe Cys
            100                 105                 110

Thr Lys Cys Lys Ser Gly Phe Tyr Leu His Leu Gly Lys Cys Leu Asp
        115                 120                 125

Asn Cys Pro Glu Gly Leu Glu Ala Asn Asn His Thr Met Glu Cys Val
    130                 135                 140

Ser Ile Val His Cys Glu Val Ser Glu Trp Asn Pro Trp Ser Pro Cys
145                 150                 155                 160

Thr Lys Lys Gly Lys Thr Cys Gly Phe Lys Arg Gly Thr Glu Thr Arg

165                 170                 175

Val Arg Glu Ile Ile Gln His Pro Ser Ala Lys Gly Asn Leu Cys Pro
            180                 185                 190

Pro Thr Asn Glu Thr Arg Lys Cys Thr Val Gln Arg Lys Lys Cys Gln
        195                 200                 205

Lys Gly Glu Arg Gly Lys Lys Gly Arg
    210                 215

<210>  107
<211>  1884
<212>  DNA
<213>  Artificial Sequence

<220>
<223>  PTPRK(e7)-RSPO3(e2) translocation fusion polynucleotide

<400>  107
atggatacga ctgcggcggc ggcgctgcct gcttttgtgg cgctcttgct cctctctcct      60

tggcctctcc tgggatcggc ccaaggccag ttctccgcag gtggctgtac ttttgatgat     120

ggtccagggg cctgtgatta ccaccaggat ctgtatgatg actttgaatg ggtgcatgtt     180

agtgctcaag agcctcatta tctaccaccc gagatgcccc aaggttccta tatgatagtg     240

gactcttcag atcacgaccc tggagaaaaa gccagacttc agctgcctac aatgaaggag     300

aacgacactc actgcattga tttcagttac ctattatata gccagaaagg actgaatcct     360

ggcactttga acatattagt tagggtgaat aaaggacctc ttgccaatcc aatttggaat     420

gtgactggat tcacgggtag agattggctt cgggctgagc tagcagtgag caccttttgg     480

cccaatgaat atcaggtaat atttgaagct gaagtctcag gagggagaag tggttatatt     540

gccattgatg acatccaagt actgagttat ccttgtgata aatctcctca tttcctccgt     600

ctaggggatg tagaggtgaa tgcagggcaa aacgctacat ttcagtgcat tgccacaggg     660

agagatgctg tgcataacaa gttatggctc cagagacgaa atggagaaga tataccagta     720

gcccagacta agaacatcaa tcatagaagg tttgccgctt ccttcagatt gcaagaagtg     780

acaaaaactg accaggattt gtatcgctgt gtaactcagt cagaacgagg ttccggtgtg     840

tccaattttg ctcaacttat tgtgagagaa ccgccaagac ccattgctcc tcctcagctt     900

cttggtgttg ggcctacata tttgctgatc caactaaatg ccaactcgat cattggcgat     960

ggtcctatca tcctgaaaga agtagagtac cgaatgacat caggatcctg gacagaaacc    1020

catgcagtca atgctccaac ttacaaatta tggcatttag atccagatac cgaatatgag    1080

atccgagttc tacttacaag acctggtgaa ggtggaacgg ggctcccagg acctccacta    1140

atcaccagaa caaaatgtgc agtgcatcct aacgttagtc aaggctgcca aggaggctgt    1200

gcaacatgct cagattacaa tggatgtttg tcatgtaagc ccagactatt ttttgctctg 1260

gaaagaattg gcatgaagca gattggagta tgtctctctt catgtccaag tggatattat 1320

ggaactcgat atccagatat aaataagtgt acaaaatgca aagctgactg tgatacctgt 1380

ttcaacaaaa atttctgcac aaaatgtaaa agtggatttt acttacacct tggaaagtgc 1440

cttgacaatt gcccagaagg gttggaagcc aacaaccata ctatggagtg tgtcagtatt 1500

gtgcactgtg aggtcagtga atggaatcct tggagtccat gcacgaagaa gggaaaaaca 1560

tgtggcttca aaagagggac tgaaacacgg gtccgagaaa taatacagca tccttcagca 1620

aagggtaacc tgtgtccccc aacaaatgag acaagaaagt gtacagtgca aaggaagaag 1680

tgtcagaagg gagaacgagg aaaaaaagga agggagagga aaagaaaaaa acctaataaa 1740

ggagaaagta aagaagcaat acctgacagc aaaagtctgg aatccagcaa agaaatccca 1800

gagcaacgag aaaacaaaca gcagcagaag aagcgaaaag tccaagataa acagaaatcg 1860

gtatcagtca gcactgtaca ctag 1884

<210> 108
<211> 627
<212> PRT
<213> Artificial Sequence

<220>
<223> PTPRK(e7)-RSPO3(e2) translocation fusion polypeptide

<400> 108

Met Asp Thr Thr Ala Ala Ala Ala Leu Pro Ala Phe Val Ala Leu Leu
1               5                   10                  15

Leu Leu Ser Pro Trp Pro Leu Leu Gly Ser Ala Gln Gly Gln Phe Ser
            20                  25                  30

Ala Gly Gly Cys Thr Phe Asp Asp Gly Pro Gly Ala Cys Asp Tyr His
        35                  40                  45

Gln Asp Leu Tyr Asp Asp Phe Glu Trp Val His Val Ser Ala Gln Glu
    50                  55                  60

Pro His Tyr Leu Pro Pro Glu Met Pro Gln Gly Ser Tyr Met Ile Val
65                  70                  75                  80

Asp Ser Ser Asp His Asp Pro Gly Glu Lys Ala Arg Leu Gln Leu Pro
                85                  90                  95

Thr Met Lys Glu Asn Asp Thr His Cys Ile Asp Phe Ser Tyr Leu Leu
            100                 105                 110

Tyr Ser Gln Lys Gly Leu Asn Pro Gly Thr Leu Asn Ile Leu Val Arg
        115                 120                 125

Val Asn Lys Gly Pro Leu Ala Asn Pro Ile Trp Asn Val Thr Gly Phe
    130                 135                 140

Thr Gly Arg Asp Trp Leu Arg Ala Glu Leu Ala Val Ser Thr Phe Trp
145                 150                 155                 160

Pro Asn Glu Tyr Gln Val Ile Phe Glu Ala Glu Val Ser Gly Gly Arg
                165                 170                 175

Ser Gly Tyr Ile Ala Ile Asp Asp Ile Gln Val Leu Ser Tyr Pro Cys
            180                 185                 190

Asp Lys Ser Pro His Phe Leu Arg Leu Gly Asp Val Glu Val Asn Ala
        195                 200                 205

Gly Gln Asn Ala Thr Phe Gln Cys Ile Ala Thr Gly Arg Asp Ala Val
    210                 215                 220

His Asn Lys Leu Trp Leu Gln Arg Arg Asn Gly Glu Asp Ile Pro Val
225                 230                 235                 240

Ala Gln Thr Lys Asn Ile Asn His Arg Arg Phe Ala Ala Ser Phe Arg
                245                 250                 255

Leu Gln Glu Val Thr Lys Thr Asp Gln Asp Leu Tyr Arg Cys Val Thr
            260                 265                 270

Gln Ser Glu Arg Gly Ser Gly Val Ser Asn Phe Ala Gln Leu Ile Val
        275                 280                 285

Arg Glu Pro Pro Arg Pro Ile Ala Pro Pro Gln Leu Leu Gly Val Gly
    290                 295                 300

Pro Thr Tyr Leu Leu Ile Gln Leu Asn Ala Asn Ser Ile Ile Gly Asp
305                 310                 315                 320

Gly Pro Ile Ile Leu Lys Glu Val Glu Tyr Arg Met Thr Ser Gly Ser
                325                 330                 335

Trp Thr Glu Thr His Ala Val Asn Ala Pro Thr Tyr Lys Leu Trp His
            340                 345                 350

Leu Asp Pro Asp Thr Glu Tyr Glu Ile Arg Val Leu Leu Thr Arg Pro
        355                 360                 365

Gly Glu Gly Gly Thr Gly Leu Pro Gly Pro Pro Leu Ile Thr Arg Thr
    370                 375                 380

Lys Cys Ala Val His Pro Asn Val Ser Gln Gly Cys Gln Gly Gly Cys
385                 390                 395                 400

Ala Thr Cys Ser Asp Tyr Asn Gly Cys Leu Ser Cys Lys Pro Arg Leu
                405                 410                 415

Phe Phe Ala Leu Glu Arg Ile Gly Met Lys Gln Ile Gly Val Cys Leu
            420                 425                 430

Ser Ser Cys Pro Ser Gly Tyr Tyr Gly Thr Arg Tyr Pro Asp Ile Asn
        435                 440                 445

Lys Cys Thr Lys Cys Lys Ala Asp Cys Asp Thr Cys Phe Asn Lys Asn
    450                 455                 460

Phe Cys Thr Lys Cys Lys Ser Gly Phe Tyr Leu His Leu Gly Lys Cys
465                 470                 475                 480

Leu Asp Asn Cys Pro Glu Gly Leu Glu Ala Asn Asn His Thr Met Glu
                485                 490                 495

Cys Val Ser Ile Val His Cys Glu Val Ser Glu Trp Asn Pro Trp Ser
            500                 505                 510

Pro Cys Thr Lys Lys Gly Lys Thr Cys Gly Phe Lys Arg Gly Thr Glu
        515                 520                 525

Thr Arg Val Arg Glu Ile Ile Gln His Pro Ser Ala Lys Gly Asn Leu
    530                 535                 540

Cys Pro Pro Thr Asn Glu Thr Arg Lys Cys Thr Val Gln Arg Lys Lys
545                 550                 555                 560

Cys Gln Lys Gly Glu Arg Gly Lys Lys Gly Arg Glu Arg Lys Arg Lys
                565                 570                 575

Lys Pro Asn Lys Gly Glu Ser Lys Glu Ala Ile Pro Asp Ser Lys Ser
            580                 585                 590

Leu Glu Ser Ser Lys Glu Ile Pro Glu Gln Arg Glu Asn Lys Gln Gln
        595                 600                 605

Gln Lys Lys Arg Lys Val Gln Asp Lys Gln Lys Ser Val Ser Val Ser

610                 615                 620

Thr Val His
625

<210>  109
<211>  19
<212>  DNA
<213>  Artificial Sequence

<220>
<223>  5′ PCR primer

<400>  109
cttgcggaaa ggatgttgg                                                    19

<210>  110
<211>  68
<212>  DNA
<213>  Artificial Sequence

<220>
<223>  5′ PCR primer

<400>  110
caccccgctg cctctaggtt ctgggaagat ggcgaaggtc tcagagcttt acgatgtcac      60

ttgggaag                                                                68

<210>  111
<211>  19
<212>  DNA
<213>  Artificial Sequence

<220>
<223>  5′ PCR primer

<400>  111
actactcgca tcgcgcact                                                    19

<210>  112
<211>  20
<212>  DNA
<213>  Artificial Sequence

<220>
<223>  5′ PCR primer

<400>  112
aaactcggca tggatacgac                                                   20

<210>  113
<211>  20
<212>  DNA
<213>  Artificial Sequence

<220>

<223>  5′ PCR primer

<400>  113
tgcagtcaat gctccaactt                                                    20


<210>  114
<211>  20
<212>  DNA
<213>  Artificial Sequence

<220>
<223>  5′ PCR primer

<400>  114
aagcccatca acctctctca                                                    20


<210>  115
<211>  20
<212>  DNA
<213>  Artificial Sequence

<220>
<223>  5′ PCR primer

<400>  115
ctctacaccc ccaagtgcat                                                    20


<210>  116
<211>  20
<212>  DNA
<213>  Artificial Sequence

<220>
<223>  5′ PCR primer

<400>  116
aacaggagac ccgtacatgc                                                    20


<210>  117
<211>  21
<212>  DNA
<213>  Artificial Sequence

<220>
<223>  5′ PCR primer

<400>  117
ccagctgcta gctactgtgg a                                                  21


<210>  118
<211>  21
<212>  DNA
<213>  Artificial Sequence

<220>
<223>  5′ PCR primer

<400>  118

tgaaccgaag tttagcaatg g                                                21

<210>  119
<211>  20
<212>  DNA
<213>  Artificial Sequence

<220>
<223>  5′ PCR primer

<400>  119
tgatgaactt tgcagccact                                                  20

<210>  120
<211>  20
<212>  DNA
<213>  Artificial Sequence

<220>
<223>  5′ PCR primer

<400>  120
agggccagat ttgagtgtgt                                                  20

<210>  121
<211>  20
<212>  DNA
<213>  Artificial Sequence

<220>
<223>  5′ PCR primer

<400>  121
gtgtatggcg tcgtgatgtc                                                  20

<210>  122
<211>  20
<212>  DNA
<213>  Artificial Sequence

<220>
<223>  5′ PCR primer

<400>  122
catgtcggag aacatctgga                                                  20

<210>  123
<211>  20
<212>  DNA
<213>  Artificial Sequence

<220>
<223>  5′ PCR primer

<400>  123
ccttactgcc ttgtgggaga                                                  20

<210>  124
<211>  20
<212>  DNA
<213>  Artificial Sequence

<220>
<223>  5′ PCR primer

<400>  124
cagagacccg tgctgagttt                                                    20


<210>  125
<211>  20
<212>  DNA
<213>  Artificial Sequence

<220>
<223>  5′ PCR primer

<400>  125
gactttggtg ccctcaacat                                                    20


<210>  126
<211>  20
<212>  DNA
<213>  Artificial Sequence

<220>
<223>  5′ PCR primer

<400>  126
aacgggaact cttagcagca                                                    20


<210>  127
<211>  20
<212>  DNA
<213>  Artificial Sequence

<220>
<223>  5′ PCR primer

<400>  127
gagacttcat gcgggagttc                                                    20


<210>  128
<211>  21
<212>  DNA
<213>  Artificial Sequence

<220>
<223>  5′ PCR primer

<400>  128
tggccttcgc taactacaag a                                                  21


<210>  129
<211>  18
<212>  DNA

<213>  Artificial Sequence

<220>
<223>  5′ PCR primer

<400>  129
gctctttggc gcggatta                                                    18


<210>  130
<211>  20
<212>  DNA
<213>  Artificial Sequence

<220>
<223>  5′ PCR primer

<400>  130
gttgcaaaag gcttgctgat                                                  20


<210>  131
<211>  20
<212>  DNA
<213>  Artificial Sequence

<220>
<223>  5′ PCR primer

<400>  131
tgattgatgc tgccaaacat                                                  20


<210>  132
<211>  20
<212>  DNA
<213>  Artificial Sequence

<220>
<223>  5′ PCR primer

<400>  132
atgaacctta tctcggccct                                                  20


<210>  133
<211>  20
<212>  DNA
<213>  Artificial Sequence

<220>
<223>  5′ PCR primer

<400>  133
atgtgtacgc agaagagcca                                                  20


<210>  134
<211>  21
<212>  DNA
<213>  Artificial Sequence

<220>

<223>  5′ PCR primer

<400>  134
ggaaaatcct catatttgcc a                                                21


<210>  135
<211>  20
<212>  DNA
<213>  Artificial Sequence

<220>
<223>  5′ PCR primer

<400>  135
agacccagga ggagtgaggt                                                  20


<210>  136
<211>  20
<212>  DNA
<213>  Artificial Sequence

<220>
<223>  5′ PCR primer

<400>  136
agatgcccag atgcaaaagt                                                  20


<210>  137
<211>  20
<212>  DNA
<213>  Artificial Sequence

<220>
<223>  5′ PCR primer

<400>  137
ggctgagggt ggagtttgta                                                  20


<210>  138
<211>  20
<212>  DNA
<213>  Artificial Sequence

<220>
<223>  5′ PCR primer

<400>  138
ccccagttag aaggggaaga                                                  20


<210>  139
<211>  20
<212>  DNA
<213>  Artificial Sequence

<220>
<223>  3′ PCR primer

<400>  139

tggtgatcca gagaagaagc                                                    20

<210>  140
<211>  70
<212>  DNA
<213>  Artificial Sequence

<220>
<223>  3' PCR primer

<400>  140
gttcgtggcg gagagatgct gatcgcgctg aactgaccgg tgcggcccgg gggtgagtgg        60

cgagtctccc                                                               70

<210>  141
<211>  20
<212>  DNA
<213>  Artificial Sequence

<220>
<223>  3' PCR primer

<400>  141
gggaggactc agagggagac                                                    20

<210>  142
<211>  20
<212>  DNA
<213>  Artificial Sequence

<220>
<223>  3' PCR primer

<400>  142
tgcaggcact ctccatactg                                                    20

<210>  143
<211>  20
<212>  DNA
<213>  Artificial Sequence

<220>
<223>  3' PCR primer

<400>  143
gcttcatgcc aattctttcc                                                    20

<210>  144
<211>  20
<212>  DNA
<213>  Artificial Sequence

<220>
<223>  3' PCR primer

<400>  144
gccaattctt tccagagcaa                                                    20

<210>  145
<211>  20
<212>  DNA
<213>  Artificial Sequence

<220>
<223>  3′ PCR primer

<400>  145
gggctgaggt tgtagcactc                                                   20


<210>  146
<211>  21
<212>  DNA
<213>  Artificial Sequence

<220>
<223>  3′ PCR primer

<400>  146
tgacaccata atggattcct g                                                 21


<210>  147
<211>  20
<212>  DNA
<213>  Artificial Sequence

<220>
<223>  3′ PCR primer

<400>  147
aaagggcaca gattgccata                                                   20


<210>  148
<211>  20
<212>  DNA
<213>  Artificial Sequence

<220>
<223>  3′ PCR primer

<400>  148
actaggtggt ccagggtgtg                                                   20


<210>  149
<211>  20
<212>  DNA
<213>  Artificial Sequence

<220>
<223>  3′ PCR primer

<400>  149
tgctcaagca ggtaagatgc                                                   20


<210>  150

<211>  20
<212>  DNA
<213>  Artificial Sequence

<220>
<223>  3' PCR primer

<400>  150
atggtctcca tcagctctcg                                                   20


<210>  151
<211>  20
<212>  DNA
<213>  Artificial Sequence

<220>
<223>  3' PCR primer

<400>  151
aaactgaaaa tccccgctgt                                                   20


<210>  152
<211>  20
<212>  DNA
<213>  Artificial Sequence

<220>
<223>  3' PCR primer

<400>  152
gctccagtca ccaaaaggag                                                   20


<210>  153
<211>  20
<212>  DNA
<213>  Artificial Sequence

<220>
<223>  3' PCR primer

<400>  153
tgtggagtct cttgcgtgtc                                                   20


<210>  154
<211>  20
<212>  DNA
<213>  Artificial Sequence

<220>
<223>  3' PCR primer

<400>  154
tggggatgag gtcgatgtat                                                   20


<210>  155
<211>  20
<212>  DNA
<213>  Artificial Sequence

<220>
<223>  3' PCR primer

<400>  155
ccaaaaggtg tttcgtcctt                                                    20


<210>  156
<211>  21
<212>  DNA
<213>  Artificial Sequence

<220>
<223>  3' PCR primer

<400>  156
caatttttcc actccaacac c                                                  21


<210>  157
<211>  20
<212>  DNA
<213>  Artificial Sequence

<220>
<223>  3' PCR primer

<400>  157
catgtcaaac caccatccac                                                    20


<210>  158
<211>  20
<212>  DNA
<213>  Artificial Sequence

<220>
<223>  3' PCR primer

<400>  158
atctggaagc aggggtcttt                                                    20


<210>  159
<211>  20
<212>  DNA
<213>  Artificial Sequence

<220>
<223>  3' PCR primer

<400>  159
tccccatatt tctgcactcc                                                    20


<210>  160
<211>  18
<212>  DNA
<213>  Artificial Sequence

<220>
<223>  3' PCR primer

<400>  160
ggagctacct gtggccct                                                    18


<210>  161
<211>  20
<212>  DNA
<213>  Artificial Sequence

<220>
<223>  3' PCR primer

<400>  161
acgaaggctt cctcacagaa                                                  20


<210>  162
<211>  20
<212>  DNA
<213>  Artificial Sequence

<220>
<223>  3' PCR primer

<400>  162
cacgcttttc atattcccgt                                                  20


<210>  163
<211>  20
<212>  DNA
<213>  Artificial Sequence

<220>
<223>  3' PCR primer

<400>  163
tcccaaaggc ttcttcttga                                                  20


<210>  164
<211>  19
<212>  DNA
<213>  Artificial Sequence

<220>
<223>  3' PCR primer

<400>  164
gtcgtgtacc ccagaggct                                                   19


<210>  165
<211>  20
<212>  DNA
<213>  Artificial Sequence

<220>
<223>  3' PCR primer

<400>  165
gtgcaggaat tgggctatgt                                                  20

<210>  166
<211>  20
<212>  DNA
<213>  Artificial Sequence

<220>
<223>  3′ PCR primer

<400>  166
agcagggaag cctcctagtc                                                    20


<210>  167
<211>  20
<212>  DNA
<213>  Artificial Sequence

<220>
<223>  3′ PCR primer

<400>  167
ggtcagccag tgaggtcttc                                                    20


<210>  168
<211>  20
<212>  DNA
<213>  Artificial Sequence

<220>
<223>  3′ PCR primer

<400>  168
caaagcagac tttccaacgc                                                    20


<210>  169
<211>  20
<212>  DNA
<213>  Artificial Sequence

<220>
<223>  3′ PCR primer

<400>  169
cttctgatcg aagctttccg                                                    20


<210>  170
<211>  20
<212>  DNA
<213>  Artificial Sequence

<220>
<223>  3′ PCR primer

<400>  170
cactctcatc tctgggctcc                                                    20


<210>  171

<211>  446
<212>  PRT
<213>  Artificial Sequence

<220>
<223>  Antibody Sequence hu4A6.L4H2 Heavy Chain (human IgG1 N297G)

<400>  171

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala His Asp Arg Ser Phe Asp Tyr Trp Gly Gln Gly Thr Met Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Gly Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  172
<211>  446
<212>  PRT
<213>  Artificial Sequence

<220>
<223>  hu11C10.L5H1 Heavy Chain (human IgG1 N297G)

<400>  172

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr His Asp Lys Thr Phe Asp Tyr Trp Gly Gln Gly Thr Met Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Gly Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  173
<211>  446
<212>  PRT
<213>  Artificial Sequence

<220>
<223>  hu15F3.L4H2 Heavy Chain (human IgG1 N297G)

<400>  173

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asp Tyr
            20                  25                  30

Asp Met Ala Trp Val Arg Gln Ala Pro Gly Lys Gly Leu Glu Trp Val
        35                  40                  45

Ala Thr Ile Ile Tyr Asp Gly Ser Arg Ala Tyr Phe Gly Asp Ser Val
    50                  55                  60

Arg Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Ala His Asp Arg Ser Phe Asp Tyr Trp Gly Gln Gly Thr Met Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Gly Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445